# EUROPEAN PATENT APPLICATION

(11) **EP 2 500 032 A1**
(43) Date of publication of application: **19.09.2012**
(21) Application number: 11183690.4
(22) Date of filing: 23.06.2003
(51) Int. Cl.: A61K 38/19, C12N 15/19, C07K 14/525

(54) **APO-2 ligand/trail variants and uses thereof**

(30) Priority: 24.06.2002 US 391050 P
(62) Divisional of application: 03761262.9
(71) Applicant: Genentech, Inc., South San Francisco, CA 94080 (US)
(72) Inventor: Kelley, Robert F., San Bruno, CA 94066 (US); Hymowitz, Sarah, San Francisco, CA 94123 (US); Lindstrom, Stephanie Ho, Millbrae, CA 94030 (US)
(74) Representative: Kiddle, Simon John

(57) **Abstract**

The disclosure provides Apo-2 ligand variant polypeptides. Methods of making and chemically modifying Apo-2 ligand variant polypeptides are also provided. In addition, formulations of Apo-2 ligand variant polypeptides are provided. In addition, therapeutic methods for using Apo-2 ligand variant polypeptides are provided.

## Description

### FIELD OF THE INVENTION

The present invention relates to Apo-2 ligand/TRAIL variants, to methods for preparing such variants, and to methods, compositions and assays utilizing such variants. In particular, the invention relates to Apo-2 ligand/TRAIL variants which have binding affinity properties for the Apo-2 ligand/TRAIL receptors, DR4 and DR5, different from that of native sequence Apo-2 ligand/TRAIL. In addition, the invention relates to Apo-2 ligand/TRAIL variants that have cysteine substitutions which can, for example, facilitate chemical modification by moieties such as polyethylene glycol.

### BACKGROUND OF THE INVENTION

Control of cell numbers in mammals is believed to be determined, in part, by a balance between cell proliferation and cell death. One form of cell death, sometimes referred to as necrotic cell death, is typically characterized as a pathologic form of cell death resulting from some trauma or cellular injury. In contrast, there is another, "physiologic" form of cell death which usually proceeds in an orderly or controlled manner. This orderly or controlled form of cell death is often referred to as "apoptosis" [see, e.g., Barr et al., Bio/Technology, 12:487-493 (1994); Steller et al., Science, 267:1445-1449 (1995)]. Apoptotic cell death naturally occurs in many physiological processes, including embryonic development and clonal selection in the immune system [Itoh et al., Cell, 66:233-243 (1991)]. Various molecules, such as tumor necrosis factor-alpha ("TNF-alpha"), tumor necrosis factor-beta ("TNF-beta" or "lymphotoxin-alpha"), lymphotoxin-beta ("LT-beta"), CD30 ligand, CD27 ligand, CD40 ligand, OX-40 ligand, 4-1BB ligand, Apo-1 ligand (also referred to as Fas ligand or CD95 ligand), Apo-2 ligand (also referred to as Apo2L or TRAIL), Apo-3 ligand (also referred to as TWEAK), APRIL, OPG ligand (also referred to as RANK ligand, ODF, or TRANCE), and TALL-1 (also referred to as BlyS, BAFF or THANK) have been identified as members of the tumor necrosis factor ("TNF") family of cytokines [See, e.g., Gruss and Dower, Blood, 85:3378-3404 (1995); Schmid et al., Proc. Natl. Acad. Sci., 83:1881 (1986); Dealtry et al., Eur. J. Immunol., 17:689 (1987); Pitti et al., J. Biol. Chem., 271:12687-12690 (1996); Wiley et al., Immunity, 3:673-682 (1995); Browning et al., Cell, 72:847-856 (1993); Armitage et al. Nature, 357:80-82 (1992), WO 97/01633 published January 16, 1997; WO 97/25428 published July 17, 1997; Marsters et al., Curr. Biol., 8:525-528 (1998); Chicheportiche et al., Biol. Chem., 272:32401-32410 (1997); Hahne et al., J. Exp. Med., 188:1185-1190 (1998); WO98/28426 published July 2, 1998; WO98/46751 published October 22, 1998; WO/98/18921 published May 7, 1998; Moore et al., Science, 285:260-263 (1999); Shu et al., J. Leukocyte Biol., 65:680 (1999); Schneider et al., J. Exp. Med., 189:1747-1756 (1999); Mukhopadhyay et al., J. Biol. Chem., 274:15978-15981 (1999)]. Among these molecules, TNF-alpha, TNF-beta, CD30 ligand, 4-1BB ligand, Apo-1 ligand, Apo-2 ligand (Apo2L/TR.AIL) and Apo-3 ligand (TWEAK) have been reported to be involved in apoptotic cell death.

Apo2L/TRAIL was identified several years ago as a member of the TNF family of cytokines. [see, e.g., Wiley et al., Immunity, 3:673-682 (1995); Pitti et al., J. Biol. Chem., 271:12697-12690 (1996); US Patent 6,284,236 issued September 4, 2001] The full-length native sequence human Apo2L/TRAIL polypeptide is a 281 amino acid long, Type II transmembrane protein. Some cells can produce a natural soluble form of the polypeptide, through enzymatic cleavage of the polypeptide's extracellular region [Mariani et al., J. Cell. Biol., 137:221-229 (1997)]. Crystallographic studies of soluble forms of Apo2L/TRAIL reveal a homotrimeric structure similar to the structures of TNF and other related proteins [Hymowitz et al., Molec. Cell, 4:563-571 (1999); Hymowitz et al., Biochemistry, 39:633-644 (2000)]. Apo2L/TRAIL, unlike other TNF family members however, was found to have a unique structural feature in that three cysteine residues (at position 230 of each subunit in the homotrimer) together coordinate a zinc atom, and that the zinc binding is important for trimer stability and biological activity. [Hymowitz et al., supra; Bodmer et al., J. Biol. Chem., 275:20632-20637 (2000)]

It has been reported in the literature that Apo2L/TRAIL may play a role in immune system modulation, including autoimmune diseases such as rheumatoid arthritis [see, e.g., Thomas et al., J. Immunol., 161:2195-2200 (1998); Johnsen et al., Cytokine, 11:664-672 (1999); Griffith et al., J. Exp. Med., 189:1343-1353 (1999); Song et al., J. Exp. Med., 191:1095-1103 (2000)].

Soluble forms of Apo2L/TRAIL have also been reported to induce apoptosis in a variety of cancer cells *in vitro,* including colon, lung, breast, prostate, bladder, kidney, ovarian and brain tumors, as well as melanoma, leukemia, and multiple myeloma [see, e.g., Wiley et al., supra; Pitti et al., supra; Rieger et al., FEBS Letters, 427:124-128 (1998); Ashkenazi et al., J. Clin. Invest., 104:155-162 (1999); Walczak et al., Nature Med., 5:157-163 (1999); Keane et al., Cancer Research, 59:734-741 (1999); Mizutani et al., Clin. Cancer Res., 5:2605-2612 (1999); Gazitt, Leukemia, 13:1817-1824 (1999); Yu et al., Cancer Res., 60:2384-2389 (2000); Chinnaiyan et al., Proc. Natl. Acad. Sci., 97:1754-1759 (2000)]. *In vivo* studies in murine tumor models further suggest that Apo2L/TRAIL, alone or in combination with chemotherapy or radiation therapy, can exert substantial anti-tumor effects [see, e.g., Ashkenazi et al., supra; Walzcak et al., supra; Gliniak et al., Cancer Res., 59:6153-6158 (1999); Chinnaiyan et al., supra; Roth et al., Biochem. Biophys. Res. Comm., 265:1999 (1999)]. In contrast to many types of cancer cells, most normal human cell types appear to be resistant to apoptosis induction by certain recombinant forms of Apo2L/TRAIL [Ashkenazi et al., supra; Walzcak et al., supra]. Jo et al. has reported that a polyhistidine-tagged soluble form of Apo2L/TRAIL induced apoptosis *in vitro* in normal isolated human, but not non-human, hepatocytes [Jo et al., Nature Med., 6:564-567 (2000); see also, Nagata, Nature Med., 6:502-503 (2000)]. It is believed that certain recombinant Apo2L/TRAIL preparations may vary in terms of biochemical properties and biological activities on diseased versus normal cells, depending, for example, on the presence or absence of a tag molecule, zinc content, and % trimer content [See, Lawrence et al., Nature Med., Letter to the Editor, 7:383-385 (2001); Qin et al., Nature Med., Letter to the Editor, 7:385-386 (2001)].

Induction of various cellular responses mediated by the TNF family cytokines is believed to be initiated by their binding to specific cell receptors. Two distinct TNF receptors of approximately 55-kDa (TNFR1) and 75-kDa (TNFR2) have been identified [Hohman et al., J. Biol. Chem., 264:14927-14934 (1989); Brockhaus et al., Proc. Natl. Acad. Sci., 87:3127-3131 (1990); EP 417,563, published March 20, 1991] and human and mouse cDNAs corresponding to both receptor types have been isolated and characterized [Loetscher et al., Cell, 61:351 (1990); Schall et al., Cell, 61:361 (1990); Smith et al., Science, 248:1019-1023 (1990); Lewis et al., Proc. Natl. Acad. Sci., 88:2830-2834 (1991); Goodwin et al., Mol. Cell. Biol., 11:3020-3026 (1991)]. Extensive polymorphisms have been associated with both TNF receptor genes [see, e.g., Takao et al., Immunogenetics, 37:199-203 (1993)]. Both TNFRs share the typical structure of cell surface receptors including extracellular, transmembrane and intracellular regions. The extracellular portions of both receptors are found naturally also as soluble TNF-binding proteins [Nophar, Y. et al., EMBO J., 9:3269 (1990); and Kohno, T. et al., Proc. Natl. Acad. Sci. U.S.A., 87:8331 (1990)]. The cloning of recombinant soluble TNF receptors was reported by Hale et al. [J. Cell. Biochem. Supplement 15F, 1991, p. 113 (P424)].

The extracellular portion of type 1 and type 2 TNFRs (TNFR1 and TNFR2) contains a repetitive amino acid sequence pattern of four cysteine-rich domains (CRDs) designated 1 through 4, starting from the NH₂-terminus. Each CRD is about 40 amino acids long and contains 4 to 6 cysteine residues at positions which are well conserved [Schall et al., supra; Loetscher et al., supra; Smith et al., supra; Nophar et al., supra; Kohno et al., supra]. In TNFR1, the approximate boundaries of the four CRDs are as follows: CRD1-amino acids 14 to about 53; CRD2- amino acids from about 54 to about 97; CRD3- amino acids from about 98 to about 138; CRD4- amino acids from about 139 to about 167. In TNFR2, CRD1 includes amino acids 17 to about 54; CRD2- amino acids from about 55 to about 97; CRD3- amino acids from about 98 to about 140; and CRD4- amino acids from about 141 to about 179 [Banner et al., Cell, 73:431-435 (1993)]. The potential role of the CRDs in ligand binding is also described by Banner et al., supra.

A similar repetitive pattern of CRDs exists in several other cell-surface proteins, including the p75 nerve growth factor receptor (NGFR) [Johnson et al., Cell, 47:545 (1986); Radeke et al., Nature, 325:593 (1987)], the B cell antigen CD40 [Stamenkovic et al., EMBO J., 8:1403 (1989)], the T cell antigen OX40 [Mallet et al., EMBO J., 9:1063 (1990)] and the Fas antigen [Yonehara et al., J. Exp. Med., 169:1747-1756 (1989) and Itoh et al., Cell, 66:233-243 (1991)]. CRDs are also found in the soluble TNFR (sTNFR)-like T2 proteins of the Shope and myxoma poxviruses [Upton et al., Virology, 160:20-29 (1987); Smith et al., Biochem. Biophys. Res. Commun., 176:335 (1991); Upton et al., Virology, 184:370 (1991)]. Optimal alignment of these sequences indicates that the positions of the cysteine residues are well conserved. These receptors are sometimes collectively referred to as members of the TNF/NGF receptor superfamily. Recent studies on p75NGFR showed that the deletion of CRD1 [Welcher, A.A. et al., Proc. Natl. Acad. Sci. USA, 88:159-163 (1991)] or a 5-amino acid insertion in this domain [Yan, H. and Chao, M.V., J. Biol. Chem., 266:12099-12104 (1991)] had little or no effect on NGF binding [Yan, H. and Chao, M.V., supra]. p75 NGFR contains a proline-rich stretch of about 60 amino acids, between its CRD4 and transmembrane region, which is not involved in NGF binding [Peetre, C. et al., Eur. J. Hematol., 41:414-419 (1988); Seckinger, P. et al., J. Biol. Chem., 264:11966-11973 (1989); Yan, H. and Chao, M.V., supra]. A similar proline-rich region is found in TNFR2 but not in TNFR1.

The TNF family ligands identified to date, with the exception of lymphotoxin-α, are type II transmembrane proteins, whose C-terminus is extracellular. In contrast, most receptors in the TNF receptor (TNFR) family identified to date are type I transmembrane proteins. In both the TNF ligand and receptor families, however, homology identified between family members has been found mainly in the extracellular domain ("ECD"). Several of the TNF family cytokines, including TNF-α, Apo-1 ligand and CD40 ligand, are cleaved proteolytically at the cell surface; the resulting protein in each case typically forms a homotrimeric molecule that functions as a soluble cytokine. TNF receptor family proteins are also usually cleaved proteolytically to release soluble receptor ECDs that can function as inhibitors of the cognate cytokines.

Recently, other members of the TNFR family have been identified. Such newly identified members of the TNFR family include CAR1, HVEM and osteoprotegerin (OPG) [Brojatsch et al., Cell, 87:845-855 (1996); Montgomery et al., Cell, 87:427-436 (1996); Marsters et al., J. Biol. Chem., 272:14029-14032 (1997); Simonet et al., Cell, 89:309-319 (1997)]. Unlike other known TNFR-like molecules, Simonet et al., supra, report that OPG contains no hydrophobic transmembrane-spanning sequence. OPG is believed to act as a decoy receptor, as discussed below.

Pan et al. have disclosed another TNF receptor family member referred to as "DR4" [Pan et al., Science, 276:111-113 (1997)]. The DR4 was reported to contain a cytoplasmic death domain capable of engaging the cell suicide apparatus. Pan et al. disclose that DR4 is believed to be a receptor for the ligand known as Apo-2 ligand or TRAIL.

In Sheridan et al., Science, 277:818-821 (1997) and Pan et al., Science, 277:815-818 (1997), another molecule believed to be a receptor for Apo2L/TRAIL is described [see also, WO98/51793 published November 19, 1998; WO98/41629 published September 24, 1998]. That molecule is referred to as DR5 (it has also been alternatively referred to as Apo-2; TRAIL-R, TR6, Tango-63, hAPO8 TRICK2 or KILLER [Screaton et al., Curr. Biol., 7:693-696 (1997); Walczak et al., EMBO J., 16:5386-5387 (1997); Wu et al., Nature Genetics, 17:141-143 (1997); WO98/35986 published August 20, 1998; EP870,827 published October 14, 1998; WO98/46643 published October 22, 1998; WO99/02653 published January 21, 1999; WO99/09165 published February 25, 1999; WO99/11791 published March 11, 1999]. Like DR4, DR5 is reported to contain a cytoplasmic death domain and be capable of signaling apoptosis. The crystal structure of the complex formed between Apo-2L/TRAIL and DR5 is described in Hymowitz et al., Molecular Cell, 4:563-571 (1999).

A further group of recently identified TNFR family members are referred to as "decoy receptors," which are believed to function as inhibitors, rather than transducers of signaling. This group includes DCR1 (also referred to as TRID, LIT or TRAIL-R3) [Pan et al., Science, 276:111-113 (1997); Sheridan et al., Science, 277:818-821 (1997); McFarlane et al., J. Biol. Chem., 272:25417-25420 (1997); Schneider et al., FEBS Letters, 416:329-334 (1997); Degli-Esposti et al., J. Exp. Med., 186:1165-1170 (1997); and Mongkolsapaya et al., J. Immunol., 160:3-6 (1998)] and DCR2 (also called TRUNDD or TRAIL-R4) [Marsters et al., Curr. Biol., 7:1003-1006 (1997); Pan et al., FEBS Letters, 424:41-45 (1998); Degli-Esposti et al., Immunity, 7:813-820 (1997)], both cell surface molecules, as well as OPG [Simonet et al., supra] and DCR3 [Pitti et al., Nature, 396:699-703 (1998)], both of which are secreted, soluble proteins. Apo2L/TRAIL has been reported to bind those receptors referred to as DcR1, DcR2 and OPG.

Apo2L/TRAIL is believed to act through the cell surface "death receptors" DR4 and DR5 to activate caspases, or enzymes that carry out the intracellular cell death program. [See, e.g., Salvesen et al., Cell, 91:443-446 (1997)]. Upon ligand binding, both DR4 and DR5 can trigger apoptosis independently by recruiting and activating the apoptosis initiator, caspase-8, through the death-domain-containing adaptor molecule referred to as FADD/Mort1 [Kischkel et al., Immunity, 12:611-620 (2000); Sprick et al., Immunity, 12:599-609 (2000); Bodmer et al., Nature Cell Biol., 2:241-243 (2000)]. In contrast to DR4 and DR5, the DcR1 and DcR2 receptors do not signal apoptosis.

For a review of the TNF family of cytokines and their receptors, see Ashkenazi and Dixit, Science, 281:1305-1308 (1998); Ashkenazi and Dixit, Curr. Opin. Cell Biol., 11:255-260 (2000); Golstein, Curr. Biol., 7:750-753 (1997); Gruss and Dower, supra, and Nagata, Cell, 88:355-365 (1997); Locksley et al., Cell, 104:487-501 (2001); Wallach, "TNF Ligand and TNF/NGF Receptor Families", Cytokine Research, Academic Press, pages 377-411 (2000).

While zinc binding sites have been shown to play structural roles in protein-protein interactions in certain proteins involving diverse interfaces [Feese et al., Proc. Natl. Acad. Sci., 91:3544-3548 (1994); Somers et al., Nature, 372:478-481 (1994); Raman et al., Cell, 95:939-950 (1998)], none of the previously structurally-characterized members of the TNF family (CD40 ligand, TNF-alpha, or TNF-beta) bind metals. The use of metal ions, such as zinc, in formulations of various hormones, such as human growth hormone (hGH), has been described in the literature. [See, e.g., WO 92/17200 published October 15, 1992). Zinc involvement in hGH binding to receptors was likewise described in WO 92/03478 published March 5, 1992. The roles of zinc binding in interferon-alpha dimers and interferon-beta dimers were reported in Walter et al-, Structure, 4:1453-1463 (1996) and Karpusas et al., Proc. Natl. Acad. Sci., 94:11813-11818 (1997), respectively. The structures and biological roles of various metal ions such as zinc have been reviewed in the art, see, e.g., Christianson et al., Advances in Protein Chemistry, 42:281-355 (1991).

### SUMMARY OF THE INVENTION

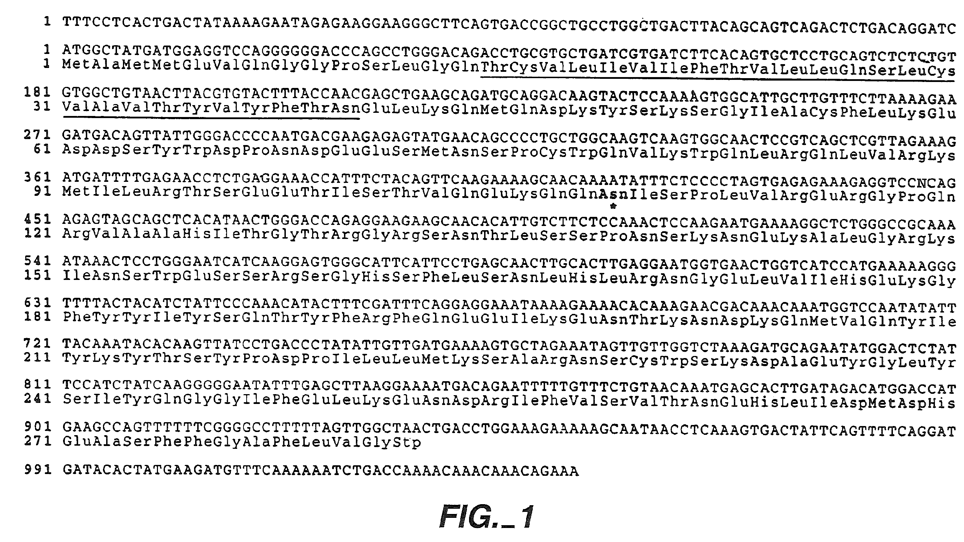

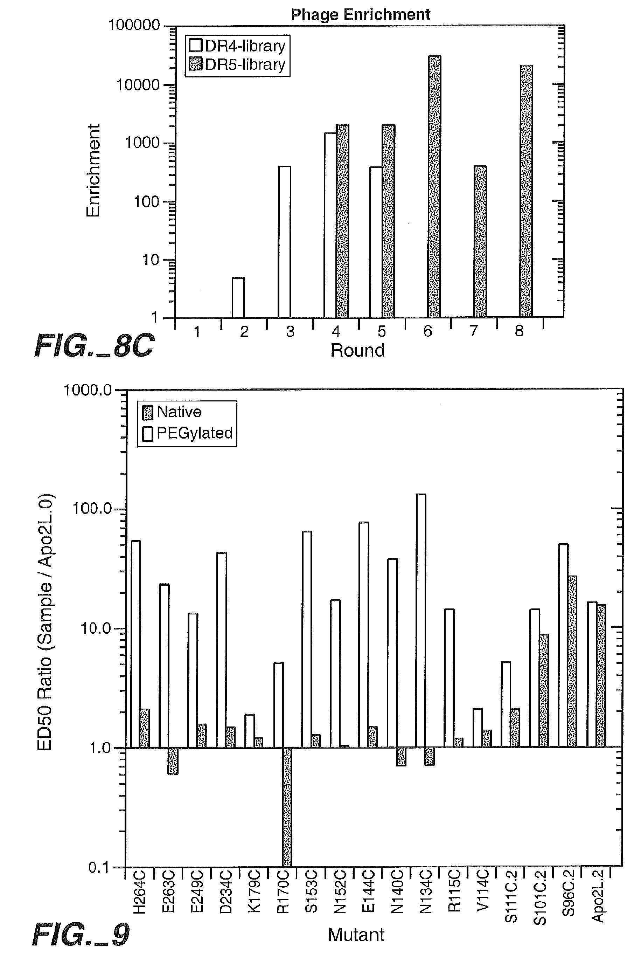

The present invention provides Apo-2 ligand/TRAIL variants. Particularly, the invention provides Apo-2 ligand/TRAIL variants comprising one or more amino acid substitutions in the native sequence of Apo-2 ligand/TRAIL shown in Figure 1. Optionally, the Apo-2 ligand/TRAIL variants may comprise cysteine substitutions, such as those identified in Figure 9. A related embodiment of the invention includes the Apo-2 ligand/TRAIL variant polypeptides provided in Figure 9 that are conjugated or linked to one or more polyol groups such as poly(ethylene glycol) 2000. The invention also provides nucleic acid molecules encoding such Apo-2 ligand/TRAIL variants and vectors and host cells containing nucleic acid molecules encoding the Apo-2 ligand/TRAIL variants.

The present invention also provides Apo-2 ligand/TRAIL variants comprising substitutions, such as those provided in Tables II, III, VII and VIII below that alter their DR4 receptor and/or DR5 receptor binding properties. Applicants surprisingly found that Apo-2 ligand/TRAIL variants such as those provided in Tables II, III, VII and VIII below exhibited altered binding affinities with respect to the DR4 and/or DR5 receptors (as compared to native Apo-2 ligand shown in Figure 1) and further exhibited selective binding affinity for the DR4 receptor or DR5 receptor. The invention also provides nucleic acid molecules encoding such Apo-2 ligand/TRAIL variants and vectors and host cells containing nucleic acid molecules encoding the Apo-2 ligand/TRAIL variants.

A further embodiment of the invention provides articles of manufacture and kits that include such Apo-2 ligand/TRAIL variants. The articles of manufacture and kits include a container, a label on the container, and an agent contained within the container. The label on the container indicates that the agent (or a formulation containing the agent) can be used for certain therapeutic or non-therapeutic applications. The agent contains one or more of the Apo-2 ligand/TRAIL variants disclosed herein.

In addition, therapeutic and non-therapeutic methods for using the Apo-2 ligand/TRAIL variant polypeptides disclosed herein are provided. Embodiments of the invention include methods of inducing apoptosis in mammalian cells comprising exposing mammalian cells expressing a receptor selected from the group consisting of DR4 receptor and DR5 receptor to a therapeutically effective amount of isolated Apo-2 ligand variant polypeptide. Further embodiments of the invention include methods of treating cancer in a mammal, comprising administering to said mammal an effective amount of isolated Apo-2 ligand variant. Optionally, in the methods, the cancer is lung cancer, breast cancer, glioma, colon cancer or colorectal cancer. Further embodiments of the invention include methods of treating an immune-related disease in a mammal comprising administering to said mammal an effective amount of isolated Apo-2 ligand variant polypeptide. Optionally, in the methods, the immune-related disease is arthritis or multiple sclerosis.

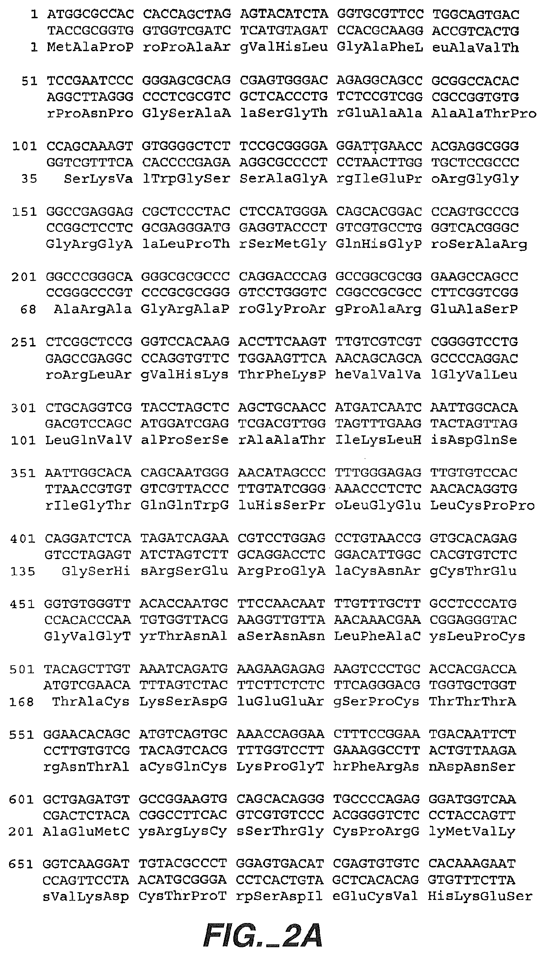

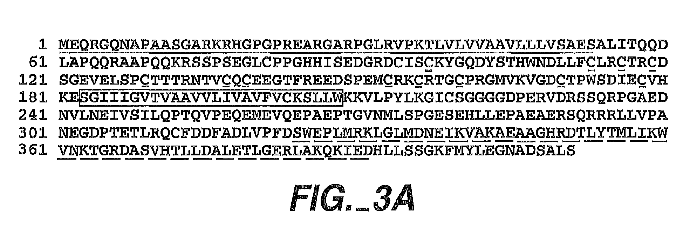

Various embodiments of the invention are described further below:
1. An isolated Apo-2 ligand variant polypeptide comprising an amino acid sequence which differs from the native sequence Apo-2 ligand polypeptide sequence of Figure 1 (SEQ ID NO:1) and has one or more of the following amino acid substitutions at the residue position(s) in Figure 1 (SEQ ID NO:1): S96C; S101C; S111C; R170C; K179C.
2. An isolated Apo-2 ligand variant polypeptide comprising one or more amino acid mutations in the amino acid sequence of native Apo-2 ligand polypeptide sequence of Figure 1 (SEQ ID NO:1), said mutations comprising one or more amino acid substitutions recited in Table II.
3. The Apo-2 ligand variant polypeptide of claim 2 wherein said Apo-2 ligand variant polypeptide has selective binding affinity for DR4 receptor.
4. The Apo-2 ligand variant polypeptide of claim 2 wherein said Apo-2 ligand variant polypeptide induces apoptosis in at least one type of mammalian cell.
5. The Apo-2 ligand variant polypeptide of claim 4 wherein said mammalian cell is a cancer cell.
6. The Apo-2 ligand variant polypeptide of claim 3 wherein said DR4 receptor comprises amino acids 1 to 218 of Fig. 2A-2B (SEQ ID NO:3).
7. The Apo-2 ligand variant polypeptide of claim 2 wherein said one or more amino acid mutations comprises one or more amino acid substitutions at positions 189, 193, 199, or 201 of the native Apo-2 ligand sequence.
8. The Apo-2 ligand variant polypeptide of claim 2 wherein said Apo-2 ligand variant polypeptide retains native residues at positions corresponding to Arg149, Gln205, Val207, Tyr216, Glu236 and/or Tyr237.
9. An isolated Apo-2 ligand variant polypeptide comprising an amino acid sequence which differs from the native sequence Apo-2 ligand polypeptide sequence of Figure 1 (SEQ ID NO:1) and has a set of amino acid substitutions at the residue position(s) in Figure 1 (SEQ ID NO:1) selected from the group consisting of:
   Y189A:R191K:Q193K,
   Y189A:R191K:Q193K:H264A,
   Y189Q:R191K:Q193R:H264R:I266L:D267Q,
   Y189A:R191K:Q193K:H264D:I266L:D267Q:D269E, and
   Y189A:R191K:Q193R:H264S:I266L:D269E.
10. An isolated Apo-2 ligand variant polypeptide comprising one or more amino acid mutations in the amino acid sequence of native Apo-2 ligand polypeptide sequence of Figure 1 (SEQ ID NO:1), said mutations comprising one or more amino acid substitutions recited in Table III.
11. The Apo-2 ligand variant polypeptide of claim 10 wherein said Apo-2 ligand variant polypeptide has selective binding affinity for DR5 receptor.
12. The Apo-2 ligand variant polypeptide of claim 10 wherein said Apo-2 ligand variant polypeptide induces apoptosis in at least one type of mammalian cell.
13. The Apo-2 ligand variant polypeptide of claim 12 wherein said mammalian cell is a cancer cell.
14. The Apo-2 ligand variant polypeptide of claim 11 wherein said DR5 receptor comprises amino acids 1 to 184 of Fig. 3A (SEQ ID N0:4).
15. The Apo-2 ligand variant polypeptide of claim 10 wherein said one or more amino acid mutations comprises one or more amino acid substitutions at positions 189, 191, 193, 264, 266, 267, or 269 of the native Apo-2 ligand sequence.
16. The Apo-2 ligand variant polypeptide of claim 10 wherein said Apo-2 ligand variant polypeptide retains native residues at positions corresponding to Arg149, Gln205, Val207, Tyr216, Glu236 and/or Tyr237.
17. An isolated Apo-2 ligand variant polypeptide comprising one or more amino acid mutations in the amino acid sequence of native Apo-2 ligand polypeptide sequence of Figure 1 (SEQ ID NO:1), said mutations comprising one or more amino acid substitutions recited in Table VII.
18. The Apo-2 ligand variant polypeptide of claim 17 wherein said Apo-2 ligand variant polypeptide has selective binding affinity for DR5 receptor.
19. The Apo-2 ligand variant polypeptide of claim 17 wherein said Apo-2 ligand variant polypeptide induces apoptosis in at least one type of mammalian cell.
20. The Apo-2 ligand variant polypeptide of claim 19 wherein said mammalian cell is a cancer cell.
21. The Apo-2 ligand variant polypeptide of claim 18 wherein said DR5 receptor comprises amino acids 1 to 184 of Fig. 3A (SEQ ID NO:4).
22. An isolated Apo-2 ligand variant polypeptide comprising an amino acid sequence which differs from the native sequence Apo-2 ligand polypeptide sequence of Figure 1 (SEQ ID NO:1) and has a set of amino acid substitutions at the residue position(s) in Figure 1 (SEQ ID NO:1) selected from the group consisting of:
   Y189Q:R191K:Q193R; H264R; I266L; D267Q;
   Y189Q:R191K:Q193R; and
   Y189Q:R191K:Q193R:I266L.
23. The Apo-2 ligand variant polypeptide of any of claims 1-22 wherein said polypeptide is conjugated or linked to one or more polyols.
24. The Apo-2 ligand variant polypeptide of claim 23 wherein said polyol is polyethylene glycol.
25. The Apo-2 ligand variant polypeptide of claim 24 wherein said polyethylene glycol has an average molecular weight of about 1000 daltons to about 25,000 daltons.
26. An isolated nucleic acid molecule comprising DNA encoding the Apo-2 ligand variant polypeptide of any of claims 1-22.
27. A vector comprising the encoding DNA of claim 26.
28. A host cell comprising the vector of claim 27.
29. The host cell of claim 28 wherein said host cell is an E. coli cell, CHO cell or yeast cell.
30. A method of producing Apo-2 ligand variant polypeptide comprising culturing the host cell of claim 28 under conditions sufficient to express said Apo-2 ligand variant polypeptide and recovering said Apo-2 ligand variant polypeptide from said culture.
31. A composition comprising the Apo-2 ligand variant polyeptide of any of claims 1-25.
32. The composition of claim 31 wherein said composition comprises a therapeutically acceptable formulation which contains one or more divalent metal ions.
33. A method of inducing apoptosis in mammalian cells comprising exposing mammalian cells expressing DR4 and/or DR5 receptor to an effective amount of Apo-2 ligand variant polypeptide of any of claims 1-25.
34. A method of treating cancer comprising exposing mammalian cancer cells to an effective amount of Apo-2 ligand variant polypeptide of any of claims 1-25.
35. The method of claim 34 wherein said mammalian cancer cells comprise lung cancer cells, breast cancer cells, glioma cancer cells, or colon or colorectal cancer cells.
36. The method of claim 34 wherein said method further comprises exposing said mammalian cancer cells to a prodrug, cytotoxic agent, chemotherapeutic agent, growth inhibitory agent, or cytokine.
37. A method of treating an immune-related disease in a mammal, comprising administering to said mammal an effective amount of Apo-2 ligand variant polypeptide of any of claims 1-25.
38. The method of claim 37 wherein said immune-related disease is arthritis or multiple sclerosis.

### BRIEF DESCRIPTION OF THE DRAWINGS

Figure 1 shows the nucleotide sequence of human Apo-2 ligand cDNA (SEQ ID NO:2) and its derived amino acid sequence (SEQ ID NO:1). The "N" at nucleotide position 447 (in SEQ ID NO:2) is used to indicate the nucleotide base may be a "T" or "G".

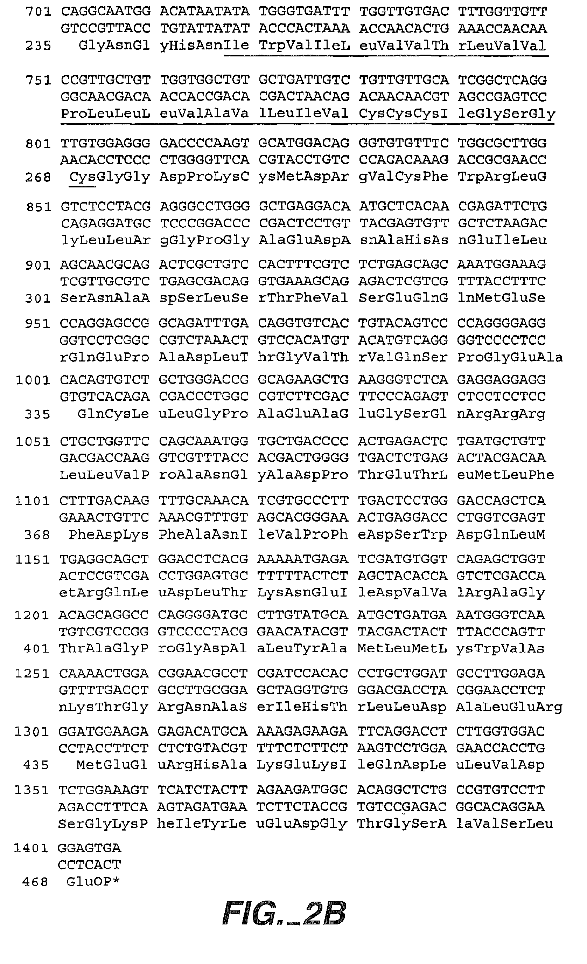

Figures 2A and 2B show the nucleotide sequence of a cDNA (SEQ ID NO:4) for full length human DR4 receptor and its derived amino acid sequence (SEQ ID NO:3). The respective nucleotide and amino acid sequences for human DR4 receptor are also reported in Pan et al., Science, 276:111 (1997).

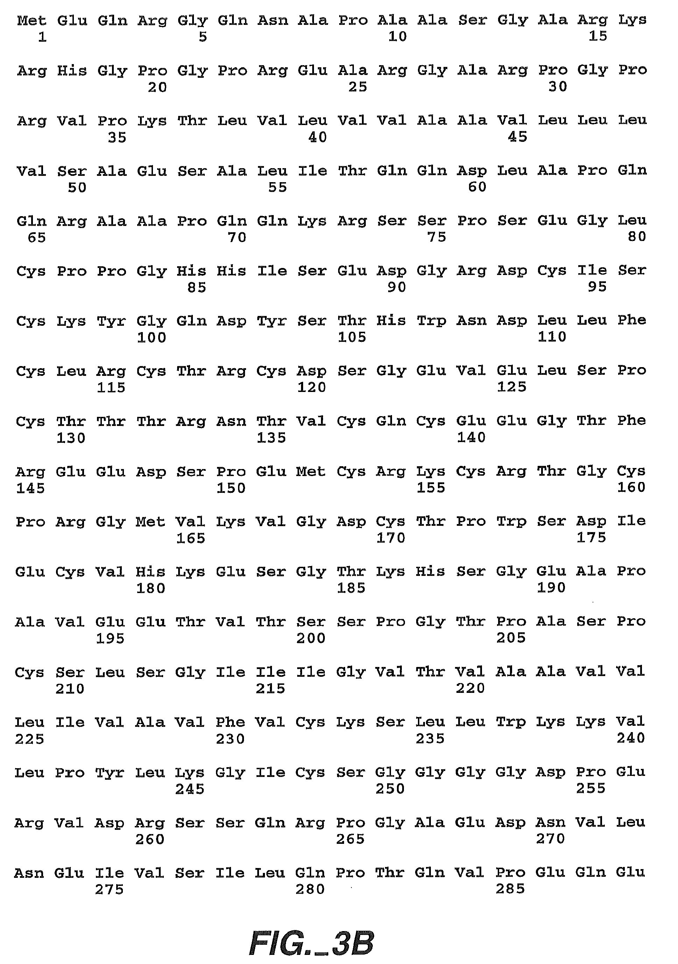

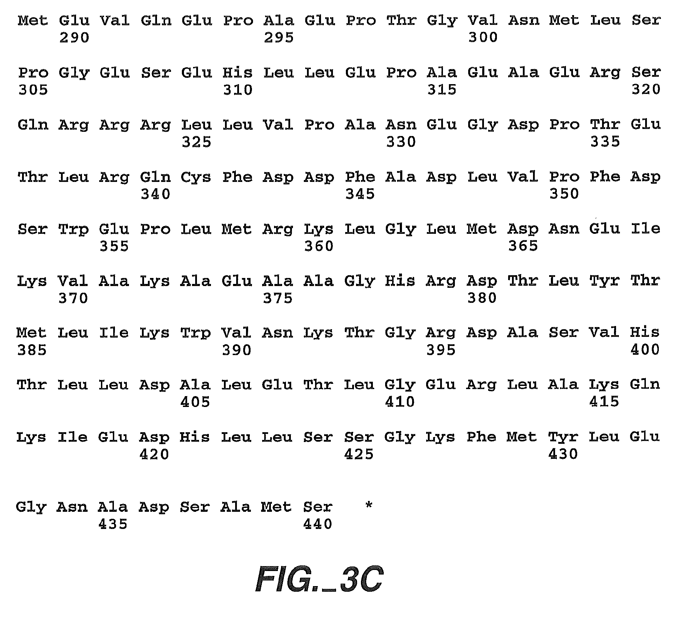

Figure 3A shows the 411 amino acid sequence of human DR5 receptor (SEQ ID NO:5) as published in WO 98/51793 on November 19, 1998. Another form of DR5 receptor is a 440 amino acid sequence of human DR5 (SEQ ID NO:6) shown in Figures 3B and 3C, as also published in WO 98/35986 on August 20, 1998.

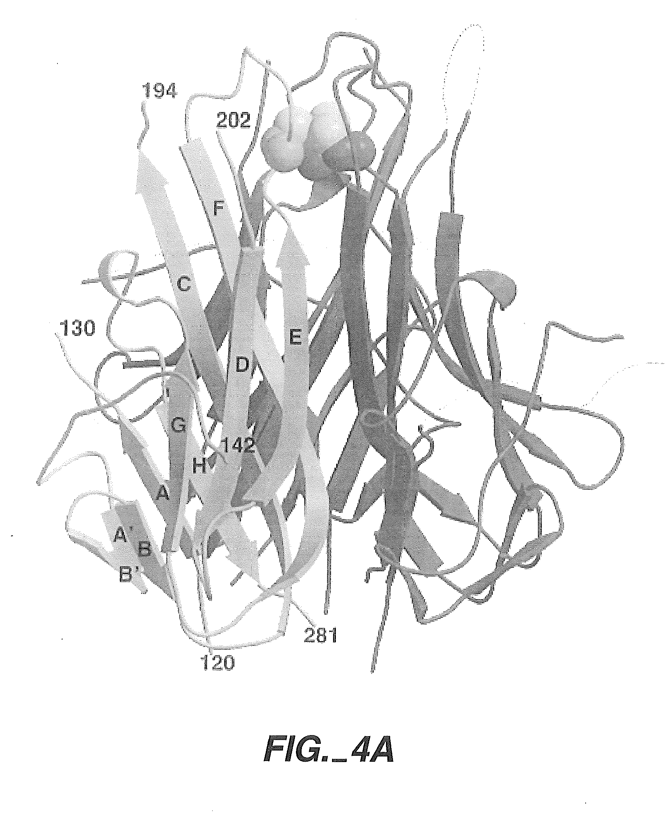

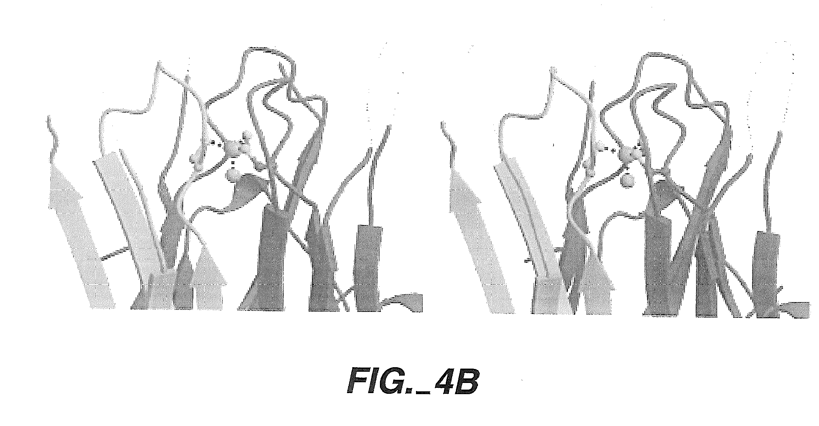

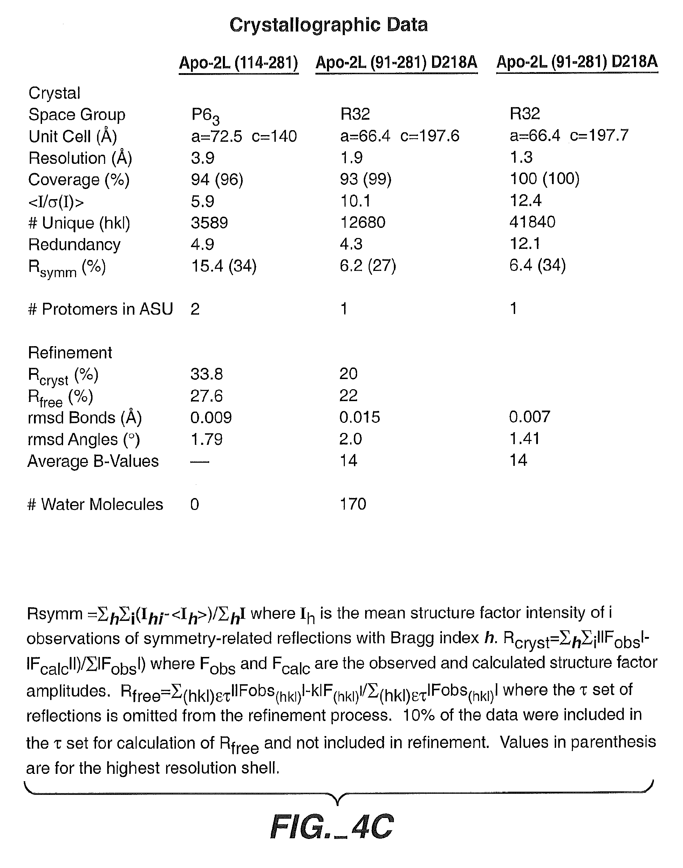

Figure 4 provides the crystal structure of Apo-2L. Figure 4A shows a view of the trimer along the three-fold axis. Each monomer is identical. The ordered protein structure commences at residue 120, residues 131-141 are disordered, as are residues 195-201 (marked as dashed lines). The zinc binding site including the three symmetry related cysteines and the solvent ligand are shown as space filling diagrams. Figure 4B provides cross-eyed stereo close up view of the zinc binding site; the angles between Sy-zinc-Sy are 112° and the Sy-zinc-solvent angles are 107° with 2.3 Angstrom zinc-sulfur and 2.3 Angstrom zinc-solvent bond distances. Figure 4 was made with the programs Molscript [Kraulis et al., J. Appl. Cryst., 24:946-950 (1991)] and Raster3D [Merrit et al., Acta Cryst., D50:869-873 (1994)). Figure 4C provides a summary of the crystallographic data.

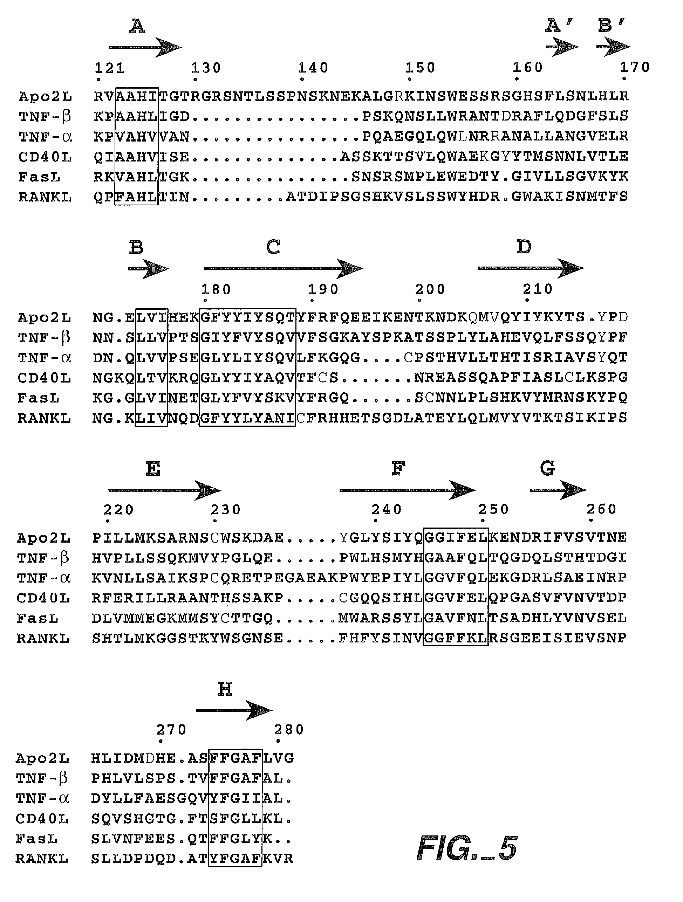

Figure 5 shows a sequence alignment of selected TNF family members: Apo2L (SEQ ID NO:7); TNF-beta (SEQ ID NO:8); TNF-alpha (SEQ ID NO:9); CD40L (SEQ ID NO:10); FasL (SEQ ID NO:11); RANKL (SEQ ID NO:12). Arrows over the sequence indicate beta-strands in Apo2L. The numbering over the aligned sequences corresponds to the Apo2L sequence numbering provided in Figure 1 (SEQ ID NO:1).

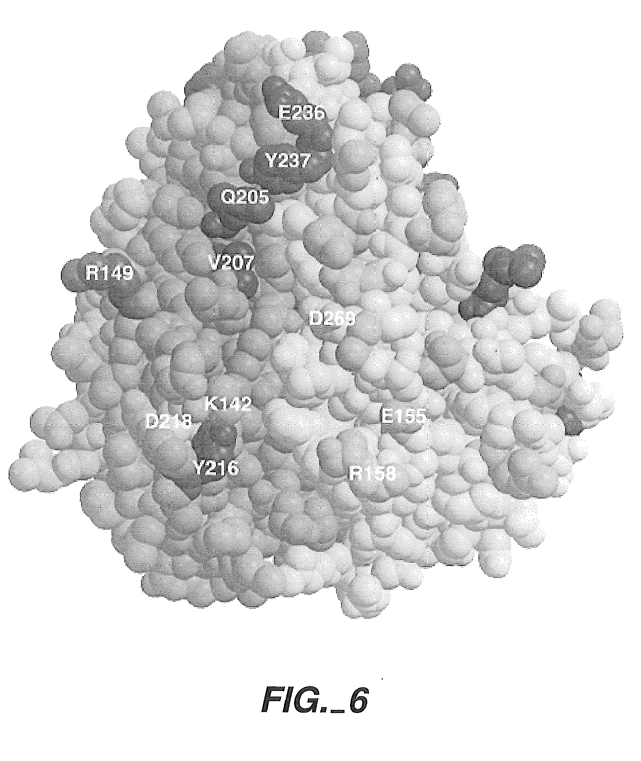

Figure 6 shows mutational analysis mapped onto a space-filling model of Apo-2L. Residues with a greater than 5-fold decrease in bioactivity when mutated to alanine are labeled and darkly shaded. Other residues that have been mutated are shown in medium shading and a few of these residues are also labeled.

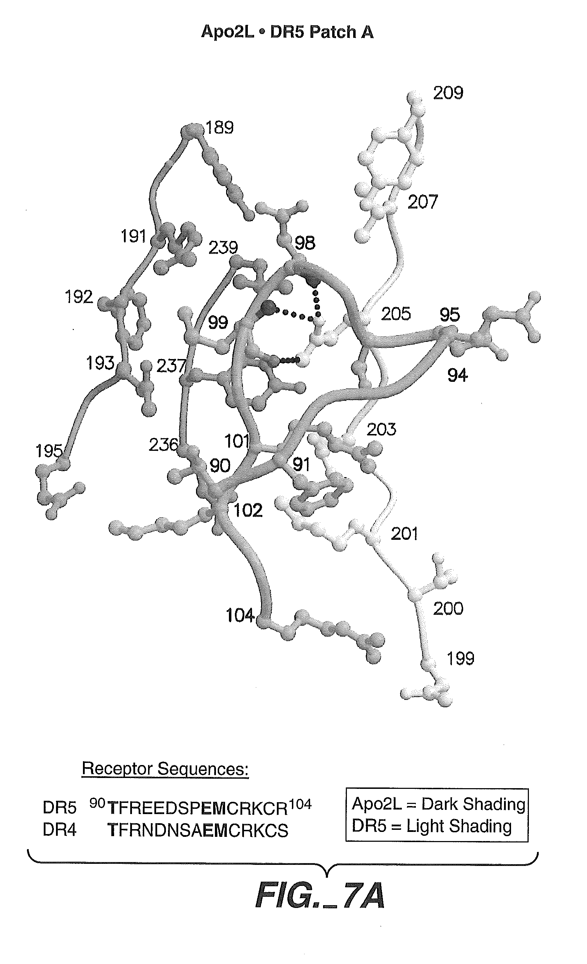

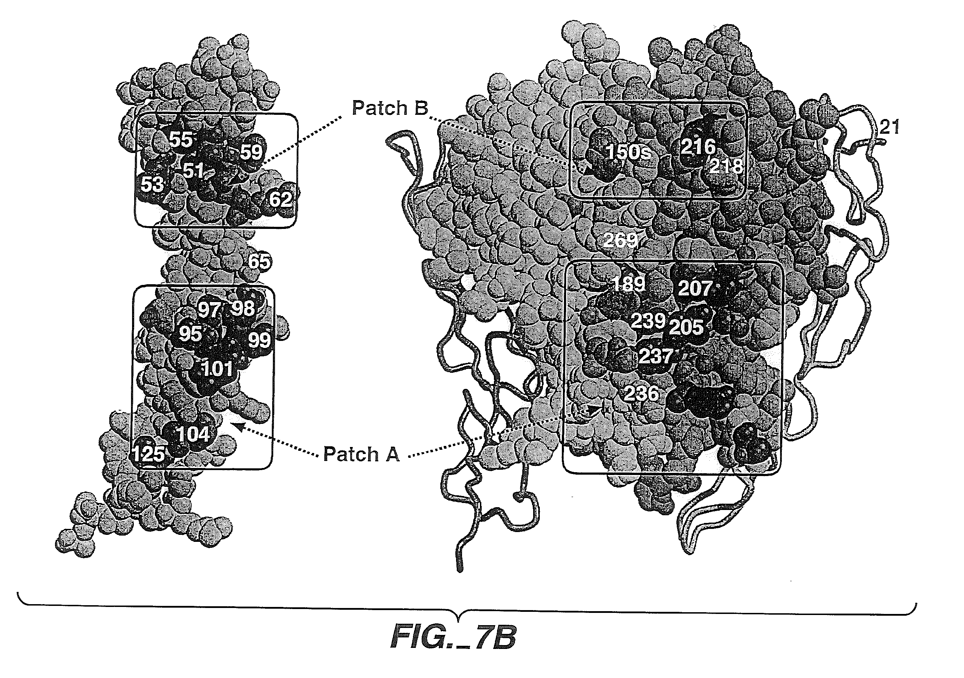

Figure 7A shows the "Patch A" contact observed in x-ray crystal structure of Apo2L·DR5 complex (Hymowitz et al., (1999) Mol. Cell. 4, 563). Backbone trace and selected side chains of Apo2L are shown by dark shading. Backbone trace and selected side chains of DR5 are shown in light shading. The receptor sequence illustrated for DR5 recites amino acids 143-157 of SEQ ID NO:5, and the receptor sequence illustrated for DR4 recites amino acids 194-208 of SEQ ID NO:3. Figure 7B provides another view of the x-ray structure determined for the Apo2L·DR5-ECD complex.

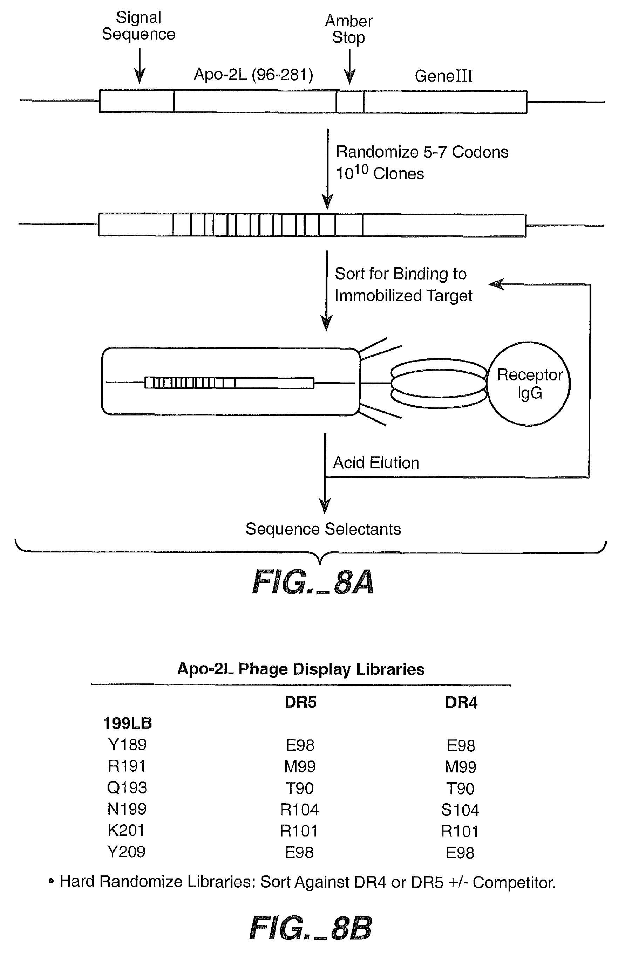

Figure 8A provides a schematic representation of a monovalent phage display used for the identification of Apo-2L variants. Figure 8B provides illustrative embodiments of the Apo-2L phage display libraries. Figure 8C provides a graphic representation of the enrichment of phage libraries for specific binding to DR4 (gray bars) or DR5 (black bars). Enrichment at each round was calculated as the ratio of phage eluted from a receptor coated well to that eluted from a blank well. The DR4 library was subjected to 5 rounds of sorting for binding to DR4-IgG with competitor DR5-IgG included in rounds 3-5. The DR5 library was subjected to 8 rounds of sorting for binding to DR5-IgG with competitor DR4-IgG included in rounds 5-8.
Figure 9 provides a graphic representation of the ED50 ratios of various native and pegylated Apo-2L variants having cysteine substitutions at the enumerated residues.

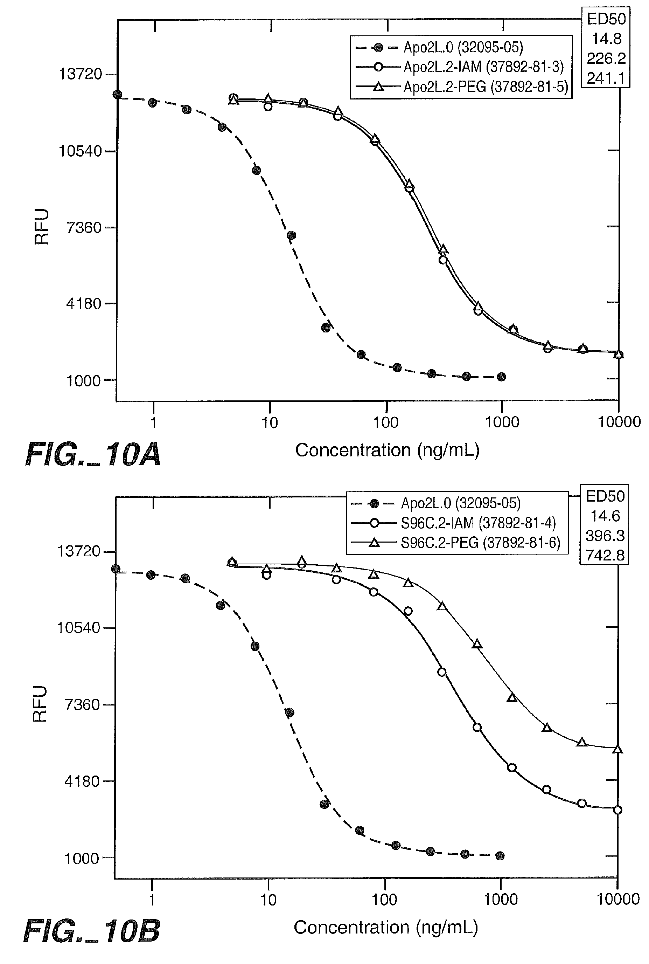

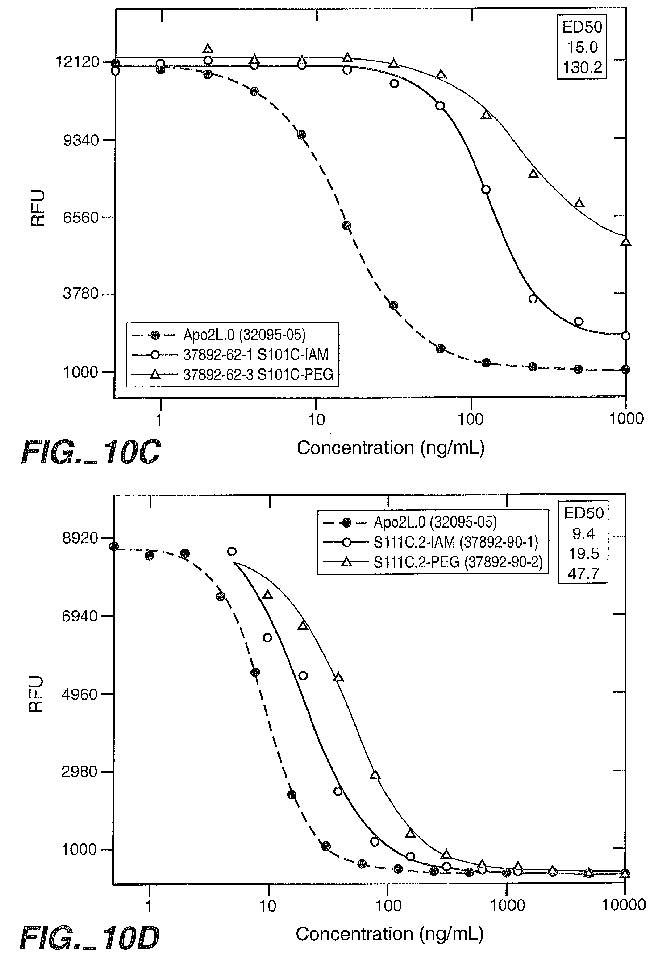

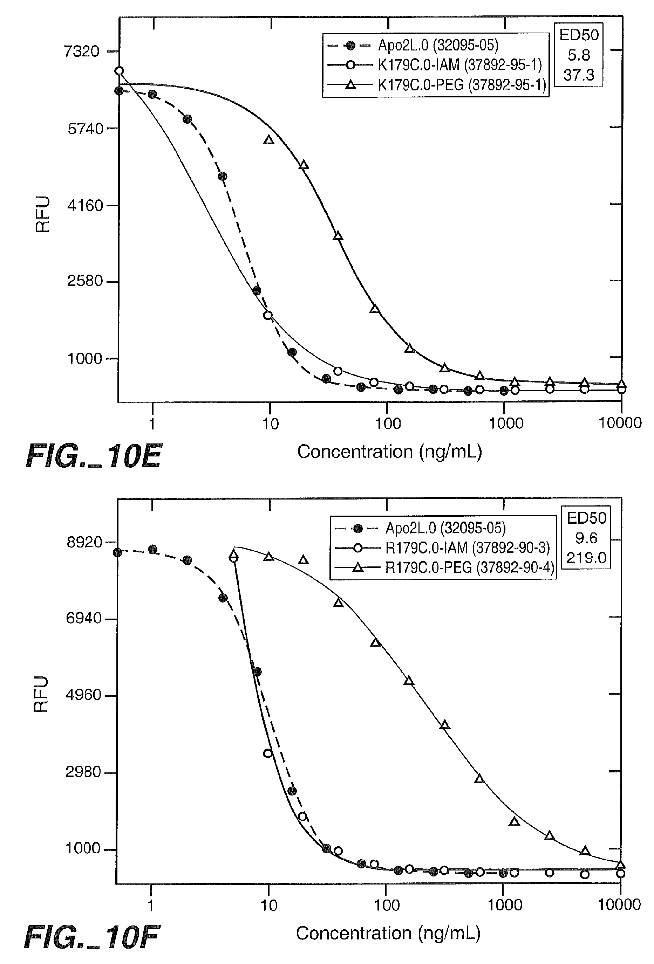

Figures 10A-10G show the apoptosis-inducing activity of Apo-2L and a variety of Apo-2L cysteine substitution variants modified with moieties such as polyethylene glycol (PEG) and iodoacetamide (IAM). The apoptosis-inducing activity of these Apo-2L molecules was assessed using SK-MES lung carcinoma cells and alamar blue assays. In these figures "Apo2L.0" refers to the Apo-2 ligand consisting of amino acids 114-281 of SEQ ID NO:1, "Apo2L.2" refers to the Apo-2 ligand consisting of amino acids 91-281 of SEQ ID NO:1, "K179C.0-PEG" refers to a K179C substitution variant (i.e., a 114-281 amino acid form of Apo2L having a cysteine substituted at position 179 in the sequence) having a 2000 MW PEG moiety attached at this residue, "K179C.0-IAM" refers to a K179C substitution variant having an iodoacetamide moiety attached at this residue, "R170C-5Kp" refers to a partially pegylated R170C substitution variant having a 5000 MW PEG moiety attached at this residue, and "R170C-20Kp" refers to a partially pegylated R170C substitution variant having a 20,000 MW PEG moiety attached at this residue.

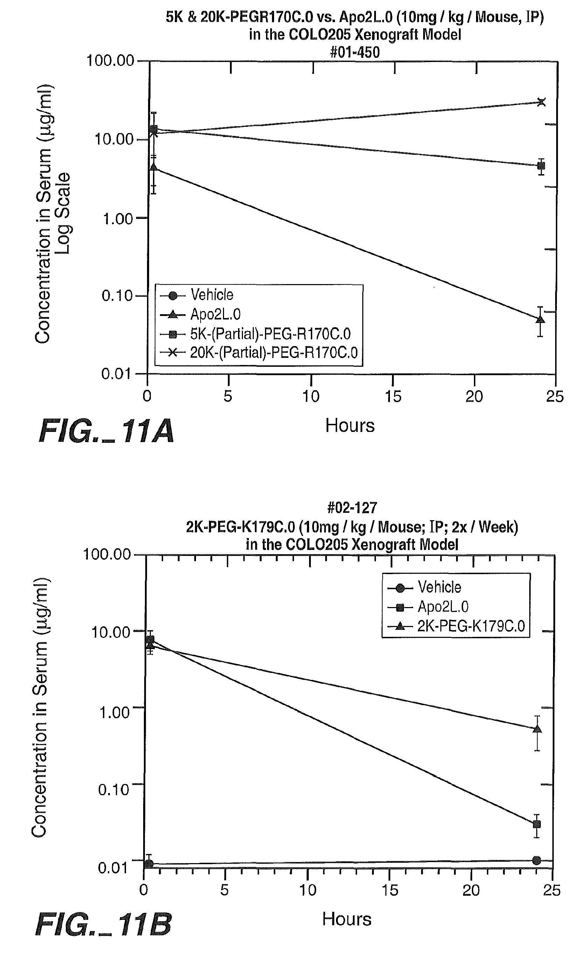

Figures 11A and 11B show the pharmacokinetics of partially pegylated 5K and 20K PEG-R170C or 2K-PEG-K179C and Apo2L.0 in the mouse. Mice were given i.p. (intraperitoneal) injections of Apo2L.0 (10 mg/kg) or PEG-R170C-Apo2L.0 (10 mg/kg) or 2K-PEG-K179C at time zero. These data show that the partially pegylated variants have a longer half-life than Apo2L.0.

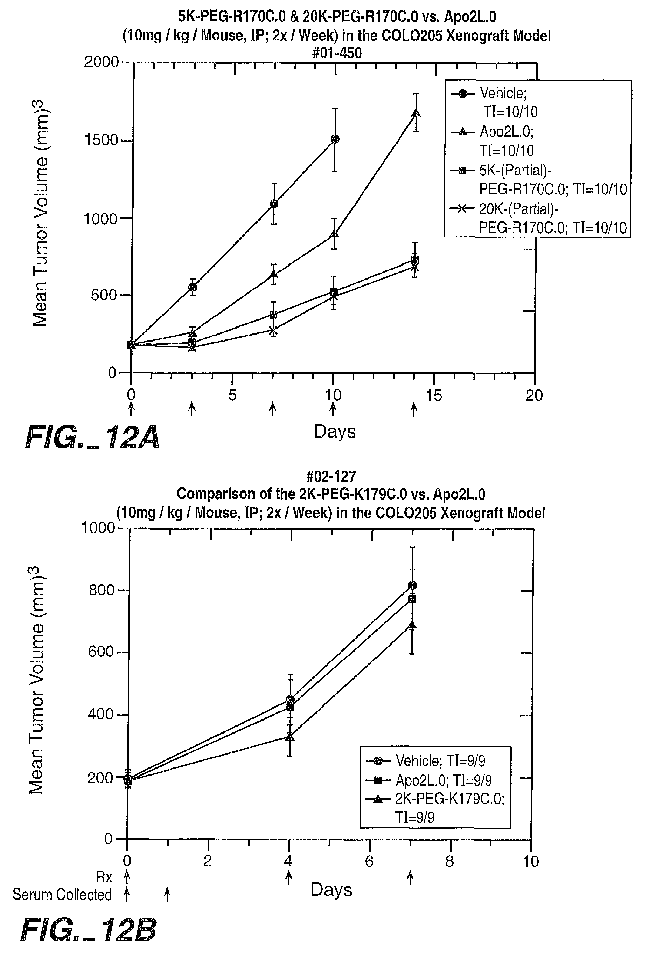

Figures 12A and 12B show the effect of partially pegylated Apo-2L cysteine substitution variants on the growth of human COL0205 tumors in a mouse xenograft model. Athymic nude mice (Jackson Laboratories) were injected subcutaneously with 5 x 10⁶ COLO205 human colon carcinoma cells (NCI). Tumors were allowed to form and grow to a volume of about 500-1500 mm³ as judged by caliper measurement. Mice were given i.p. injections of vehicle (2x/week), Apo2L.0 (10 mg/kg, 2x/week), or pegylated Apo-2L variants (10 mg/kg, 2x/week). Tumor volume was measured every third day and treatment was stopped after two weeks.

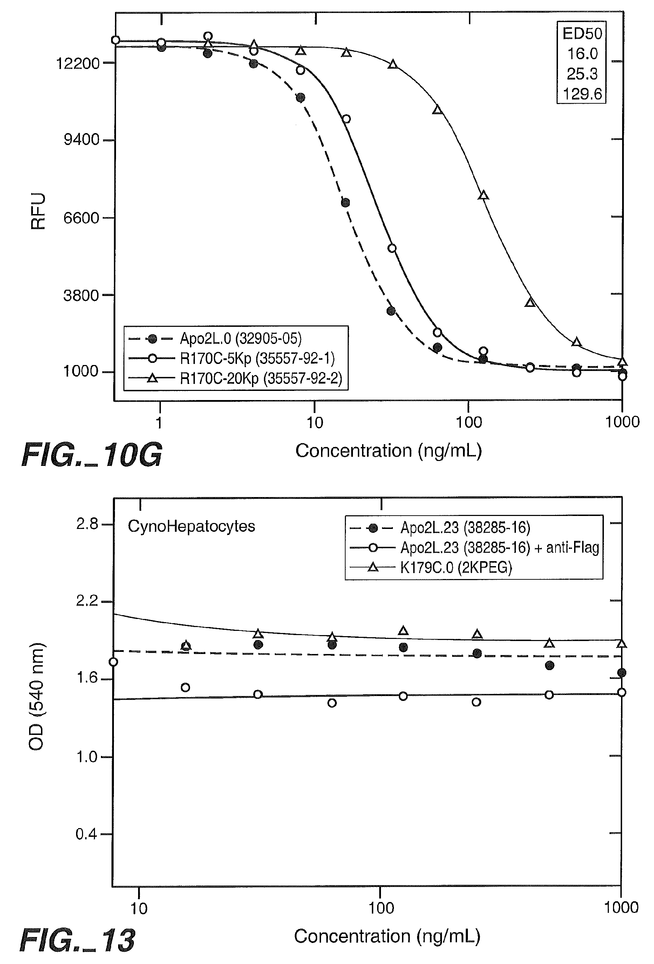

Figure 13 shows the effects of Apo-2L and K179C-Apo2L.0 on cynomologous monkey hepatocytes in a crystal violet assay.

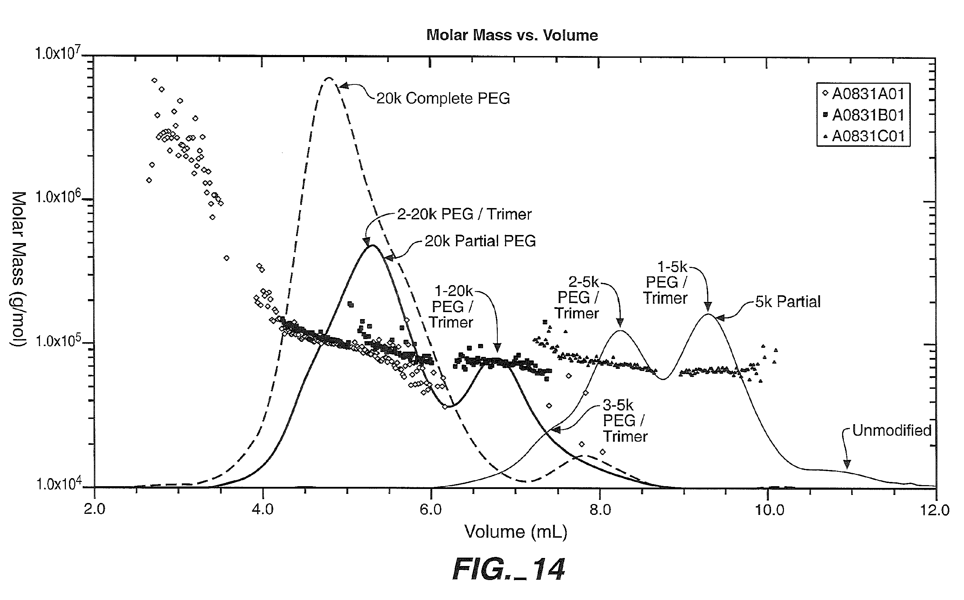

Figure 14 shows an analysis of R170C-Apo2L that had been partially PEGylated with either a 5K or 20 K PEG-maleimide by size exclusion chromatography (SEC) on a Superdex 200 column (Amersham Biotech) using a chromatographic system equipped with an on-line light scattering detector (MALS) (Wyatt Technology, Inc.). Solid lines represent the UV trace and symbols indicate the molar mass calculated from the light scattering data.

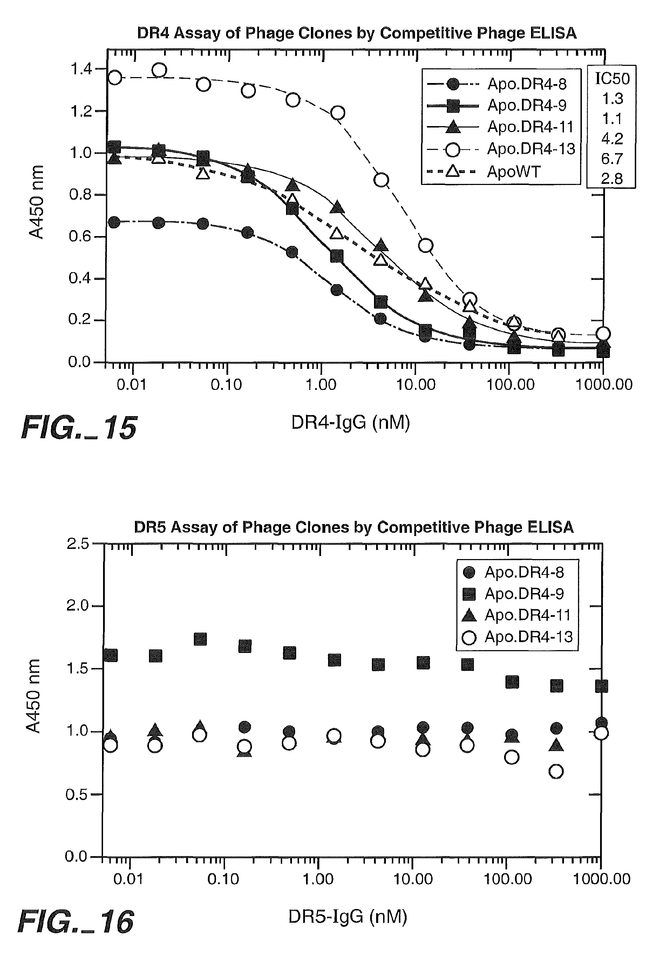

Figure 15 shows the results of a competition phage ELISA with DR4 library clones using DR4-IgG. Phage ELISA utilized microtiter plate wells coated with DR4-IgG and an anti-M13 antibody-HRP conjugate to detect bound phage. Binding was competed by increasing solution concentrations of DR4-IgG.
Figure 16 shows the results of a competition phage ELISA with DR4 library clones using DRS-IgG. Phage ELISA utilized microtiter plate wells coated with DR4-IgG and an anti-M13 antibody-HRP conjugate to detect bound phage. Binding was competed by increasing solution concentrations of DR5-IgG.

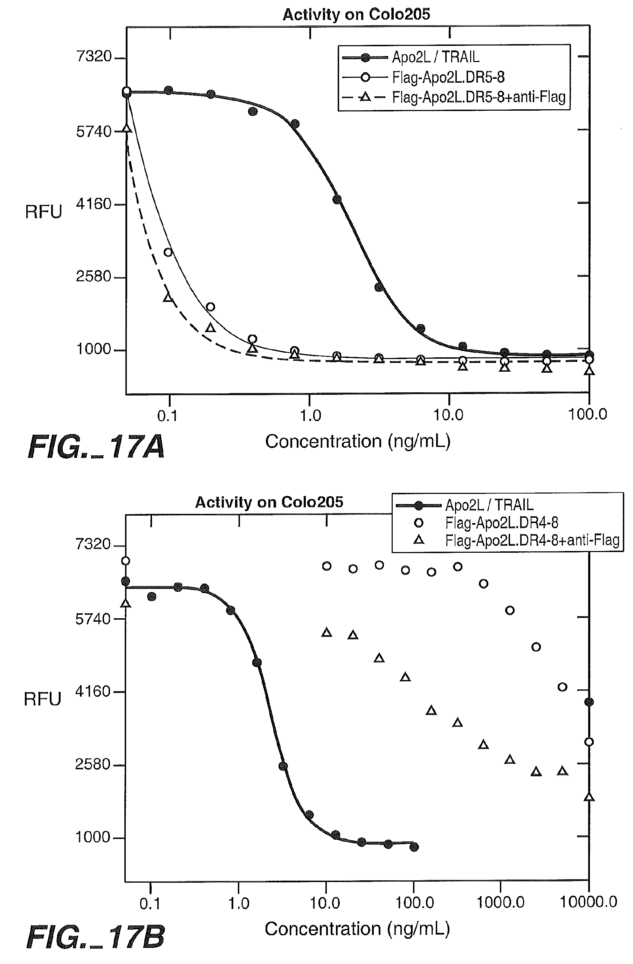

Figure 17 shows an assay of apoptosis-induction on Colo205 colon carcinoma cells by flag-tagged Apo2L/TRAIL mutants. A fluorescence assay described in Hymowitz et al., (2000) Biochemistry 39, 633-640 was used to test DR5-selective (panel A) or DR4-selective (panel B) mutants. "antiFlag" indicates that 2 µg/mL M2 antibody (Sigma) was added along with the specified concentration of Apo2L/TRAIL mutant. Curves represent fitting using the 4-parameter equation.

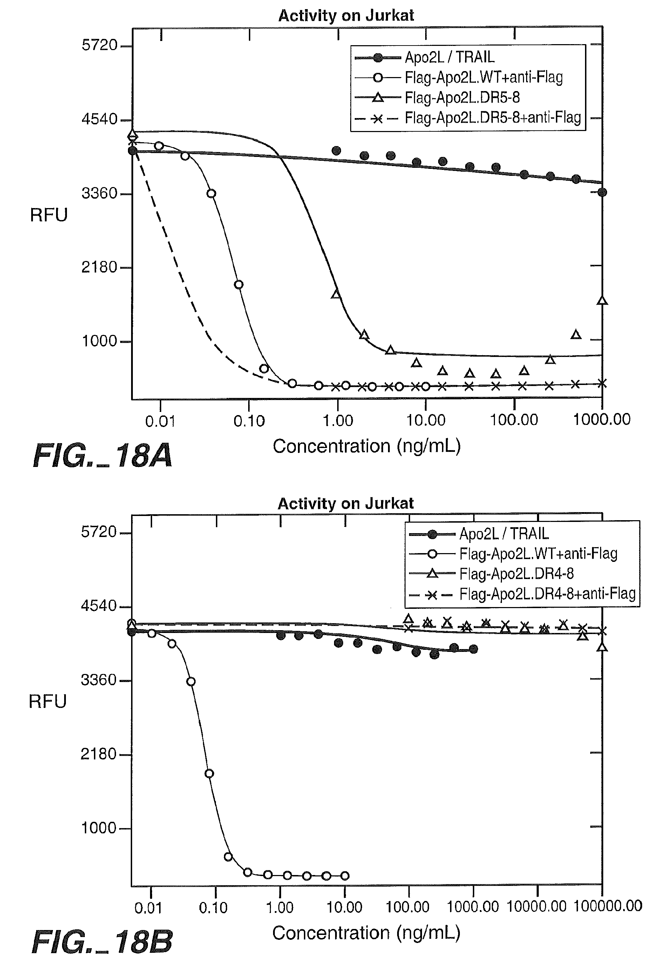

Figure 18 shows the apoptosis-induction on Jurkat cells by receptor-selective mutants (variants) of Apo2L/TRAIL. A fluorescence assay described in Hymowitz et al., (2000) Biochemistry 39, 633-640 was used to test DR5-selective (panel A) or DR4-selective (panel B) mutants. "antiFlag" indicates that 2 µg/mL M2 antibody (Sigma) was added along with the specified concentration of Apo2L/TRAIL variant. Curves represent fitting using the 4-parameter equation.

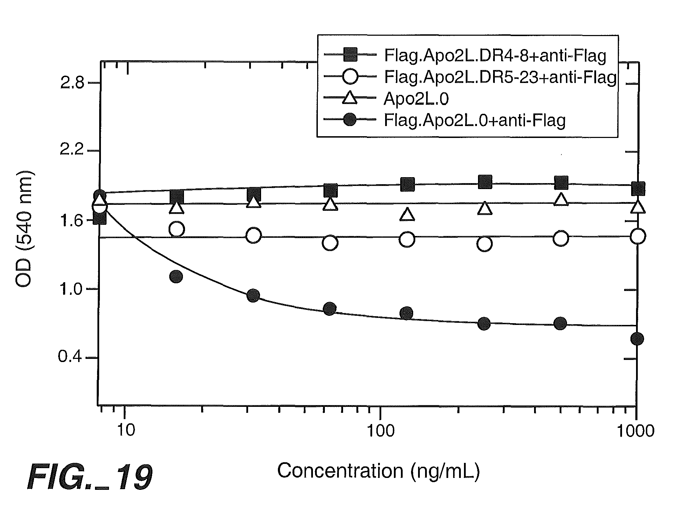

Figure 19 shows the viability of cynomologous monkey hepatocytes in the presence of the indicated concentration of variant Apo2L/TRAIL and 2 µg/mL M2 antibody (Sigma). Crystal violet staining was used to measure hepatocyte viability.

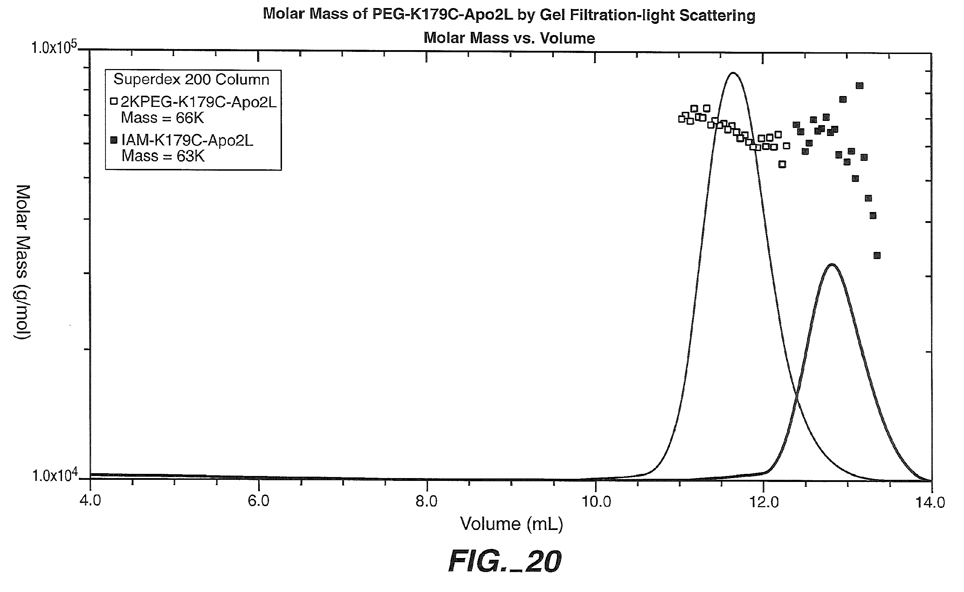

Figure 20 shows an analysis of 2KPEG-K179C-Apo2L and also carboxyamidomethylated K179C-Apo2L (IAM-K179C-Apo2L) by size exlusion chromatography (SEC) on a Superdex 200 column (Amersham Biotech) using a chromatographic system equipped with an on-line light scattering detector (MALS) (Wyatt Technology, Inc.). Solid lines represent the UV trace and the filled squares indicate the molar mass calculated from the light scattering data.

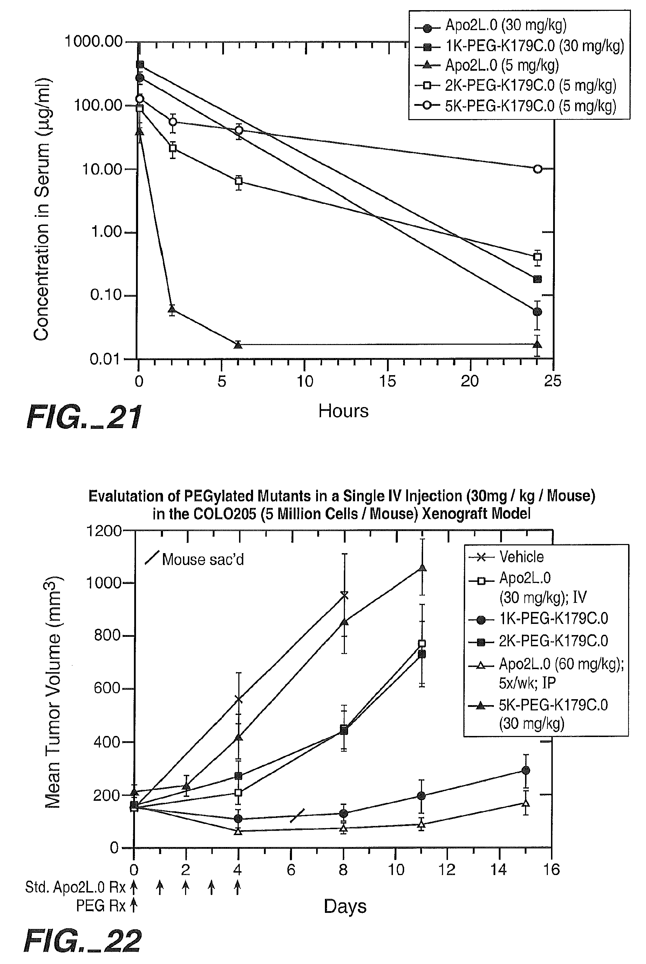

Figure 21 shows the pharmacokinetics of PEGylated K179C-Apo2L and Apo2L.0 in the mouse. Mice were given i.v. (intravenous) injections of 5 mg/kg or 30 mg/kg protein. Serum samples were collected at the indicated times and the Apo2L concentration was determined by ELISA.
Figure 22 shows the effects of PEGylated K179C-Apo2L on the growth of COLO205 tumors in a mouse xenograft model. Mice were given a single i.v. injection of 30 mg/kg Apo2L.0 or PEGylated K179C-Apo2L. An additional group of mice were given 5 i.p. injections of 60 mg/kg Apo2L.0 corresponding to the "standard" treatment regimen.

### DETAILED DESCRIPTION OF THE PREFERRED EMBODIMENTS

### I. Definitions

The terms "Apo-2 ligand", "Apo-2L", "Apo2L", Apo-2 ligand/TRAIL" and "TRAIL" are used herein interchangeably to refer to a polypeptide sequence which includes amino acid residues 114-281, inclusive, 95-281, inclusive, residues 92-281, inclusive, residues 91-281, inclusive, residues 41-281, inclusive, residues 39-281, inclusive, residues 15-281, inclusive, or residues 1-281, inclusive, of the amino acid sequence shown in Figure 1 (SEQ ID NO:1), as well as biologically active fragments, deletional, insertional, or substitutional variants of the above sequences. In one embodiment, the polypeptide sequence comprises residues 114-281 of Figure 1 (SEQ ID NO:1). Optionally, the polypeptide sequence comprises residues 92-281 or residues 91-281 of Figure 1 (SEQ ID NO:1). The Apo-2L polypeptides may be encoded by the native nucleotide sequence shown in Figure 1 (SEQ ID NO:2). Optionally, the codon which encodes residue Pro119 (Figure 1; SEQ ID NO:2) may be "CCT" or "CCG". Optionally, the fragments or variants are biologically active and have at least about 80% amino acid sequence identity, more preferably at least about 90% sequence identity, and even more preferably, at least 95%, 96%, 97%, 98%, or 99% sequence identity with any one of the above sequences. The definition encompasses substitutional variants of Apo-2 ligand in which at least one of its native amino acids are substituted by another amino acid such as an alanine residue. Optional substitutional variants include one or more of the residue substitutions identified in Figure 9 and Tables II, III, VII and VIII below. The definition also encompasses a native sequence Apo-2 ligand isolated from an Apo-2 ligand source or prepared by recombinant or synthetic methods. The Apo-2 ligand of the invention includes the polypeptides referred to as Apo-2 ligand or TRAIL disclosed in WO97/01633 published January 16, 1997, WO97/25428 published July 17, 1997, WO99/36535 published July 22, 1999, WO 01/00832 published January 4, 2001, WO02/09755 published February 7, 2002, and WO 00/75191 published December 14, 2000. The terms are used to refer generally to forms of the Apo-2 ligand which include monomer, dimer, trimer, hexamer or hight oligomer forms of the polypeptide. All numbering of amino acid residues referred to in the Apo-2L sequence use the numbering according to Figure 1 (SEQ ID NO:1), unless specifically stated otherwise. For instance, "D203" or "Asp203" refers to the aspartic acid residue at position 203 in the sequence provided in Figure 1 (SEQ ID NO:1).

The term "Apo-2 ligand selective variant" as used herein refers to an Apo-2 ligand polypeptide which includes one or more amino acid mutations in a native Apo-2 ligand sequence and has selective binding affinity for either the DR4 receptor or the DR5 receptor. In one embodiment, the Apo-2 ligand variant has a selective binding affinity for the DR4 receptor and includes one or more amino acid substitutions in any one of positions 189, 191, 193, 199, 201 or 209 of a native Apo-2 ligand sequence. In another embodiment, the Apo-2 ligand variant has a selective binding affinity for the DR5 receptor and includes one or more amino acid substitutions in any one of positions 189, 191, 193, 264, 266, 267 or 269 of a native Apo-2 ligand sequence.

Preferred Apo-2 ligand selective variants include one or more amino acid mutations and exhibit binding affinity to the DR4 receptor which is equal to or greater (≥) than the binding affinity of native sequence Apo-2 ligand to the DR4 receptor, and even more preferably, the Apo-2 ligand variants exhibit less binding affinity (<) to the DR5 receptor than the binding affinity exhibited by native sequence Apo-2 ligand to DR5. When binding affinity of such Apo-2 ligand variant to the DR4 receptor is approximately equal (unchanged) or greater than (increased) as compared to native sequence Apo-2 ligand, and the binding affinity of the Apo-2 ligand variant to the DR5 receptor is less than or nearly eliminated as compared to native sequence Apo-2 ligand, the binding affinity of the Apo-2 ligand variant, for purposes herein, is considered "selective" for the DR4 receptor. Preferred DR4 selective Apo-2 ligand variants of the invention will have at least 10-fold less binding affinity to DR5 receptor (as compared to native sequence Apo-2 ligand), and even more preferably, will have at least 100-fold less binding affinity to DR5 receptor (as compared to native sequence Apo-2 ligand). The respective binding affinity of the Apo-2 ligand variant may be determined and compared to the binding properties of native Apo-2L (such as the 114-281 form) by ELISA, RIA, and/or BIAcore assays, known in the art and described further in the Examples below. Preferred DR4 selective Apo-2 ligand variants of the invention will induce apoptosis in at least one type of mammalian cell (preferably a cancer cell), and such apoptotic activity can be determined by known art methods such as the alamar blue or crystal violet assay in the Examples. The DR4 selective Apo-2 ligand variants may or may not have altered binding affinities to any of the decoy receptors for Apo-2L, those decoy receptors being referred to in the art as DcR1, DcR2 and OPG.

Further preferred Apo-2 ligand selective variants include one or more amino acid mutations and exhibit binding affinity to the DR5 receptor which is equal to or greater (≥) than the binding affinity of native sequence Apo-2 ligand to the DR5 receptor, and even more preferably, such Apo-2 ligand variants exhibit less binding affinity (<) to the DR4 receptor than the binding affinity exhibited by native sequence Apo-2 ligand to DR4. When binding affinity of such Apo-2 ligand variant to the DR5 receptor is approximately equal (unchanged) or greater than (increased) as compared to native sequence Apo-2 ligand, and the binding affinity of the Apo-2 ligand variant to the DR4 receptor is less than or nearly eliminated as compared to native sequence Apo-2 ligand, the binding affinity of the Apo-2 ligand variant, for purposes herein, is considered "selective" for the DR5 receptor. Preferred DR5 selective Apo-2 ligand variants of the invention will have at least 10-fold less binding affinity to DR4 receptor (as compared to native sequence Apo-2 ligand), and even more preferably, will have at least 100-fold less binding affinity to DR4 receptor (as compared to native sequence Apo-2 ligand). The respective binding affinity of the Apo-2 ligand variant may be determined and compared to the binding properties of native Apo2L (such as the 114-281 form) by ELISA, RIA, and/or BIAcore assays, known in the art and described further in the Examples below. Preferred DR4 selective Apo-2 ligand variants of the invention will induce apoptosis in at least one type of mammalian cell (preferably a cancer cell), and such apoptotic activity can be determined by known art methods such as the alamar blue or crystal violet assay in the Examples. The DR5 selective Apo-2 ligand variants may or may not have altered binding affinities to any of the decoy receptors for Apo-2L, those decoy receptors being referred to in the art as DcR1, DcR2 and OPG.

For purposes of shorthand designation of Apo-2 ligand variants described herein, it is noted that numbers refer to the amino acid residue position along the amino acid sequence of the putative native Apo-2 ligand (see Fig. 1). Amino acid identification uses the single-letter alphabet of amino acids, i.e.,

| | | | | | |
|---|---|---|---|---|---|
| Asp | D | Aspartic acid | Ile | I | Isoleucine |
| Thr | T | Threonine | Leu | L | Leucine |
| Ser | S | Serine | Tyr | Y | Tyrosine |
| Glu | E | Glutamic acid | Phe | F | Phenylalanine |
| Pro | P | Proline | His | H | Histidine |
| Gly | G | Glycine | Lys | K | Lysine |
| Ala | A | Alanine | Arg | R | Arginine |
| Cys | C | Cysteine | Trp | W | Tryptophan |
| Val | V | Valine | Gln | Q | Glutamine |
| Met | M | Methionine | Asn | N | Asparagine |

The term "DR4" and "DR4 receptor" as used herein refers to full length and soluble, extracellular domain forms of the receptor described in Pan et al., Science, 276:111-113 (1997); WO98/32856 published July 30, 1998; US Patent 6,342,363 issued January 29, 2002; and WO99/37684 published July 29, 1999. The full length amino acid sequence of DR4 receptor is provided herein in Figs. 2A-2B. Ig-fusion proteins comprising an extracellular domain of DR4 are described in the Examples below.

The term "DR5" and "DR5 receptor" as used herein refers to the full length and soluble, extracellular domain forms of the receptor described in Sheridan et al., Science, 277:818-821 (1997); Pan et al., Science, 277:815-818 (1997), US Patent 6,072,047 issued June 6, 2000; US Patent 6,342,369, WO98/51793 published November 19, 1998; WO98/41629 published September 24, 1998; Screaton et al., Curr. Biol., 7:693-696 (1997); Walczak et al., EMBO J., 16:5386-5387 (1997); Wu et al., Nature Genetics, 17:141-143 (1997); WO98/35986 published August 20, 1998; EP870,827 published October 14, 1998; WO98/46643 published October 22, 1998; WO99/02653 published January 21, 1999; WO99/09165 published February 25, 1999; WO99/1179 published March 11, 1999. The DR5 receptor has also been referred to in the art as Apo-2; TRAIL-R, TR6, Tango-63, hAPO8 TRICK2 or KILLER. The term DR5 receptor used herein includes the full length 411 amino acid polypeptide provided in Fig. 3A and the full length 440 amino acid polypeptide provided in Figs. 3B-3C. Ig-fusion proteins comprising an extracellular domain of DR5 are described in the Examples below.

The term "polyol" when used herein refers broadly to polyhydric alcohol compounds. Polyols can be any water-soluble poly(alkylene oxide) polymer for example, and can have a linear or branched chain. Preferred polyols include those substituted at one or more hydroxyl positions with a chemical group, such as an alkyl group having between one and four carbons. Typically, the polyol is a poly(alkylene glycol), preferably poly(ethylene glycol) (PEG). However, those skilled in the art recognize that other polyols, such as, for example, poly(propylene glycol) and polyethylene-polypropylene glycol copolymers, can be employed using the techniques for conjugation described herein for PEG. The polyols of the invention include those well known in the art and those publicly available, such as from commercially available sources.

The term "conjugate" is used herein according to its broadest definition to mean joined or linked together. Molecules are "conjugated" when they act or operate as if joined.

The term "extracellular domain" or "ECD" refers to a form of ligand or receptor which is essentially free of transmembrane and cytoplasmic domains. Ordinarily, the soluble ECD will have less than 1% of such transmembrane and cytoplasmic domains, and preferably, will have less than 0.5% of such domains.

The term "Apo-2 ligand monomer" or "Apo-2L monomer" refers to a covalent chain of an extracellular domain sequence of Apo-2L.

The term "Apo-2 ligand dimer" or "Apo-2L dimer" refers to two Apo-2L monomers joined in a covalent linkage via a disulfide bond. The term as used herein includes free standing Apo-2L dimers and Apo-2L dimers that are within trimeric forms of Apo-2L (i.e., associated with another Apo-2L monomer).

The term "Apo-2 ligand trimer" or "Apo-2L trimer" refers to three Apo-2L monomers that are non-covalently associated.

The term "epitope tagged" when used herein refers to a chimeric polypeptide comprising a protein such as Apo-2 ligand or Apo-2 ligand variant, or a portion thereof, fused to a "tag polypeptide". The tag polypeptide has enough residues to provide an epitope against which an antibody can be made, yet is short enough such that it does not interfere with activity of the Apo-2 ligand or Apo-2 ligand variant. The tag polypeptide preferably also is fairly unique so that the antibody does not substantially cross-react with other epitopes. Suitable tag polypeptides generally have at least six amino acid residues and usually between about 8 to about 50 amino acid residues (preferably, between about 10 to about 20 residues).

The term "divalent metal ion" refers to a metal ion having two positive charges. Examples of divalent metal ions for use in the present invention include but are not limited to zinc, cobalt, nickel, cadmium, magnesium, and manganese. Particular forms of such metals that may be employed include salt forms (e.g., pharmaceutically acceptable salt forms), such as chloride, acetate, carbonate, citrate and sulfate forms of the above mentioned divalent metal ions. A preferred divalent metal ion for use in the present invention is zinc, and more preferably, the salt form, zinc sulfate. Divalent metal ions, as described herein, are preferably employed in concentrations or amounts (e.g., effective amounts) which are sufficient to, for example, (1) enhance storage stability of Apo-2L trimers over a desired period of time, (2) enhance production or yield of Apo-2L trimers in a recombinant cell culture or purification method, (3) enhance solubility (or reduce aggregation) of Apo-2L trimers, or (4) enhance Apo-2L trimer formation.

"Isolated," when used to describe the various proteins disclosed herein, means protein that has been identified and separated and/or recovered from a component of its natural environment. Contaminant components of its natural environment are materials that would typically interfere with diagnostic or therapeutic uses for the protein, and may include enzymes, hormones, and other proteinaceous or non-proteinaceous solutes. In preferred embodiments, the protein will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated protein includes protein in situ within recombinant cells, since at least one component of the Apo-2 ligand natural environment will not be present. Ordinarily, however, isolated protein will be prepared by at least one purification step.

An "isolated" Apo-2 ligand nucleic acid molecule is a nucleic acid molecule that is identified and separated from at least one contaminant nucleic acid molecule with which it is ordinarily associated in the natural source of the Apo-2 ligand nucleic acid. An isolated Apo-2 ligand nucleic acid molecule is other than in the form or setting in which it is found in nature. Isolated Apo-2 ligand nucleic acid molecules therefore are distinguished from the Apo-2 ligand nucleic acid molecule as it exists in natural cells. However, an isolated Apo-2 ligand nucleic acid molecule includes Apo-2 ligand nucleic acid molecules contained in cells that ordinarily express Apo-2 ligand where, for example, the nucleic acid molecule is in a chromosomal location different from that of natural cells.

"Percent (%) amino acid sequence identity" with respect to the sequences identified herein is defined as the percentage of amino acid residues in a candidate sequence that are identical with the amino acid residues in the Apo-2 ligand sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art can determine appropriate parameters for measuring alignment, including assigning algorithms needed to achieve maximal alignment over the full-length sequences being compared. For purposes herein, percent amino acid identity values can be obtained using the sequence comparison computer program, ALIGN-2, which was authored by Genentech, Inc. and the source code of which has been filed with user documentation in the US Copyright Office, Washington, DC, 20559, registered under the US Copyright Registration No. TXU510087. The ALIGN-2 program is publicly available through Genentech, Inc., South San Francisco, CA. All sequence comparison parameters are set by the ALIGN-2 program and do not vary.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

"Biologically active" or "biological activity" for the purposes herein means (a) having the ability to induce or stimulate apoptosis in at least one type of mammalian cell (preferably a cancer cell) or virally-infected cell in vivo or ex vivo; (b) capable of raising an antibody, i.e., immunogenic; (c) capable of binding and/or stimulating a receptor for Apo-2L, such as DR4, DR5, DcR1, DcR2, or OPG; or (d) retaining the activity of a native or naturally-occurring Apo-2L polypeptide.

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured using well known art methods, for instance, by cell viability assays, FACS analysis or DNA electrophoresis.

The terms "cancer", "cancerous", and "malignant" refer to or describe the physiological condition in mammals that is typically characterized by unregulated cell growth. Examples of cancer include but are not limited to, carcinoma including adenocarcinoma, lymphoma, blastoma, melanoma, sarcoma, and leukemia. More particular examples of such cancers include squamous cell cancer, small-cell lung cancer, non-small cell lung cancer, gastrointestinal cancer, Hodgkin's and non-Hodgkin's lymphoma, pancreatic cancer, glioblastoma, glioma, cervical cancer, ovarian cancer, liver cancer such as hepatic carcinoma and hepatoma, bladder cancer, breast cancer, colon cancer, colorectal cancer, endometrial carcinoma, myeloma (such as multiple myeloma), salivary gland carcinoma, kidney cancer such as renal cell carcinoma and Wilms' tumors, basal cell carcinoma, melanoma, prostate cancer, vulval cancer, thyroid cancer, testicular cancer, esophageal cancer, and various types of head and neck cancer.

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are autoimmune diseases, immune-mediated inflammatory diseases, non-immune-mediated inflammatory diseases, infectious diseases, and immunodeficiency diseases. Examples of immune-related and inflammatory diseases, some of which are immune or T cell mediated, which can be treated according to the invention include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjogren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory and fibrotic lung diseases such as inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft-versus-host-disease. Infectious diseases include AIDS (HIV infection), hepatitis A, B, C, D, and E, bacterial infections, fungal infections, protozoal infections and parasitic infections.

"Autoimmune disease" is used herein in a general sense to refer to disorders or conditions in mammals in which destruction of normal or healthy tissue arises from humoral or cellular immune responses of the individual mammal to his or her own tissue constituents. Examples include, but are not limited to, lupus erythematous, thyroiditis, rheumatoid arthritis, psoriasis, multiple sclerosis, autoimmune diabetes, and inflammatory bowel disease (IBD).

The term "prodrug" as used in this application refers to a precursor or derivative form of a pharmaceutically active substance that is less cytotoxic to cancer cells compared to the parent drug and is capable of being enzymatically activated or converted into the more active parent form. See, *e.g.*, Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, beta-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described below.

The term "cytotoxic agent" as used herein refers to a substance that inhibits or prevents the function of cells and/or causes destruction of cells. The term is intended to include radioactive isotopes (*e.g.* At I¹³¹, I¹²⁵, Y⁹⁰, Re¹⁸⁶, ,Re¹⁸⁸ Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

A "chemotherapeutic agent" is a chemical compound useful in the treatment of conditions like cancer. Examples of chemotherapeutic agents include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin γ₁^{I} and calicheamicin θ^{I}₁, see, *e.g.*, Agnew Chem Intl. Ed. Engl., 33:183-186 (1994); dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; podophyllinic acid; 2-ethylhydrazide; procarbazine; PSK^{®}; razoxane; rhizoxin; sizofiran; spirogermanium; tenuazonic acid; triaziquone; 2, 2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL^{®}, Bristol-Myers Squibb Oncology, Princeton, NJ) and doxetaxel (TAXOTERE^{®}, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylornithine (DMFO); retinoic acid; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

A "growth inhibitory agent" when used herein refers to a compound or composition which inhibits growth of a cell, either *in vitro* or *in vivo.* Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in *The Molecular Basis of Cancer,* Mendelsohn and Israel, eds., Chapter 1, entitled "Cell cycle regulation, oncogens, and antineoplastic drugs" by Murakami *et al.* (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors; platelet-growth factor; transforming growth factors (TGFs) such as TGF-α and TGF-β; insulin-like growth factor-I and -II; erythropoietin (EPO); osteoinductive factors; interferons such as interferon-α, -β, and -gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-2, IL-3, IL-4, IL-5, IL-6, IL-7, IL-8, IL-9, IL-11, IL-12; and other polypeptide factors including LIF and kit ligand (KL). As used herein, the term cytokine includes proteins from natural sources or from recombinant cell culture and biologically active equivalents of the native sequence cytokines.

The terms "treating", "treatment" and "therapy" as used herein refer to curative therapy, prophylactic therapy, and preventative therapy.

The term "mammal" as used herein refers to any mammal classified as a mammal, including humans, cows, horses, dogs and cats. In a preferred embodiment of the invention, the mammal is a human.

### II. Compositions and Methods of the Invention

A novel cytokine related to the TNF ligand family, the cytokine identified herein as "Apo-2 ligand" or "TRAIL" has been described. The predicted mature amino acid sequence of native human Apo-2 ligand contains 281 amino acids, and has a calculated molecular weight of approximately 32.5 kDa. The absence of a signal sequence and the presence of an internal hydrophobic region suggest that Apo-2 ligand is a type II transmembrane protein. Soluble extracellular domain Apo-2 ligand polypeptides have also been described. See, e.g., WO97/25428 published July 17, 1997. Apo-2L substitutional variants have further been described. Alanine scanning techniques have been utilized to identify various substitutional variant molecules having biological activity. Particular substitutional variants of the Apo-2 ligand include those in which at least one amino acid is substituted by another amino acids such as an alanine residue. These substitutional variants are identified, for example, as "D203A"; "D218A" and "D269A." This nomenclature is used to identify Apo-2 ligand variants wherein the aspartic acid residues at positions 203, 218, and/or 269 (using the numbering shown in Figure 1(SEQ ID NO:1)) are substituted by alanine residues. Optionally, the Apo-2L variants of the present invention may comprise one or more of the amino acid substitutions which are identified in Figure 9 or recited in Tables II, III, VII and VIII below. Optionally, such Apo-2L variants will be DR4 or DR5 receptor selective variants. It is believed that such DR4 or DR5 receptor selective variants will be useful in a variety of applications, e.g., for treating cancer cells which may express only either DR4 or DR5 receptors and for purifying preparations of DR4 and DR5 receptors.

The x-ray crystal structure of the extracellular domain of Apo-2 ligand is described herein, and alanine-scanning mutagenesis has been performed to provide the mapping of its receptor contact regions. The structure obtained for Apo-2 ligand reveals a homotrimeric protein which contains a novel divalent metal ion (zinc) binding site that coordinates the interaction of the Apo-2 ligand trimer molecule's three subunits.

The x-ray structure of Apo-2L was determined by molecular replacement using a model of TNF-alpha [Eck et al., J. Biol. Chem., 264:17595-17605 (1989)] and refined to 3.9 Angstrom (for the 114-281 residue form) and 1.3 Angstrom (for the D218A variant; 91-281 form). Like other members of the TNF family, Apo-2L appears to comprise a compact trimer formed of three jelly roll monomers which bury approximately 5100 Angstrom² (1700 Angstrom² per monomer) to form the globular trimer (See Figure 4). The position of the core beta-strands was well conserved compared to the other structurally characterized members of the TNF family, TNF-alpha [Eck et al., supra; Jones et al., Nature, 338:225-228 (1989)], TNF-beta [Eck et al., J. Biol. Chem., 267:2119-2122 (1992)], and CD40L [Karpusas et al., Structure, 3:1031-1039 (1995)], with a r.m.s.d. of 0.8 Angstrom when compared to the core strands of TNF-alpha or TNF-beta. None of the residues in the Apo-2L trimer interface appear to be absolutely conserved across the sequences of the all the presently known human TNF family members; however, the hydrophobic chemical nature of these residues is preserved. The conserved residues in the Apo-2L trimer interface cluster near the base (the widest part of the trimer) and along the three-fold axis. Near the top of the Apo-2L trimer interface in the vicinity of Cys230, the structures appear to diverge, and the conformation of the 190's and 230's loops are variable in each structure.

In contrast to the beta-scaffold core, the structure of the loops and receptor binding surfaces varies considerably among the TNF family members. One difference between the structure of Apo-2 ligand and the structures of TNF-alpha, TNF-beta, and CD40L is the connections between strands A and A'. In TNF-alpha, TNF-beta, and CD40L, strand A is followed by a compact loop. In Apo-2 ligand, a 15-residue insertion lengthens this loop and alters its conformation. The first part of the loop (residues 131 to 141) is disordered while the second part of the loop (residues 142 to 154) crosses the surface of the molecule from one monomer-monomer interface to the next with a conformation that resembles CD40L in its C-terminal portion.

A divalent metal ion (zinc) binding site is buried near the top of the trimerization interface. The TNF family members can be divided by sequence analysis into three groups with respect to Cys230: (1) proteins such as TNF-alpha and Fas ligand in which a cysteine residue at the position corresponding to Cys230 is accompanied by another cysteine in the adjacent loop (the 194-203 loop in Apo-2L) with which it can form a disulfide bridge precluding it from interacting with a metal ion, (2) proteins without a cysteine corresponding to Cys230 (such as TNF-beta and OPGL), and (3) proteins which have only one cysteine residue corresponding to Cys230. Apo-2L and its orthologs in other species meet the latter criteria (i.e., proteins which have only Cys230) and are expected to bind divalent metal ions at the trimer surface. The conformation of the main chain immediately prior to Cys230 in Apo-2L differs from the disulfide containing TNF family members such as TNF-alpha and CD40L. In Apo-2L, the side chain of Cys230 is oriented towards the interface instead of away from it.

The Cys230 residue in each Apo-2L monomer point inward toward the trimer axis and coordinate a divalent metal ion in conjunction with an interior solvent molecule. This divalent metal ion binding site exhibits slightly distorted tetrahedral geometry with bonds and angles appropriate for a zinc binding site and is completely inaccessible to solvent. The identity of the bound metal was confirmed using inductively coupled plasma atomic emission spectrometry (ICP-AES). In a quantitative analysis for Cd, Co, Zn, Ni, and Cu using ICP-AES, 0.79 moles of Zn and 0.06 moles of Co per molecule of Apo-2L trimer were detected demonstrating that the bound ion in the structure was zinc at approximately a one to one molar ratio. The importance of this site is demonstrated by the observation that alanine substitution of Cys230 results in a >8-fold decreased apoptotic activity. Furthermore, removal of the bound metal from Apo-2L by dialysis against chelating agents results in a 7-fold decrease in DR5 affinity and a >90-fold decrease in apoptotic activity. Upon removal of the Zn, the cysteines became prone to oxidation and disulfide-linked Apo-2L dimers were formed which had decreased apoptotic activity. Since the metal binding site appears to be buried in the Apo-2L trimer structure and is not expected to contact receptor, the data suggests that divalent metal ion binding may be important to maintain the trimer structure and stability of Apo-2L.

In order to map Apo-2 ligand's receptor binding site, amino acid residues important for receptor binding and biological activity were identified by alanine-scanning mutagenesis. [Cunningham et al., Science, 244:1081-1085 (1989)]. Single alanine substitutions at residues Arg149, Gln205, Val207, Tyr216, Glu236, or Tyr237 resulted in a greater than 5-fold decrease in apoptotic activity in a bioassay and showed decreased affinity for the receptors. Apo-2L binding to DR4, DR5 and DcR2 was most affected by alanine substitutions at residues Gln205, Tyr216, Glu236, or Tyr237, which resulted in at least a 5-fold decreased affinity against all three receptors. All of these variants with reduced apoptotic activity also exhibited impaired binding to either DR4 or DR5 (or both) suggesting that receptor binding is required for apoptotic activity.

Alanine substitutions at residues Asp218 and Asp269 resulted in Apo-2L variants having increased apoptotic activity. Residue Asp218 is located near Tyr216, which is one of the required residues for apoptotic activity. A comparison to the low resolution Apo-2L structure (114-281 form) suggests that the conformation of the 216-220 loop does not appear to be significantly altered by the presence of the D218A mutation.

When the results of the mutagenesis analysis were mapped to the Apo-2L trimer structure, the functional epitope on Apo-2L for receptor binding and biological activity was found to be located on the surface formed by the junction of two monomers, similar to TNF-beta. A shallow groove at the monomer-monomer interface forms the receptor binding site with both monomers contributing to the binding site. Residues Arg32, Tyr87, and Asp143 in TNF-alpha (corresponding to Apo-2L residues Arg158, Tyr216, and Asp267) also make contributions to TNF receptor binding. [Goh et al., Protein Engineering, 4:785-791 (1991)]. In contrast, residues of TNF-alpha (corresponding to residues Gln205, Glu236, and Tyr237 of Apo-2L) play only a minor role in TNFR binding. Thus, while for TNF-alpha the base of the trimer structure makes the most important contribution to receptor binding, in Apo-2L, important receptor binding residues are also presented on the top of the trimer structure. Apo-2L appears to be unique among the TNF family members of known structure in having a larger and more extended contact surface for interaction with its target receptors. In optional embodiments, Apo-2L variants will comprise native residues (i.e., will not be mutated) at positions corresponding to Arg149, Gln205, Val207, Tyr216, Glu236, and/or Tyr237.

The description below relates to methods of producing Apo-2 ligand such as the Apo-2 ligand variants described herein by culturing host cells transformed or transfected with a vector containing Apo-2 ligand encoding nucleic acid and recovering the polypeptide from the cell culture.

The DNA encoding Apo-2 ligand may be obtained from any cDNA library prepared from tissue believed to possess the Apo-2 ligand mRNA and to express it at a detectable level. Accordingly, human Apo-2 ligand DNA can be conveniently obtained from a cDNA library prepared from human tissues, such as the bacteriophage library of human placental cDNA as described in WO97/25428. The Apo-2 ligand-encoding gene may also be obtained from a genomic library or by oligonucleotide synthesis.

Libraries can be screened with probes (such as antibodies to the Apo-2 ligand or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding Apo-2 ligand is to use PCR methodology [Sambrook et al., supra; Dieffenbach et al., PCR Primer:A Laboratory Manual (Cold Spring Harbor Laboratory Press, 1995)].

Amino acid sequence fragments or variants of Apo-2 ligand can be prepared by introducing appropriate nucleotide changes into the Apo-2 ligand DNA, or by synthesis of the desired Apo-2 ligand polypeptide. Such fragments or variants represent insertions, substitutions, and/or deletions of residues within or at one or both of the ends of the intracellular region, the transmembrane region, or the extracellular region, or of the amino acid sequence shown for the full-length Apo-2 ligand in Figure 1 (SEQ ID NO:1). Any combination of insertion, substitution, and/or deletion can be made to arrive at the final construct, provided that the final construct possesses, for instance, a desired biological activity, such as apoptotic activity, as defined herein. Optionally, the Apo-2 ligand variants may be identified by phage library selection techniques, such as those described in the Examples. In a preferred embodiment, the fragments or variants have at least about 80% amino acid sequence identity, more preferably, at least about 90% sequence identity, and even more preferably, at least 95%, 96%, 97%, 98% or 99% sequence identity with the sequences identified herein for the intracellular, transmembrane, or extracellular domains of Apo-2 ligand, or the full-length sequence for Apo-2 ligand. The amino acid changes also may alter post-translational processes of the Apo-2 ligand, such as changing the number or position of glycosylation sites or altering the membrane anchoring characteristics.

Variations in the Apo-2 ligand sequence as described above can be made using any of the techniques and guidelines for conservative and non-conservative mutations set forth in U.S. Pat. No. 5,364,934. These include oligonucleotide-mediated (site-directed) mutagenesis, alanine scanning, and PCR mutagenesis.

Scanning amino acid analysis can be employed to identify one or more amino acids along a contiguous sequence. Among the preferred scanning amino acids are relatively small, neutral amino acids. Such amino acids include alanine, glycine, serine and cysteine. Alanine is typically a preferred scanning amino acid among this group because it eliminates the side-chain beyond the beta-carbon and is less likely to alter the main-chain conformation of the variant. [Cunningham et al., Science, 244:1081 (1989)]. Alanine is also typically preferred because it is the most common amino acid. Further, it is frequently found in both buried and exposed positions [Creighton, The Proteins, (W.H. Freeman & Co., NY); Chothia, J. Mol. Biol., 150:1 (1976)].

Particular Apo-2L variants of the present invention include those Apo-2L polypeptides which include one or more of the recited substitutions provided in Figure 9 or TABLES II, III, VII, or VIII. Such Apo-2L variants will typically comprise an amino acid sequence which differs from a native Apo-2L amino acid sequence (such as provided in Figure 1; SEQ ID NO:1, for a full length or mature form of Apo-2L or an extracellular domain sequence thereof such as the 114-281 amino acid form) in at least one or more amino acids. Optionally, the one or more amino acids which differ in the Apo-2L variant as compared to a native Apo-2L will comprise amino acid substitution(s) such as those indicated in Figure 9 or TABLES II, III, VII or VIII. Apo-2L variants of the invention include soluble Apo-2L variants comprising residues 39-281, 41-281, 91-281, 92-281, 95-281, 96-281 or 114-281 of Figure 1 (SEQ ID NO:1) and having one or more amino acid substitutions recited in Figure 9 or TABLES II, III, VII or VIII. Preferred Apo-2L variants will include those variants comprising residues 91-281, 92-281, 95-281 or 114-281 of Figure 1 (SEQ ID NO:1) and having one or more amino acid substitutions recited in TABLES II, III, VII or VIII which enhance biological activity, such as receptor binding or receptor selectivity for DR4 or DR5.

Variations in the Apo-2 ligand sequence also included within the scope of the invention relate to amino-terminal derivatives or modified forms. Such Apo-2 ligand sequences include any of the Apo-2 ligand polypeptides described herein having a methionine or modified methionine (such as formyl methionyl or other blocked methionyl species) at the N-terminus of the polypeptide sequence.

The nucleic acid (e.g., cDNA or genomic DNA) encoding native or variant Apo-2 ligand may be inserted into a replicable vector for further cloning (amplification of the DNA) or for expression. Various vectors are publicly available. The vector components generally include, but are not limited to, one or more of the following: a signal sequence, an origin of replication, one or more marker genes, an enhancer element, a promoter, and a transcription termination sequence, each of which is described below. Optional signal sequences, origins of replication, marker genes, enhancer elements and transcription terminator sequences that may be employed are known in the art and described in further detail in WO97/25428.

Expression and cloning vectors usually contain a promoter that is recognized by the host organism and is operably linked to the Apo-2 ligand nucleic acid sequence. Promoters are untranslated sequences located upstream (5') to the start codon of a structural gene (generally within about 100 to 1000 bp) that control the transcription and translation of a particular nucleic acid sequence, such as the Apo-2 ligand nucleic acid sequence, to which they are operably linked. Such promoters typically fall into two classes, inducible and constitutive. Inducible promoters are promoters that initiate increased levels of transcription from DNA under their control in response to some change in culture conditions, e.g., the presence or absence of a nutrient or a change in temperature. At this time a large number of promoters recognized by a variety of potential host cells are well known. These promoters are operably linked to Apo-2 ligand encoding DNA by removing the promoter from the source DNA by restriction enzyme digestion and inserting the isolated promoter sequence into the vector. Both the native Apo-2 ligand promoter sequence and many heterologous promoters may be used to direct amplification and/or expression of the Apo-2 ligand DNA.

Promoters suitable for use with prokaryotic and eukaryotic hosts are known in the art, and are described in further detail in WO97/25428.

A preferred method for the production of soluble Apo-2L in *E. coli* employs an inducible promoter for the regulation of product expression. The use of a controllable, inducible promoter allows for culture growth to the desirable cell density before induction of product expression and accumulation of significant amounts of product which may not be well tolerated by the host.

Three inducible promoter systems (T7 polymerase, trp and alkaline phosphatase (AP)) have been evaluated by Applicants for the expression of Apo-2L (form 114-281). The use of each of these three promoters resulted in significant amounts of soluble, biologically active Apo-2L trimer being recovered from the harvested cell paste. The AP promoter is preferred among these three inducible promoter systems tested because of tighter promoter control and the higher cell density and titers reached in harvested cell paste.

Construction of suitable vectors containing one or more of the above-listed components employs standard ligation techniques. Isolated plasmids or DNA fragments are cleaved, tailored, and religated in the form desired to generate the plasmids required. For analysis to confirm correct sequences in plasmids constructed, the ligation mixtures can be used to transform *E. coli* K12 strain 294 (ATCC 31,446) and successful transformants selected by ampicillin or tetracycline resistance where appropriate. Plasmids from the transformants are prepared, analyzed by restriction endonuclease digestion, and/or sequenced using standard techniques known in the art. [See, e.g., Messing et al., Nucleic Acids Res., 9:309 (1981); Maxam et al., Methods in Enzymology, 65:499 (1980)].

Expression vectors that provide for the transient expression in mammalian cells of DNA encoding Apo-2 ligand may be employed. In general, transient expression involves the use of an expression vector that is able to replicate efficiently in a host cell, such that the host cell accumulates many copies of the expression vector and, in turn, synthesizes high levels of a desired polypeptide encoded by the expression vector [Sambrook et al., supra]. Transient expression systems, comprising a suitable expression vector and a host cell, allow for the convenient positive identification of polypeptides encoded by cloned DNAs, as well as for the rapid screening of such polypeptides for desired biological or physiological properties. Thus, transient expression systems are particularly useful in the invention for purposes of identifying analogs and variants of Apo-2 ligand that are biologically active Apo-2 ligand.

Other methods, vectors, and host cells suitable for adaptation to the synthesis of Apo-2 ligand in recombinant vertebrate cell culture are described in Gething et al., Nature, 293:620-625 (1981); Mantei et al., Nature, 281:40-46 (1979); EP 117,060; and EP 117,058.

Suitable host cells for cloning or expressing the DNA in the vectors herein include prokaryote, yeast, or higher eukaryote cells. Suitable prokaryotes for this purpose include but are not limited to eubacteria, such as Gram-negative or Gram-positive organisms, for example, *Enterobacteriaceae* such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as P. *aeruginosa,* and *Streptomyces.* Preferably, the host cell should secrete minimal amounts of proteolytic enzymes.

In addition to prokaryotes, eukaryotic microbes such as filamentous fungi or yeast are suitable cloning or expression hosts for Apo-2 ligand-encoding vectors. Suitable host cells for the expression of glycosylated Apo-2 ligand are derived from multicellular organisms. Examples of all such host cells, including CHO cells, are described further in WO97/25428.

Host cells are transfected and preferably transformed with the above-described expression or cloning vectors for Apo-2 ligand production and cultured in nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO₄ and electroporation. Successful transfection is generally recognized when any indication of the operation of this vector occurs within the host cell.

Transformation means introducing DNA into an organism so that the DNA is replicable, either as an extrachromosomal element or by chromosomal integrant. Depending on the host cell used, transformation is done using standard techniques appropriate to such cells. The calcium treatment employing calcium chloride, as described in Sambrook et al., supra, or electroporation is generally used for prokaryotes or other cells that contain substantial cell-wall barriers. Infection with Agrobacterium tumefaciens is used for transformation of certain plant cells, as described by Shaw et al., Gene, 23:315 (1983) and WO 89/05859 published 29 June 1989. In addition, plants may be transfected using ultrasound treatment as described in WO 91/00358 published 10 January 1991.

For mammalian cells without such cell walls, the calcium phosphate precipitation method of Graham and van der Eb, Virology, 52:456-457 (1978) may be employed. General aspects of mammalian cell host system transformations have been described in U.S. Pat. No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Prokaryotic cells used to produce Apo-2 ligand may be cultured in suitable culture media as described generally in Sambrook et al., supra. Particular forms of culture media that may be employed for culturing E. coli are described further in the Examples below. Mammalian host cells used to produce Apo-2 ligand may be cultured in a variety of culture media.

Examples of commercially available culture media include Ham's F10 (Sigma), Minimal Essential Medium ("MEM", Sigma), RPMI-1640 (Sigma), and Dulbecco's Modified Eagle's Medium ("DMEM", Sigma). Any such media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin" drug), trace elements (defined as inorganic compounds usually present at final concentrations in the micromblar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

In general, principles, protocols, and practical techniques for maximizing the productivity of mammalian cell cultures can be found in Mammalian Cell Biotechnology: A Practical Approach, M. Butler, ed. (IRL Press, 1991).

In accordance with one aspect of the present invention, one or more divalent metal ions will typically be added to or included in the culture media for culturing or fermenting the host cells. The divalent metal ions are preferably present in or added to the culture media at a concentration level sufficient to enhance storage stability, enhance solubility, or assist in forming stable Apo-2L trimers coordinated by one or more zinc ions. The amount of divalent metal ions which may be added will be dependent, in part, on the host cell density in the culture or potential host cell sensitivity to such divalent metal ions. At higher host cell densities in the culture, it may be beneficial to increase the concentration of divalent metal ions. If the divalent metal ions are added during or after product expression by the host cells, it may be desirable to adjust or increase the divalent metal ion concentration as product expression by the host cells increases. It is generally believed that trace levels of divalent metal ions which may be present in typical commonly available cell culture media may not be sufficient for stable trimer formation. Thus, addition of further quantities of divalent metal ions, as described herein, is preferred.

The divalent metal ions are preferably added to the culture media at a concentration which does not adversely or negatively affect host cell growth, if the divalent metal ions are being added during the growth phase of the host cells in the culture. In shake flask cultures, it was observed that ZnSO₄ added at concentrations of greater than 1 mM can result in lower host cell density. Those skilled in the art appreciate that bacterial cells can sequester metal ions effectively by forming metal ion complexes with cellular matrices. Thus, in the cell cultures, it is preferable to add the selected divalent metal ions to the culture media after the growth phase (after the desired host cell density is achieved) or just prior to product expression by the host cells. To ensure that sufficient amounts of divalent metal ions are present, additional divalent metal ions may be added or fed to the cell culture media during the product expression phase.

The divalent metal ion concentration in the culture media should not exceed the concentration which may be detrimental or toxic to the host cells. In the methods of the invention employing the host cell, *E. coli*, it is preferred that the concentration of the divalent metal ion concentration in the culture media does not exceed about 1mM (preferably, ≤ 1mM). Even more preferably, the divalent metal ion concentration in the culture media is about 50 micromolar to about 250 micromolar. Most preferably, the divalent metal ion used in such methods is zinc sulfate. It is desirable to add the divalent metal ions to the cell culture in an amount wherein the metal ions and Apo-2 ligand trimer can be present at a one to one molar ratio.

The divalent metal ions can be added to the cell culture in any acceptable form. For instance, a solution of the metal ion can be made using water, and the divalent metal ion solution can then be added or fed to the culture media.

Expression of the Apo-2L may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or in situ hybridization, using an appropriately labeled probe, based on the sequences provided herein. Various labels may be employed, most commonly radioisotopes, and particularly ³²P. However, other techniques may also be employed, such as using biotin-modified nucleotides for introduction into a polynucleotide. The biotin then serves as the site for binding to avidin or antibodies, which may be labeled with a wide variety of labels, such as radionucleotides, fluorescers or enzymes. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected. Gene expression, alternatively, may be measured by immunological methods, such as immunohistochemical staining of cells or tissue sections and assay of cell culture or body fluids, to quantitate directly the expression of gene product. With immunohistochemical staining techniques, a cell sample is prepared, typically by dehydration and fixation, followed by reaction with labeled antibodies specific for the gene product coupled, where the labels are usually visually detectable, such as enzymatic labels, fluorescent labels, luminescent labels, and the like.

Antibodies useful for immunohistochemical staining and/or assay of sample fluids may be either monoclonal or polyclonal, and may be prepared in any mammal. Conveniently, the antibodies may be prepared against a native Apo-2 ligand polypeptide or against a synthetic peptide based on the DNA sequences provided herein or against exogenous sequence fused to Apo-2 ligand DNA and encoding a specific antibody epitope.

Apo-2 ligand preferably is recovered from the culture medium as a secreted polypeptide, although it also may be recovered from host cell lysates when directly produced without a secretory signal. If the Apo-2 ligand is membrane-bound, it can be released from the membrane using a suitable detergent solution (e.g. Triton-X 100) or its extracellular region may be released by enzymatic cleavage.

When Apo-2 ligand is produced in a recombinant cell other than one of human origin, the Apo-2 ligand is free of proteins or polypeptides of human origin. However, it is usually necessary to recover or purify Apo-2 ligand from recombinant cell proteins or polypeptides to obtain preparations that are substantially homogeneous as to Apo-2 ligand. As a first step, the culture medium or lysate may be centrifuged to remove particulate cell debris. Apo-2 ligand thereafter is purified from contaminant soluble proteins and polypeptides, with the following procedures being exemplary of suitable purification procedures: by fractionation on an ion-exchange column; ethanol precipitation; reverse phase HPLC; chromatography on silica or on a cation-exchange resin such as DEAE or CM; chromatofocusing: SDS-PAGE; ammonium sulfate precipitation; gel filtration using, for example, Sephadex G-75; diafiltration and protein A Sepharose columns to remove contaminants such as IgG.

In a preferred embodiment, the Apo-2 ligand can be isolated by affinity chromatography. Apo-2 ligand fragments or variants in which residues have been deleted, inserted, or substituted are recovered in the same fashion as native Apo-2 ligand, taking account of any substantial changes in properties occasioned by the variation. For example, preparation of an Apo-2 ligand fusion with another protein or polypeptide, e.g., a bacterial or viral antigen, facilitates purification; an immunoaffinity column containing antibody to the antigen can be used to adsorb the fusion polypeptide.

A protease inhibitor such as phenyl methyl sulfonyl fluoride (PMSF) also may be useful to inhibit proteolytic degradation during purification, and antibiotics may be included to prevent the growth of adventitious contaminants. One skilled in the art will appreciate that purification methods suitable for native Apo-2 ligand may require modification to account for changes in the character of Apo-2 ligand or its variants upon expression in recombinant cell culture.

During any such purification steps, it may be desirable to expose the recovered Apo-2L to a divalent metal ion-containing solution or to purification material (such as a chromatography medium or support) containing one or more divalent metal ions. In a preferred embodiment, the divalent metal ions and/or reducing agent is used during recovery or purification of the Apo-2L. Optionally, both divalent metal ions and reducing agent, such as DTT or BME, may be used during recovery or purification of the Apo-2L. It is believed that use of divalent metal ions during recovery or purification will provide for stability of Apo-2L trimer or preserve Apo-2L trimer formed during the cell culturing step.

The description below also relates to methods of producing Apo-2 ligand variants covalently attached (hereinafter "conjugated") to one or more chemical groups. Chemical groups suitable for use in an Apo-2L variant conjugate of the present invention are preferably not significantly toxic or immunogenic. The chemical group is optionally selected to produce an Apo-2L variant conjugate that can be stored and used under conditions suitable for storage. A variety of exemplary chemical groups that can be conjugated to polypeptides are known in the art and include for example carbohydrates, such as those carbohydrates that occur naturally on glycoproteins, polyglutamate, and non-proteinaceous polymers, such as polyols (see, e.g., U.S. Patent No. 6,245,901).

A polyol, for example, can be conjugated to polypeptides such as an Apo-2L variant at one or more amino acid residues, including lysine residues, as is disclosed in WO 93/00109, supra. The polyol employed can be any water-soluble poly(alkylene oxide) polymer and can have a linear or branched chain. Suitable polyols include those substituted at one or more hydroxyl positions with a chemical group, such as an alkyl group having between one and four carbons. Typically, the polyol is a poly(alkylene glycol), such as poly(ethylene glycol) (PEG), and thus, for ease of description, the remainder of the discussion relates to an exemplary embodiment wherein the polyol employed is PEG and the process of conjugating the polyol to a polypeptide is termed "pegylation." However, those skilled in the art recognize that other polyols, such as, for example, poly(propylene glycol) and polyethylene-polypropylene glycol copolymers, can be employed using the techniques for conjugation described herein for PEG.

The average molecular weight of the PEG employed in the pegylation of the Apo-2L variant can vary, and typically may range from about 500 to about 30,000 daltons (D). Preferably, the average molecular weight of the PEG is from about 1,000 to about 25,000 D, and more preferably from about 2,000 to about 5,000 D. In one embodiment, pegylation is carried out with PEG having an average molecular weight of about 2,000 D. Optionally, the PEG homopolymer is unsubstituted, but it may also be substituted at one end with an alkyl group. Preferably, the alkyl group is a C1-C4 alkyl group, and most preferably a methyl group. PEG preparations are commercially available, and typically, those PEG preparations suitable for use in the present invention are nonhomogeneous preparations sold according to average molecular weight. For example, commercially available PEG(5000) preparations typically contain molecules that vary slightly in molecular weight, usually ± 500 D.

The Apo-2 ligand variants of the invention may be in various forms, such as in monomer form or trimer form (comprising three monomers). Optionally, an Apo-2L variant trimer will be pegylated in a manner such that a PEG molecule is linked or conjugated to one, two or each of the three monomers that make up the trimeric Apo-2L variant. In such an embodiment, it is preferred that the PEG employed have an average molecular weight of about 2,000 to about 5,000 D. It is also contemplated that the Apo-2L variant trimers may be "partially" pegylated, i.e., wherein only one or two of the three monomers that make up the trimer are linked or conjugated to PEG. In such a "partially" pegylated Apo-2L variant, optionally the PEG employed have an average molecular weight of about 5,000 D or greater than 5,000 D.

A variety of methods for pegylating proteins are known in the art. Specific methods of producing proteins conjugated to PEG include the methods described in U.S. Pat. No. 4,179,337, U.S. Pat. No. 4,935,465 and U.S. Patent No. 5,849,535. Typically the protein is covalently bonded via one or more of the amino acid residues of the protein to a terminal reactive group on the polymer, depending mainly on the reaction conditions, the molecular weight of the polymer, etc. The polymer with the reactive group(s) is designated herein as activated polymer. The reactive group selectively reacts with free amino or other reactive groups on the protein. The PEG polymer can be coupled to the amino or other reactive group on the protein in either a random or a site specific manner. It will be understood, however, that the type and amount of the reactive group chosen, as well as the type of polymer employed, to obtain optimum results, will depend on the particular protein or protein variant employed to avoid having the reactive group react with too many particularly active groups on the protein. As this may not be possible to avoid completely, it is recommended that generally from about 0.1 to 1000 moles, preferably 2 to 200 moles, of activated polymer per mole of protein, depending on protein concentration, is employed. The final amount of activated polymer per mole of protein is a balance to maintain optimum activity, while at the same time optimizing, if possible, the circulatory half-life of the protein.

While the residues may be any reactive amino acids on the protein, such as the N-terminal amino acid group, preferably the reactive amino acid is cysteine, which is linked to the reactive group of the activated polymer through its free thiol group as shown, for example, in WO 99/03887, WO 94/12219, WO 94/22466, U.S. Patent No. 5,206,344, U.S. Patent No. 5,166,322, and U.S. Patent No. 5,206,344. Alternatively the reactive group is lysine, which is linked to the reactive group of the activated polymer through its free epsilon-amino group, or glutamic or aspartic acid, which is linked to the polymer through an amide bond. This reactive group can then react with, for example, the α and ε amines of proteins to form a covalent bond. Conveniently, the other end of the PEG molecule can be "blocked" with a non-reactive chemical group, such as a methoxy group, to reduce the formation of PEG-crosslinked complexes of protein molecules.

Suitable activated PEGs can be produced by a number of conventional reactions. For example, a N-hydroxysuccinimide ester of a PEG (M-NHS-PEG) can be prepared from PEG-monomethyl ether (which is commercially available from Union Carbide) by reaction with N,N'-dicyclohexylcarbodiimide (DCC) and N-hydroxysuccinimide (NHS), according to the method of Buckmann and Merr, Makromol. Chem., 182:1379-1384 (1981). In addition, a PEG terminal hydroxy group can be converted to an amino group, for example, by reaction with thionyl bromide to form PEG-Br, followed by aminolysis with excess ammonia to form PEG-NH₂. The PEG-NH₂ is then conjugated to the protein of interest using standard coupling reagents, such as Woodward's Reagent K. Furthermore, a PEG terminal -CH₂ OH group can be converted to an aldehyde group, for example, by oxidation with MnO₂. The aldehyde group is conjugated to the protein by reductive alkylation with a reagent such as cyanoborohydride. Alternatively, activated PEGs suitable for use in the present invention can be purchased from a number of vendors. For example, Shearwater Polymers, Inc. (Huntsville, Ala.) sells methoxy-PEG-maleimide, MW 2,000, in addition to a succinimidyl carbonate of methoxy-PEG and methoxy-PEG succinimidyl propionate.

The degree of pegylation of Apo-2L variant of the present invention can be adjusted to provide a desirably increased in vivo half-life (hereinafter "half-life"), compared to the corresponding non-pegylated Apo-2L variant. It is believed that the half-life of a pegylated Apo-2L variant typically increases incrementally with increasing degree of pegylation. The degree and sites of pegylation of a protein are determined, e.g., by (1) the number and reactivities of pegylation sites (e.g., primary amines) and (2) pegylation reaction conditions. As some of the pegylation sites in a protein are likely to be relatively unreactive, standard pegylation reactions typically result in less than complete pegylation.

Standard mutagenesis techniques can be used to alter the number of potential pegylation sites in a protein. Thus, to the extent that amino acid substitutions introduce or replace amino acids such as cysteine and lysine, Apo-2L variants of the present invention can contain a greater or lesser number of potential pegylation sites than native sequence Apo-2L (shown in Figure 1). The degree and sites of pegylation can also be manipulated by adjusting reaction conditions, such as the relative concentrations of the activated PEG and the protein as well as the pH. Suitable conditions for a desired degree of pegylation can be determined empirically by varying the parameters of standard pegylation reactions.

Pegylation of Apo-2L variants is carried out by any convenient method. In an exemplary embodiment, the Cys170 side chain of R170C-Apo2L.0 (i.e., a variant Apo-2L having amino acids 114-281 of Figure 1 and a cysteine residue substituted for the native arginine residue at position 170) is covalently modified by reaction with methoxy-PEG-maleimide, MW 2,000 D (Shearwater Polymers). Briefly, R170C-Apo2L.0 is prepared for modification by first reducing with 10 mM DTT at ambient temperature for about 30 minutes followed by passage over a PD-10 gel filtration column, equilibrated and eluted with HIC buffer (0.45 M Na₂SO₄, 25 mM Tris-HCl pH 7.5), to remove the reducing agent. An aliquot of a PEG-maleimide solution (10 mM in dH₂O) is then added immediately. Conditions of time and reagent concentration necessary to ensure complete reaction can be determined empirically. Molar concentration ratios of PEG-maleimide to R170C-Apo2L.0 monomer ranging from 0.5 to 5-fold and reaction times of 2 or 24 hours can be used. The reactions are terminated by addition of a 10-fold molar excess of iodoacetamide, relative to the R170C-Apo2L.0 monomer concentration, such that any unpegylated Cys170 thiol becomes carboxyamidomethylated. Modification with iodoacetamide is for about 30 minutes and then the excess reagents are removed by gel filtration on a NAP-5 column (Pharmacia) equilibrated and eluted with PBS.

The pegylated proteins can be characterized by SDS-PAGE, gel filtration, NMR, peptide mapping, liquid chromatography-mass spectrophotometry, and in vitro biological assays. The extent of pegylation is typically first shown by SDS-PAGE. Polyacrylamide gel electrophoresis in 10% SDS is typically run in 10 mM Tris-HCl pH 8.0, 100 mM NaCl as elution buffer. To demonstrate which residue is pegylated, peptide mapping using proteases such as trypsin and Lys-C protease can be performed. Thus, samples of pegylated and non-pegylated R170C-Apo2L.0 can be digested with a protease such as Lys-C protease and the resulting peptides separated by a technique such as reverse phase HPLC. The chromatographic pattern of peptides produced can be compared to a peptide map previously determined for the Apo-2L.0 polypeptide. Each peak can then be analyzed by mass spectrometry to verify the size of the fragment in the peak. The fragment(s) that carried PEG groups are usually not retained on the HPLC column after injection and disappear from the chromatograph. Such disappearance from the chromatograph is an indication of pegylation on that particular fragment that should contain at least one pegylatable amino acid residue. Pegylated Apo-2L variants may further be assayed for ability to interact with an Apo-2L receptor and/or induce apoptosis in mammalian cells and/or other biological activities using known methods in the art.

It is further contemplated that the Apo2L variants described herein may be also be linked or fused to leucine zipper sequences using techniques known in the art.

Formulations comprising Apo-2 ligand variants and one or more divalent metal ions are also provided by the present invention. It is believed that such formulations will be particularly suitable for storage (and maintain Apo-2L trimerization), as well as for therapeutic administration. Preferred formulations will comprise Apo-2L and zinc or cobalt. More preferably, the formulation will comprise an Apo-2L and zinc or cobalt solution in which the metal is at a <2X molar ratio to the protein. If an aqueous suspension is desired, the divalent metal ion in the formulation may be at a >2X molar ratio to the protein. Using zinc sulfate, Applicants have found Apo-2L (form 114-281) precipitates and forms an aqueous suspension at about a 100mM concentration of zinc sulfate in the formulation. Those skilled in the art will appreciate that at a >2X molar ratio, there may be an upper range of concentration of the divalent metal ion in the formulation at which the metal can become deleterious to the formulation or would be undesirable as a therapeutic formulation.

The formulations may be prepared by known techniques. For instance, the Apo-2L formulation may be prepared by buffer exchange on a gel filtration column.

Typically, an appropriate amount of a pharmaceutically-acceptable salt is used in the formulation to render the formulation isotonic. Examples of pharmaceutically-acceptable carriers include saline, Ringer's solution and dextrose solution. The pH of the formulation is preferably from about 6 to about 9, and more preferably from about 7 to about 7.5. Preferably, the pH is selected so as to ensure that the zinc remains bound to the Apo-2L. If the pH is too high or too low, the zinc does not remain bound to the Apo-2L and as a result, dimers of Apo-2L will tend to form. It will be apparent to those persons skilled in the art that certain carriers may be more preferable depending upon, for instance, the route of administration and concentrations of Apo-2 ligand and divalent metal ions.

Therapeutic compositions of the Apo-2L can be prepared by mixing the desired Apo-2L molecule having the appropriate degree of purity with optional pharmaceutically acceptable carriers, excipients, or stabilizers (Remington's Pharmaceutical Sciences, 16th edition, Osol, A. ed. (1980)), in the form of lyophilized formulations, aqueous solutions or aqueous suspensions. Acceptable carriers, excipients, or stabilizers are preferably nontoxic to recipients at the dosages and concentrations employed, and include buffers such as Tris, HEPES, PIPES, phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; sugars such as sucrose, mannitol, trehalose or sorbitol; salt-forming counter-ions such as sodium; and/or nonionic surfactants such as Tweeny^{™}, PLURONICS^{™} or polyethylene glycol (PEG).

Additional examples of such carriers include ion exchangers, alumina, aluminum stearate, lecithin, serum proteins, such as human serum albumin, buffer substances such as glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, colloidal silica, magnesium trisilicate, polyvinyl pyrrolidone, and cellulose-based substances. Carriers for topical or gel-based forms include polysaccharides such as sodium carboxymethylcellulose or methylcellulose, polyvinylpyrrolidone, polyacrylates, polyoxyethylene-polyoxypropylene-block polymers, polyethylene glycol, and wood wax alcohols. For all administrations, conventional depot forms are suitably used. Such forms include, for example, microcapsules, nano-capsules, liposomes, plasters, inhalation forms, nose sprays, sublingual tablets, and sustained-release preparations.

Apo-2L to be used for in vivo administration should be sterile. This is readily accomplished by filtration through sterile filtration membranes, prior to or following lyophilization and reconstitution. Apo-2L ordinarily will be stored in lyophilized form or in solution if administered systemically. If in lyophilized form, Apo-2L is typically formulated in combination with other ingredients for reconstitution with an appropriate diluent at the time for use. An example of a liquid formulation of Apo-2L is a sterile, clear, colorless unpreserved solution filled in a single-dose vial for subcutaneous injection.

Therapeutic Apo-2L variant formulations generally are placed into a container having a sterile access port, for example, an intravenous solution bag or vial having a stopper pierceable by a hypodermic injection needle. The formulations are preferably administered as repeated intravenous (i.v.), subcutaneous (s.c.), intramuscular (i.m.) injections or infusions, or as aerosol formulations suitable for intranasal or intrapulmonary delivery (for intrapulmonary delivery see, *e.g.*, EP 257,956).

Apo-2L variants can also be administered in the form of sustained-release preparations. Suitable examples of sustained-release preparations include semipermeable matrices of solid hydrophobic polymers containing the protein, which matrices are in the form of shaped articles, *e.g.*, films, or microcapsules. Examples of sustained-release matrices include polyesters, hydrogels (*e.g.*, poly(2-hydroxyethyl-methacrylate) as described by Langer et al., J. Biomed. Mater. Res., 15: 167-277 (1981) and Langer, Chem. Tech., 12: 98-105 (1982) or poly(vinylalcohol)), polylactides (U.S. Patent No. 3,773,919, EP 58,481), copolymers of L-glutamic acid and gamma ethyl-L-glutamate (Sidman et al., Biopolymers, 22: 547-556 (1983)), non-degradable ethylene-vinyl acetate (Langer *et al.*, *supra*)*,* degradable lactic acid-glycolic acid copolymers such as the Lupron Depot (injectable microspheres composed of lactic acid-glycolic acid copolymer and leuprolide acetate), and poly-D-(-)-3-hydroxybutyric acid (EP 133,988).

The Apo-2L variants and its formulations described herein can be employed in a variety of therapeutic and non-therapeutic applications. Among these applications are methods of treating various cancers and viral conditions. Such therapeutic and non-therapeutic applications are described, for instance, in WO97/25428 and WO97/01633.

The Apo2L variants described herein are useful in treating various pathological conditions, such as immune related diseases or cancer. Diagnosis in mammals of the various pathological conditions described herein can be made by the skilled practitioner. Diagnostic techniques are available in the art which allow, e.g., for the diagnosis or detection of cancer or immune related disease in a mammal. For instance, cancers may be identified through techniques, including but not limited to, palpation, blood analysis, x-ray, NMR and the like. Immune related diseases can also be readily identified. In systemic lupus erythematosus, the central mediator of disease is the production of auto-reactive antibodies to self proteins/tissues and the subsequent generation of immune-mediated inflammation. Multiple organs and systems are affected clinically including kidney, lung, musculoskeletal system, mucocutaneous, eye, central nervous system, cardiovascular system, gastrointestinal tract, bone marrow and blood. Rheumatoid arthritis (RA) is a chronic systemic autoimmune inflammatory disease that mainly involves the synovial membrane of multiple joints with resultant injury to the articular cartilage. The pathogenesis is T lymphocyte dependent and is associated with the production of rheumatoid factors, auto-antibodies directed against self IgG, with the resultant formation of immune complexes that attain high levels in joint fluid and blood. These complexes in the joint may induce the marked infiltrate of lymphocytes and monocytes into the synovium and subsequent marked synovial changes; the joint space/fluid if infiltrated by similar cells with the addition of numerous neutrophils. Tissues affected are primarily the joints, often in symmetrical pattern. However, extra-articular disease also occurs in two major forms. One form is the development of extra-articular lesions with ongoing progressive joint disease and typical lesions of pulmonary fibrosis, vasculitis, and cutaneous ulcers. The second form of extra-articular disease is the so called Felty's syndrome which occurs late in the RA disease course, sometimes after joint disease has become quiescent, and involves the presence of neutropenia, thrombocytopenia and splenomegaly. This can be accompanied by vasculitis in multiple organs with formations of infarcts, skin ulcers and gangrene. Patients often also develop rheumatoid nodules in the subcutis tissue overlying affected joints; the nodules late stage have necrotic centers surrounded by a mixed inflammatory cell infiltrate. Other manifestations which can occur in RA include: pericarditis, pleuritis, coronary arteritis, interstitial pneumonitis with pulmonary fibrosis, keratoconjunctivitis sicca, and rheumatoid nodules.

The Apo2L variants can be administered in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Optionally, administration may be performed through mini-pump infusion using various commercially available devices.

Effective dosages and schedules for administering Apo2L variants may be determined empirically, and making such determinations is within the skill in the art. Single or multiple dosages may be employed. It is presently believed that an effective dosage or amount of Apo2L variants used alone may range from about 1 µg/kg to about 100 mg/kg of body weight or more per day. Interspecies scaling of dosages can be performed in a manner known in the art, e.g., as disclosed in Mordenti et al., Pharmaceut. Res., 8:1351 (1991).

When *in vivo* administration of an Apo2L variant is employed, normal dosage amounts may vary from about 10 ng/kg to up to 100 mg/kg of mammal body weight or more per day, preferably about 1 µg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue. Those skilled in the art will understand that the dosage of Apo2L variant that must be administered will vary depending on, for example, the mammal which will receive the Apo2L variant, the route of administration, and other drugs or therapies being administered to the mammal.

It is contemplated that yet additional therapies may be employed in the methods. The one or more other therapies may include but are not limited to, administration of radiation therapy, cytokine(s), growth inhibitory agent(s), chemotherapeutic agent(s), cytotoxic agent(s), tyrosine kinase inhibitors, ras farnesyl transferase inhibitors, angiogenesis inhibitors, and cyclin-dependent kinase inhibitors which are known in the art and defined further with particularity in Section I above. It is contemplated that such other therapies may be employed as an agent separate from the Apo2L variant, as well as linked or conjugated to the Apo2L variant molecule itself. In addition, therapies based on therapeutic antibodies that target tumor antigens such as Rituxan^{™} or Herceptin^{™} as well as anti-angiogenic antibodies such as anti-VEGF, or antibodies that target Apo2L receptors, such as DR5 or DR4.

Preparation and dosing schedules for chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the Apo2L variant, or may be given simultaneously therewith.

It may be desirable to also administer antibodies against other antigens, such as antibodies which bind to CD20, CD11a, CD18, CD40, ErbB2, EGFR, ErbB3, ErbB4, vascular endothelial factor (VEGF), or other TNFR family members (such as DR4, DR5, OPG, TNFR1, TNFR2, GITR, Apo-3, TACI, BCMA, BR3). Alternatively, or in addition, two or more antibodies binding the same or two or more different antigens disclosed herein may be co-administered to the patient. Sometimes, it may be beneficial to also administer one or more cytokines to the patient. In one embodiment, the Apo2L variants herein are co-administered with a growth inhibitory agent. For example, the growth inhibitory agent may be administered first, followed by an Apo2L variant of the present invention.

The Apo2L variant (and one or more other therapies) may be administered concurrently or sequentially. Following administration of Apo2L variant, treated cells *in vitro* can be analyzed. Where there has been *in vivo* treatment, a treated mammal can be monitored in various ways well known to the skilled practitioner. For instance, tumor cells can be examined pathologically to assay for necrosis or serum can be analyzed for immune system responses.

An article of manufacture such as a kit containing Apo-2L variant useful for the diagnosis or treatment of the disorders described herein comprises at least a container and a label. Suitable containers include, for example, bottles, vials, syringes, and test tubes. The containers may be formed from a variety of materials such as glass or plastic. The container holds an Apo-2L variant or formulation that is effective for diagnosing or treating the condition and may have a sterile access port (for example, the container may be an intravenous solution bag or a vial having a stopper pierceable by a hypodermic injection needle). The label on, or associated with, the container indicates that the formulation is used for diagnosing or treating the condition of choice. The article of manufacture may further comprise a second container comprising a pharmaceutically-acceptable buffer, such as phosphate-buffered saline, Ringer's solution, and dextrose solution. It may further include other materials desirable from a commercial and user standpoint, including other buffers, diluents, filters, needles, syringes, and package inserts with instructions for use. The article of manufacture may also comprise a second or third container with another active agent as described above.

The following examples are offered for illustrative purposes only, and are not intended to limit the scope of the present invention in any way.

All patent and literature references cited in the present specification are hereby incorporated by reference in their entirety.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, Virginia.

### EXAMPLE 1

### Design and production of Apo2L/TRAIL phage display libraries

Residues in Apo2L/TRAIL were chosen for inclusion in the phage display libraries on the basis of an examination of the x-ray structure determined for the Apo2L/TRAIL·DR5-ECD complex (see, e.g. Hymowitz et al., (1999) Molecular Cell 4, 563-571). In addition, information from the alanine-scan of Apo2L/TRAIL (see, e.g. Hymowitz et al., (2000) Biochemistry 39, 633-640) was used to guide library design. For example, sites where alanine substitution gave a large decrease in affinity (>5-fold) for binding to all of the Apo2L/TRAIL receptors tested (i.e. Gln205) were not chosen for the library. Sites which gave only modest changes in affinity (<5-fold) when replaced with alanine appeared more likely to increase receptor-selectivity when mutated. An example of this type of site is Gln193 where alanine substitution causes a 1.7-fold decrease in affinity for DR4 but has no effect on binding to DR5 and DcR2 (see, e.g. Hymowitz et al., (2000) Biochemistry 39, 633-640). Further changes in receptor specificity might be obtained by substitution of Gln193 with a residue other than alanine. This rational was used to design two Apo2-L/TRAIL libraries having sites 189, 191, 193, 199, 201, and 209 randomized (DR4 library) or 189, 191, 193, 264, 266, 267, and 269 randomized (DR5 library). As shown in Figure 7A, these residues are within or near the "patch A" contact observed in the x-ray crystal structure (see, e.g. Hymowitz et al., (1999) Molecular Cell 4, 563-571). These libraries contained "NNS" codons at the specified sites such that all 20 amino acids, and only 1 stop codon, were possible at these positions.

A phagemid vector designed for the expression of Apo2L/TRAIL (residues 96-281 of Figure 1; SEQ ID NO:1) as a fusion to the geneIII protein of M13 bacteriophage was constructed as follows. The DNA encoding the 96-281 portion of Apo2L/TRAIL was amplified by PCR from the template plasmid pAPOK5 (see, e.g. Hymowitz et al., (2000) Biochemistry 39, 633-640) using oligonucleotides that create an NsiI site (5' oligo) and BamHI site (3' oligo). After cleavage with NsiI and BamHI this fragment was ligated into NsiI/BamHI cleaved pTFAA-g3 (see, e.g. Lee, G.F., & Kelley, R.F. (1998) J. Biol. Chem. 273, 4149-4154). Plasmid clones were screened for the proper insert by restriction digestion analysis and positive clones were confirmed by dideoxynucleotide sequencing. The resulting plasmid (pAPOK4) encodes a fragment having the stII bacterial signal sequence fused to the N-terminus of the 96-281 fragment of Apo2L/TRAIL. A tripeptide linker with the sequence G•S•A is appended to the C-terminus of Apo2L/TRAIL followed by an in-frame amber stop codon and the gene 3 product of M13 bacteriophage. The alkaline phosphatase promoter is used to direct expression. In strains of *E. coli* capable of suppressing amber stop codons (supE genotype), a fusion protein consisting of stII-Apo2L/TRAIL(96-281)-gene 3 is secreted into the periplasm. Since suppression of amber stop codons is not 100% efficient, some free stII-Apo2L/TRAIL is secreted as well. In the periplasm the stII signal peptide is presumed to be removed by the *E. coli* signal peptidase. When supplied with assembly proteins by co-infection with helper phage, phage particles are produced which have Apo2L/TRAIL(96-281)-gene3 displayed on their surface. Although the exact composition of the proteins displayed is unknown, it is likely that one molecule of Apo2L/TRAIL(96-281)-gene3 is assembled into a trimer with 2 molecules of free Apo2L/TRAIL(96-281). Since gene3 as opposed to gene8 is used to display Apo2L/TRAIL, this corresponds to "monovalent display" (see, e.g. Lowman, H.B., & Wells, J.A. (1993) J. Mol. Biol. 234, 564-578) where each phage particle displays no more than 1-5 copies of the Apo2L/TRAIL molecule.

Initial tests of the pAPOK4 vector suggested poor display on phage of correctly assembled Apo2L/TRAIL (Table I). Phage ELISA with immobilized DR5-IgG indicated only a weak, specific binding signal. Binding was not inhibited by purified Apo2L/TRAIL, instead exogenous ligand enhanced the signal measured by phage ELISA, consistent with incomplete trimer assembly on phage. In addition, sorting of phage against DR5-IgG gave only weak specific enrichment. Specific binding increased if the phage were produced by growth at 30 °C rather than the usual 37 °C. Given the poor display using pAPOK4, constructs with different promoters were tested for increased display. PAPOK4.2 was constructed by replacing the alkaline phosphatase promoter in pAPOK4 with the tac promoter. This was accomplished by replacing an EcoRI/NsiI restriction fragment carrying the AP promoter in pAPOK4 with an EcoRI/NsiI fragment from plasmid pW1205a (see, e.g. Sidhu et al., (2000) Methods in Enzymology 328, 333-363) that contains the tac promoter. Analysis of expression from pAPOK4.2 vector by both phage ELISA and enrichment indicated better phage display of Apo2L/TRAIL than observed with pAPOK4 (Table I). Optimum display was obtained for phage production at 30 °C with induction of the tac promoter by addition of 1 µM IPTG.

Library construction was performed as described by Sidhu et al. (see, e.g. Sidhu et al., (2000) Methods in Enzymology 328, 333-363). For the DR5 library, "TAA" stop codons were introduced into pAPOK4.2 at the library positions 189, 191, 193, 264, 266, 267, and 269. This "stop" template was used as the template for oligonucleotide-directed mutagenesis using a protocol adapted from Kunkel (see, e.g. Kunkel, T.A. (1985) Proc. Natl. Acad. Sci. (USA) 82, 488-492). Two oligonucleotides were used in the mutagenesis reaction. One encoded "NNS" codons at the library positions 189, 191, and 193 and the second produced NNS codons at sites 264, 266, 267, 269. Use of the stop template ensured that any template DNA that did not become mutated, survived the Kunkel selection, and would not produce functional Apo2L/TRAIL. For the DR4 library, TAA stops were introduced at positions 189, 191, 193, 199, 201, 209. The mutations Y213W:S215D were also introduced into this stop template since previous work (data not shown) indicated that these mutations gave better display of Apo2L on phage as well as diminished affinity for DR5. An oligonucleotide encoding NNS codons at sites 189, 191, 193, 199, 201, 209 was used for mutagenesis of this stop template. Upon electroporation into SS-320 *E. coli* the DR5 library gave a titer of 2.5 x 10⁸ independent clones. Sequencing of 10 clones from the library indicated that 7/10 were mutated at both sites suggesting an actual library size of 1.75 x 10⁸ clones. The DR4 library gave a titer of 5 x 10⁹ clones, sequencing showed 5/10 were mutated, and thus an actual library size of 2.5 x 10⁹. Since SS-320 cells do not carry an amber suppressor, XL-1 *E. coli* were used to produce phage particles for sorting. The SS-320 electroporated with the library were grown in 500 mLs of 2 YT media containing 50 µg/mL carbenecillin and 1 x 10¹⁰ PFU/mL VCS (Stratagene, Inc.) helper phage. After overnight growth at 37 °C in a 4 L baffled flask on a rotary shaker operated at 200 RPM, the cells were removed by centrifugation and the phage were precipitated from the supernatant by addition of 1/5 volume of 20 % PEG, 2.5 M NaCl. The phage were harvested by centrifugation and used to infect a 500 mL culture of early log phase XL-1 *E. coli.* Growth at 37 °C was continued for 1 hour, VCS helper phage was added to 1 x 10¹⁰ PFU/mL, and the culture was grown overnight at 30 °C in a 4 L baffled flask on a rotary shaker (200 RPM). Phage were harvested as described above and resuspended in 5 mLs of PBS. This phage stock was used in affinity-based sorting. Phage were amplified between rounds of sorting by infection of XL-1 as described above except that the culture volume used was reduced 10-fold to 50 mLs.

### EXAMPLE 2

### Sorting of phage libraries for receptor selectivity

Phage sorting for receptor binding was performed using receptor-IgG fusion proteins adsorbed on the wells of microtiter plates (Nunc-Maxisorp). Receptor-IgG proteins were diluted to 2-10 µg/mL in coating buffer (50 mM sodium carbonate pH 9.6) and 100 µL of this solution was added to several wells of a 96-well plate. In the first round of sorting, all 96 wells were used; subsequent rounds used fewer wells. The coating solution was incubated on the microliter plate at ambient temperature with gentle shaking for 2 hours. The coating solution was then removed and the wells were blocked with 200 µL of PBS containing 0.05 % Tween-20 and 5 % powdered skim milk (blocking buffer). Blocking was for 1 hour at room temperature and then the wells were rinsed with PBS/0.05% Tween-20 (wash buffer).

The Apo2L/TRAIL library phage solution was diluted 10-fold in blocking buffer and then 100 µL of this solution was added to the wells of the receptor-IgG coated plate. In addition, the diluted phage were also added to an equal number of wells of a blank plate that was prepared by blocking the wells with blocking buffer without prior protein coating. The solutions were incubated on the plates, with gentle shaking on an orbital shaker (Bellco), for 2 hours at ambient temperature. The phage solution was dumped out and the wells were rinsed by repetitive (6x) filling of the wells with wash buffer from a squirt bottle followed by dumping of the wash solution. Bound phage were eluted from the wells by addition of 100 µL of 10 mM HCl. After incubating for 20 minutes with shaking the eluant was removed by pipetting and brought to neutral pH by addition of 1/20 volume of 2 M Tris base pH 11. Half of the eluant from the receptor plate was used to infect XL-1 *E. coli* in order to propagate phage (50 mL culture) for the next round of sorting. A portion of the eluant from the receptor and blank plates was used to estimate phage concentration by titering colony forming units (CFU) with XL-1 *E. coli.* Briefly, serial 10-fold dilutions of the phage solutions were incubated with log phase XL-1 for 30 minutes at 37 °C and then the cells were streaked out on LB agar plates containing 50 µg/mL carbenecillin. After overnight incubation at 37 °C, the number of carbenecillin-resistant colonies was determined by visual inspection. Since the parent phagemid (pAPOK4.2) carries the ampicillin resistance gene, the number of colonies is proportional to the phage concentration. The enrichment of the selection for receptor binding is calculated from the ratio of the phage eluted from the receptor plate to that eluted from the blank plate.

The DR4 library was sorted for 2 rounds against DR4-IgG (construct described in Example 3 below) coated on wells followed by 3 rounds of sorting for DR4 binding in the presence of competing DR5-IgG (construct described in Example 3 below). For sorting rounds 3, 4, and 5, the phage were incubated with 50, 250, and 750 nM DR5-IgG, respectively, for 30 minutes prior to addition of these solutions to DR4-IgG coated plates. For the DR5 library, phage were sorted for 4 rounds against DR5-IgG coated wells followed by 4 rounds where DR4-IgG was used as the competitor. In rounds 5, 6, 7, and 8, phage were incubated with 1, 10, 100, and 500 nM DR4-IgG, respectively, prior to selection for DR5-IgG binding. As shown in Figure 8C, both the DR4 and DR5 libraries gave specific enrichment for receptor binding. Upon completion of sorting, individual clones from the selected libraries were screened for specific binding by "spot ELISA". Individual clones were obtained by infecting XL-1 *E. coli* with the phage pool and then streaking the cells on LB/carbenecillin agar plates. Single colonies were picked and used to inoculate 5 mL LB cultures containing 50 µg/mL carbenecillin and 1 x10¹⁰ PFU/mL VCS helper phage. After overnight growth at 30 °C, phage were harvested by PEG/NaCl precipitation of the culture supernatant. In the spot ELISA, phage clones were diluted 10-fold in binding buffer and tested for binding to different proteins coated on microtiter plate wells. Bound phage was detected by using a HRP-coupled antibody (Pharmacia) directed against the coat proteins of M13 bacteriophage. For the DR4 library clones, the test proteins were DR4-IgG, DR5-IgG, TNFR1-IgG, and BSA. Only the clones that gave an ELISA signal for DR4-IgG, and none of the other proteins, were chosen for further analysis. Test proteins for the DR5 library clones were DR5-IgG and Herceptin^{®} (Genentech, Inc., South San Francisco, CA). Clones positive for DR5-IgG binding and not Herceptin^{®} were selected for further study.

### EXAMPLE 3

### Characterization of receptor-selective clones

Receptor-selective clones from each of the 2 libraries described in Example 2 were chosen for further analysis. Single-stranded DNA was isolated from the phage particle using the Qiaprep Spin M13 kit (Qiagen) and subjected to dideoxynucleotide sequencing using the dye terminator cycle kit (Beckman-Coulter). A primer (mal-f1: 5'-TGTAAAACGACGGCCAGTCACACAGGAAACAGCCAG-3' SEQ ID NO:13) that is complimentary to a portion of the tac promoter was used to prime the sequencing reactions. The sequencing reactions were analyzed on a CEQ2000XL capillary sequencer (Beckman-Coulter) such that the entire sequence of the coding region of Apo2L/TRAIL could be determined. The amino acid identities deduced from the DNA sequence for the library positions are shown in Table II (DR4-selective) and Table III (DR5-selective). No spurious sequence changes outside of the library positions were detected.

Relative binding strengths for 4 of the clones from the DR4 library were determined by competition phage ELISA (Figures 15 and 16). In this assay, a fixed concentration of phage is added to wells coated with receptor-IgG in the presence of an increasing concentration of receptor-IgG in solution. After incubation to allow binding and washing to remove unbound phage, the bound phage is determined with the HRP-coupled, anti-M13 antibody (Pharmacia). Analysis of the ELISA signal as a function of receptor-IgG concentration in solution by using a 4-parameter fit yields the IC50 value. Since the IC50 value will vary with phage concentration, the phage clones were first titered to determine the dilution giving equal signal strength for the 4 clones. All 4 clones gave IC50 values for DR4-binding similar to, or slightly smaller than, that measured for wild-type (native sequence) Apo2L/TRAIL displayed on phage (Figure 15). In contrast, none of the clones appeared to bind to DR5-IgG as indicated by the lack of competition by soluble DR5-IgG (Figure 16).

A few representative sequences from the DR5 library were subcloned into pAPOK5.0 for further testing. Subcloning was performed by doing a PCR reaction on the phage clones using 2 oligonucleotide primers that are complimentary to the 5' and 3' ends of the Apo2L/TRAIL coding segment. After restriction digest with MluI and BamHI, the PCR fragments were ligated with MluI/BamHI cleaved pAPOK5.0 such that the wild-type Apo2L/TRAIL sequence was replaced with mutant DNA. Sequences were confirmed by dideoxynucleotide sequencing.

Apo2L/TRAIL(114-281) mutants (variants) were expressed and purified, and the purified proteins were assayed for receptor-binding by BIAcore^{®} and bioactivity on SK-MES lung carcinoma cells, as previously described (see, e.g. Hymowitz et al., (2000) Biochemistry 39, 633-640). Briefly, dissociation constants (Kd) for binding of Apo-2L variants (see Tables II and III) to immobilized receptor immunoadhesins were determined from surface plasmon resonance (SPR) measurements on a Pharmacia BIAcore 3000. DR5-IgG (also referred to as Apo-2-IgG) and DcR2-IgG receptor immunoadhesins were prepared as described in WO98/51793 published November 19, 1998 and WO99/10484 published March 9, 1999, respectively. DR4-IgG was prepared as follows. A mature DR4 ECD sequence (amino acids 1-218; Pan et al., supra) was cloned into a pCMV-1 Flag vector (Kodak) downstream of the Flag signal sequence and fused to the CH1, hinge and Fc region of human immunoglobulin G₁ heavy chain as described previously [Aruffo et al., Cell, 61:1303-1313 (1990)]. The immunoadhesin was expressed by transient transfection into human 293 cells and purified from the cell supernatants by protein A affinity chromatography, as described by Ashkenazi et al., Proc. Natl. Acad. Sci., 88:10535-10539 (1991)]. The receptor immunoadhesin proteins were coupled to the sensor chip surface at a level of 300-500 resonance units using amine coupling chemistry (Pharmacia Biosensor). Sensorgrams were recorded for Apo-2L binding at concentrations ranging from 15.6 nM to 500 nM in 2-fold increments. The kinetics constants were determined by nonlinear regression analysis and used to calculate the binding constants.

Alamar Blue assays were used to characterize the apoptotic activity of Apo-2L variants *in vitro.* SK-MES cells express both DR4 and DR5 and are sensitive to apoptosis-induction by wild-type Apo2L/TRAIL. Briefly, a bioassay which measures cell viability from the metabolic conversion of a fluorescent dye was used to determine the apoptotic activity of Apo-2L variants. Serial 2-fold dilutions of Apo-2L (form 114-281) or Apo-2L variants (see, e.g. Tables II and III) were made in RPMI-1640 media (Gibco) containing 0.1% BSA, and 50 µL of each dilution was transferred to individual wells of 96-well Falcon tissue culture microplates. 50 µL of SK-MES-1 human lung carcinoma cells (ATCC HTB58) (in RMPI-1640, 0.1 % BSA) were added at a density of 2 x 10⁴ cells/well. These mixtures were incubated at 37 °C for 24 hours. At 20 hours, 25 µL of alamar Blue (AccuMed, Inc., Westlake, Ohio) was added. Cell number was determined by measuring the relative fluorescence at 590 nm upon excitation at 530 nm. These data were analyzed by using a 4 parameter fit to calculate ED₅₀, the concentration of Apo-2L giving a 50% reduction in cell viability.

As summarized in Table IV, the DR5-selective mutants usually give reduced affinity for DR4 and affinity for DR5 comparable to or slightly reduced from wild-type Apo2L/TRAIL(114-281). Surprisingly, two of the DR5-selective mutants (DR5-21 and DR5-23, as identified in Table III) having 10-fold and 100-fold reduced affinity for DR4, respectively, retained high activity for apoptosis-induction on SK-MES cells in vitro.

### EXAMPLE 4

### Production and testing of flag-tagged Apo2L/TRAIL variants

It has been proposed that DR5 signals only in response to cross-linked Apo2L/TRAIL whereas DR4 can respond to non-cross-linked as well as cross-linked ligand (see, e.g. Muhlenbeck et al., (2000) J. Biol. Chem. 275, 32208-32213). To examine the signaling and activity profile of the receptor-selective variants described herein, epitope-tagged versions of several of the variants were prepared and assayed. The mutants were produced with an N-terminal flag-tag which enables crosslinking with the M2 anti-flag antibody (Sigma-Aldrich Chemical Co.).

Plasmids designed for *E. coli* expression of flag-tagged Apo2L/TRAIL variants were constructed by oligonucleotide-directed mutagenesis of a plasmid (pFLAG-Apo2L; Genentech) having Apo2L/TRAIL(114-281) inserted in pFLAG-MAC (Sigma). PFLAG-Apo2L directs the cytoplasmic expression of N-terminally flag-tagged Apo2L/TRAIL(114-281) under control of the tac promoter. The template for mutagenesis was the single-stranded version of the plasmid produced using the protocol of Kunkel (see, e.g. Kunkel, T.A. (1985) Proc. Natl. Acad. Sci. (USA) 82, 488-492). Mutants were identified by dideoxynucleotide sequencing. For expression of the Apo2L/TRAIL variants, the mutant plasmids were transformed into *E. coli* strain 43E7. The transformed E. coli were grown to early log phase at 37 °C in 500 mLs of 2YT media containing 50 µg/mL carbenecillin and expression was induced by addition of IPTG to a final concentration of 0.4 mM. The flag-tagged Apo2L/TRAIL variants were purified as previously described for untagged proteins (see, e.g. Hymowitz et al., (2000) Biochemistry 39, 633-640) except that a lower pH was used for the cation exchange column since the acidic peptide flag results in a lower pI for the protein.

DR4 selective variant clone #'s 8 and 9, and DR5 selective variant clone #'s 1, 2, 8, 21, and 23 were chosen for production as flag-tagged proteins. As shown in Table V, the purified flag-tagged Apo2L/TRAIL variants were initially tested for DR4 and DR5 binding by BIAcore^{®} and for apoptosis-induction on SK-MES with and without anti-flag cross-linking. Both Flag-Apo2L.DR4-8 and 9 had >1000-fold reduced affinity for DR5 while retaining high affinity binding to DR4. Flag-Apo2L.DR4-8 had 5-fold increased affinity for DR4 whereas Flag-Apo2L.DR4-9 had 4-fold decreased affinity. Both DR4-selective proteins had greatly reduced activity for apoptosis-induction on SK-MES cells. The fold increase in activity upon anti-flag cross-linking was also much smaller than observed with wild-type Flag-Apo2L. Anti-flag cross-linking did not increase the activity of Flag-Apo2L.DR4-8, and caused only a 6-fold increase in the activity of Flag-Apo2L.DR4-9, as compared to the 30-fold increase in activity observed for the wild-type protein.

All of the DR5-selective variants had reduced affinity for DR4 while maintaining high affinity binding to DR5. Flag-Apo2L.DR5-1, 2, and 21 had 11-fold reduced affinity for DR4 with only 1-2-fold decreased binding to DR5. Flag-Apo2L.DR5-23 had 100-fold reduced affinity for DR4 but only 2.4-fold decreased binding to DR5. No binding to DR4 was detected for Flag-Apo2L.DR5-8 but this variant had binding to DR5 equal to or greater than the wild-type protein. In contrast to the results observed with the DR4-selective variants, all of the DR5-selective proteins retained high levels of activity for apoptosis-induction on SK-MES. Indeed, Flag-Apo2L.DR5-1, 2, and 8 showed increased activity (lower ED₅₀) for apoptosis-induction relative to the wild-type protein. Upon cross-linking with anti-flag antibody, all of the DR5-selective variants increased in activity to the value (ED₅₀) observed for cross-linked wild-type Apo2L. These results suggested that DR5 binding may be more important than DR4 binding in signaling apoptosis on SK-MES cells. It is believed, though not fully understood, that since some of the variants having decreased affinity for DR4 are more potent for apoptosis-induction, DR4 may signal only weakly and may attenuate the signal produced by DR5.

### EXAMPLE 5

### Activity of Flag-Apo2L/TRAIL variants on Colo205 and Jurkat T cells

The apoptosis-inducing activity of Flag-Apo2L.DR4-8 and Flag-Apo21.DR5-8 was also examined using Colo205 colon carcinoma cells and Jurkat T cells. Colo205 cells express both DR4 and DR5 and are more sensitive than SK-MES to apoptosis-induction by wild-type Apo2L/TRAIL. Jurkat T cells appear to only express DR5. The Colo205 cells were more sensitive to the DR5-selective variant than to wild-type Apo2L/TRAIL (Figure 17A). Anti-flag cross-linking of Flag-Apo2L.DR5-8 did not result in a significant further increase in activity on Colo205. In contrast, Colo205 were not very sensitive to Flag-Apo2L.DR4-8 (Figure 17B). Anti-flag cross-linking of this variant caused a small increase in activity. Jurkat T cells were not sensitive to Flag-Apo2L.DR4-8 independent of ligand cross-linking (Figure 18B). Flag-Apo2L.DR5-8 induced apoptosis on Jurkat T cells without cross-linking (Figure 18A). Anti-flag cross-linking increased the activity to the level measured with cross-linked wild-type Flag-Apo2L.

### EXAMPLE 6

### Receptor binding by AlphaQuest assay

The binding of the Apo-2L variants to the 5 known Apo2L/TRAIL receptors (DcR1, DcR2, OPG, DR4, DR5) was also examined using an AlphaQuest^{®} assay. This assay produces a signal when a "donor" and "acceptor" bead are brought in close proximity facilitating singlet oxygen-mediated, fluorescence resonance energy transfer. In this case the donor bead is coated with streptavidin and is used to capture biotinylated Apo2L/TRAIL. The acceptor bead is coated with *Staphylococcal* Protein A and is used to capture the receptor-IgG protein. Binding of Apo2L/TRAIL to the receptor brings the beads in close proximity and transmits the signal. IC₅₀ values for binding can be determined by displacing the biotinylated ligand with unbiotinylated ligand. Displacement curves were generated for each of the Apo2L/TRAIL variants and were used to determine the relative IC₅₀ values shown in Table V_{I}. For DR4 and DR5 binding, the changes in IC₅₀ values are consistent with the trends measured by BIAcore^{®}. The Apo-2L variants assayed showed greatly diminished binding to OPG. The DR4-selective variants exhibited affinity for DcR1 and somewhat weaker affinity for DcR2. All of the DR5-selective variants exhibited significantly reduced affinity for DcR1 but, with the exception of Flag-Apo2L.DR5-8, retained more binding capacity for DcR2 as compared to DcR1. Flag-Apo2L.DR5-8 exhibited greatly reduced affinity to DcR1, DcR2, OPG, and DR4 but bound DR5 with relatively high affinity.

### EXAMPLE 7

### Effects of Apo-2L variants on normal cyno monkey hepatocytes

Two of the receptor-selective variants (Flag-Apo2L.DR4-8 and Flag-Apo2L.DR5-23) were examined for effects on normal cells by measuring hepatocyte viability upon exposure to ligand in vitro. Hepatocytes are typically not sensitive to Apo2L/TRAIL unless the ligand is aggregated (see, e.g. Lawrence et al., (2001) Nature Medicine 7, 383-385). For the assay, hepatocytes from cynomologous monkey were used and viability was measured by crystal violet staining. The DR4-selective and DR5-selective variants were less toxic to hepatocytes upon anti-flag cross-linking than the cross-linked, wild-type ligand (Figure 19).

### EXAMPLE 8

### Generation of Apo2L cysteine substitution variants

Cysteine substitution variants of Apo-2L were constructed by oligonucleotide-directed mutagenesis (Kunkel et al., Proc. Natl. Acad. Sci., 82:488-492 (1985); Kunkel, Methods in Enzymology, 154:367-382 (1987)) on the single-stranded form of the plasmid pAPOK5.0. This plasmid was designed for the intracellular *E*. *coli* expression of the 114-281 amino acid form of Apo2L driven by the tryptophan (trp) promoter. PAPOK5.0 was constructed from pAPOK5 (WO 99/36535 published July 22, 1999) by deletion mutagenesis of the DNA segment encoding residues 91-113 of Apo-2L shown in Figure 1. pAPOK5 was constructed by using PCR to clone the Apo-2L cDNA (encoding residues 91-281 of Figure 1) into plasmid pS1162 which carries the trp promoter. After mutagenesis, the identity of the plasmids was confirmed by dideoxynucleotide sequencing (Sanger) of the entire Apo2L portion of the plasmid.

Plasmids encoding the cysteine-substituted proteins were then transformed into *E. coli* strain 294 for expression. Cultures were grown overnight to saturation at 37°C in Luria broth plus carbenecillin at 50 µg/mL. The saturated cultures were subsequently seeded at a 50-fold dilution into sterile-filtered media comprised of Na₂HPO₄ (6 g/L), KH₂PO₄ (3 g/L), NaCl (0.5 g/L), NH₄Cl (1 g/L), glucose (4.9 g/L), Casamino acids (4.9 g/L), 27 mM MgSO₄ 0.003% Thiamine HCl and q.s. with distilled water plus carbenicillin at 40 µg/mL. The cultures were grown at 37°C until the A500 was 0.5 - 0.8 and then expression was induced by addition of 3-α-indoleacrylic acid (IAA) (Sigma, St. Louis, MO) to a final concentration of 25 µg/mL. Cells were grown overnight at 30°C with shaking, harvested by centrifugation and stored frozen at -20°C for subsequent recovery of Apo2L as described below.

The Apo-2L proteins were extracted from the frozen *E. coli* cell pellets by homogenization in 10 volumes (wt/vol) of 100mM Tris, pH8.0/200mM NaCl/5mM EDTA/1mM DTT using a model M110-F Microfluidizer (Microfluidics Corporation, Newton, MA). Polyethyeneimine (PEI) was added to a final concentration of 0.5% (vol/vol) to the homogenate which was then centrifuged to remove cell debris. Solid ammonium sulfate was added to the extraction supernatant to a final concentration of 45% saturation at ambient temperature with stirring, and the pellet was recovered by centrifugation. The ammonium sulfate pellet was washed with 50% ammonium sulfate solution to remove residual EDTA, then resuspended in 50 volumes (wt/vol) of 50mM HEPES, pH 7.5/0.1% Triton X-100. The resulting solution was clarified by centrifugation and purified by immobilized metal affinity chromatography (IMAC) using a 5 mL HiTrap Chelating Sepharose column (Pharmacia, Piscataway, NJ). The column was charged with nickel in 100mM NiSO₄/300mM Tris, Ph 7.5 and equilibrated with -350mM NaCl in phosphate-buffered saline (PBS). After loading, the column was washed with 350mM NaCl in PBS and eluted with 50mM Imidazole/350mM NaCl in PBS. The IMAC eluant was dialyzed against 20mM Tris, pH7.5, clarified by centrifugation, and further purified by cation exchange chromatography using a 5mL HiTrap SP Sepharose column (Pharmacia), which was equilibrated and washed with 20mM Tris, pH7.5. The HiTrap SP column was eluted with 20mM Tris, pH7.5/0.5M NaCl. The SP column eluent was reduced with 2mM DTT and subsequently precipitated by adding solid ammonium sulfate with stirring to a final concentration of 45% saturation at ambient temperature. The ammonium sulfate pellet was resuspended in 3.5 mL of 20mM Tris, pH7.5/100mM NaCi and exchanged into the final buffer of 20mM Tris, pH7.5/100mM NaCl/2mM DTT by gel filtration chromatography using a PD10 column (Pharmacia). The purified Apo-2L cysteine-substituted proteins were characterized by Coomassie-stained SDS-PAGE and mass spectroscopy, and stored frozen at -20°C.

### EXAMPLE 9

### Pegylation of Apo-2L on Cys residues

Cysteine-substituted Apo2L proteins were covalently modified by reaction with methoxy-PEG-maleimide MW 2,000, 5,000 or 20,000 D (Shearwater Polymers), or alternatively, iodoacetamide (IAM). The Apo2L variants were prepared for pegylation by first removing the DTT contained in the storage buffer by passage over a PD-10 gel filtration column. The column was equilibrated and eluted with HIC buffer (0.45 M Na₂SO₄, 25 mM Tris-HCI pH 7.5), or arginine formulation buffer (0.5 M Arg-succinate, 20 mM Tris-HCl pH 7.5). An aliquot of a PEG-maleimide solution (10 mM in dH₂O) was added immediately. Molar concentration ratios of PEG-maleimide to cysteine variant-Apo2L.0 monomer of 1:1, 2:1, 5:1 or 10:1 and reaction times of 2 or 24 hours were used. The reactions were terminated by addition of DTT to 2mM, followed by a 30 minute incubation at ambient temperature, and then iodoactemide was added to 10mM. This quenching procedure ensured that any disulfide bonds formed during the reaction procedure were reduced and any unpegylated Cys thiol became carboxyamidomethylated. Modification with iodoacetamide was for 30 minutes and then the excess reagents were removed by gel filtration on a NAP-5 column (Pharmacia) equilibrated and eluted with PBS. These samples were analyzed by SDS-PAGE and SEC-MALS. Apoptosis-inducing activity on SK-MES cells was also assayed as described herein.

### EXAMPLE 10

### Preparation of partially PEGylated Apo2L cysteine variants

In an effort to generate APO2L trimers having properties which combine the slower clearance of PEGylated Apo2L cysteine variants while avoiding disulfide dimer formation that can occur at cysteine residues, PEGylation experiments were conducted in which, rather than introducing cys into the Apo2L monomers and then attaching 3 PEG molecules to the trimer, only 1-2 PEG molecules were attached to the trimer, with the third remaining cys being blocked with carboxymethyl iodoacetamide (IAM) to block dimer formation.

Trimeric forms of APO-2L having 1-2 covalently attached PEG chains per trimer are believed to exhibit a number of coexisting optimal characteristics including a significant bioactivity profile and a decrease in the tendency to form disulfide dimers.

Partial PEGylation of R170C-Apo2L with 5K or 20K PEG-maleimide was conducted as follows. The R170C-Apo2L was prepared for modification by first removing the DTT by passage of the protein solution over a PD-10 (Amersham Biotech) gel filtration column equilibrated with arginine-succinate formulation buffer. Protein concentration was immediately determined by absorbance measurements at 280 nm and then PEG-maleimide (5000 or 20,000 molecular weight; Shearwater, Inc.) was added to a final ratio of 0.7 PEG: 1.0 Apo2L monomer. The PEG-maleimide was from a freshly prepared stock of 10 mM in water. The reaction solution was incubated overnight at ambient temperature. The PEGylation reaction was quenched by addition of DTT to a final concentration of 2 mM, this solution was incubated for 1 hour at room temperature, and then iodoacetamide was added to 10 mM. After a further overnight incubation, the PEGylated protein was fractionated on a Sephacryl S-200 (Amersham Biotech) gel filtration column eluted with PBS. Protein elution was monitored by absorbance at 280 nm and protein containing fractions eluting earlier than unmodified Apo2L/TRAIL were collected and pooled. This procedure was used to partially PEGylate R170C-Apo2L with a 5K or 20K PEG-maleimide. These preparations were analyzed by gel filtration chromatography with on-line light scattering (SEC-MALS) (Figure 14) which showed that they were comprised of mixtures containing predominantly trimers with 1 or 2 PEG chains per trimer. Both preparations contained smaller amounts of trimers with 0 or 3 PEG chains per trimer. As shown in Figure 10G, these mixtures retained high levels of activity for apoptosis-induction on SK-MES cells. Both 5Kp-R170C.0 and 20Kp-R170C.0 gave a longer half-life in the mouse than Apo2L.0 (Figure 11A, B) and caused a greater reduction in tumor volume in the xenograft model than unmodified wild-type Apo2L (Figure 12A, B).

### EXAMPLE 11

### Apoptotic activity of native and pegylated cysteine variants

A bioassay which measures cell viability from the metabolic conversion of a fluorescent dye was used to determine the apoptotic activity of native and pegylated Apo2L cysteine variants (see, e.g. see, e.g. Hymowitz et al., (2000) Biochemistry 39, 633-640). Briefly, serial 2-fold dilutions of Apo-2L.0 or Apo2L variants were made in RPMI-1640 media (Gibco) containing 0.1% BSA, and 50 µL of each dilution was transferred to individual wells of 96-well Falcon tissue culture microplates. 50 µL of SK-MES-1 human lung carcinoma cells (ATCC HTB58) (in RMPI-1640, 0.1% BSA) were added at a density of 2 x 10⁴ cells/well. These mixtures were incubated at 37°C for 24 hours. At 20 hours, 25 µL of alamarBlue (AccuMed, Inc., Westlake, Ohio) was added. Cell number was determined by measuring the relative fluorescence at 590 nm upon excitation at 530 nm. These data were analyzed by using a 4 parameter fit to calculate ED₅₀, the concentration of Apo2L.0 giving a 50% reduction in cell viability.

Results of these assays are shown in Figure 9.

### EXAMPLE 12

### Pharmacokinetics of PEG-R170C-Apo2L.0

The effect of PEGylation on the clearance of Apo2L was tested in the mouse. Mice were given i.p. injections of Apo2L.0 (10 mg/kg), PEG-Rl70C-Apo2L.0 (10 mg/kg) or PEG-K179C-Apo2L (10 mg/kg) at time zero. Plasma samples were collected at 24 hours. Apo2L concentrations were determined by ELISA. As shown in Figures 11A and 11B, Apo2L.0 was rapidly cleared from the circulation whereas PEG-R170C-Apo2L and PEG-K179C-Apo2L were cleared more slowly. Site-specific attachment of PEG to the Apo2L variants thus resulted in a significant decrease in the rate of clearance.

### EXAMPLE 13

### Effect of PEG-R170C-Apo2L.0 and PEG-K179C-Apo2L on the growth of human COL0205 tumors in a mouse xenograft model

Athymic nude mice (Jackson Laboratories) were injected subcutaneously with 5 x 10⁶ COL0205 human colon carcinoma cells (NCI). Tumors were allowed to form and grow to a volume of about 500-1500 mm³ as judged by caliper measurement. Mice were given i.p. injections of vehicle (2x/week), Apo2L.0 (10 mg/kg, 2x/week), PEG-K179C-Apo2L.0 (10 mg/kg, 2x/week) or PEG-R170C-Apo2L.0 (10 mg/kg, 2x/week). Tumor volume was measured every third day and treatment was stopped after two weeks. As shown in Figures 12A and 12B, treatment with 10 mg/kg PEG-Rl70C-Apo2L.0 or PEG-Kl79C-Apo2L.0 caused a greater reduction in tumor volume than an equivalent dose of Apo2L.0. PEGylation of Apo2L on Cysl70 or Cys179 thus appeared to lower the dose required to achieve efficacy in this xenograft model of human cancer.

### EXAMPLE 14

### Selection of substitutions for DRS receptor selective variants

A phage display approach was used to examine the role in determining DR5-selectivity of the substitutions observed in variant Apo2L/TRAIL.DRS-8. A "revertant library" was constructed in which the residues at 189, 191, 193, 264, 266 and 267 were allowed to vary as either the wild-type residue or the DR5-8 amino acid. The following codons were used: 189 - YAS, encoding Tyr, Amber, Gln, His; 191 - ARR, encoding Arg and Lys; 193 - CRA, encoding Gln or Arg; 264 - CRC, encoding His and Arg; 266 - MTT, encoding Ile and Leu; 267 - SAS, encoding His, Gln, Asp, and Glu. The diversity of this library was 192 amino acid sequences described by 512 nucleotide sequences. Kunkel mutagenesis was used to construct this library as described in Example 1 except that XL-1 *E. coli* were used for electroporation. This library gave a titer of 2.5 x 10¹⁰ clones, sequencing showed 5/12 were correctly mutated, to give an actual library size of 1 x 10¹⁰. Phage were produced and harvested as described above.

The revertant library was sorted for 1 round against DR5-IgG coated on microtiter plate wells followed by 3 rounds of sorting for DR5 binding in the presence of competing DR4-IgG. For sorting rounds 2, 3, and 4, the phage were incubated with 100 nM, 1 µM, and 4 µM DR4-IgG, respectively, for 30 minutes prior to addition of these solutions to the DR5-IgG coated plates. By the third round of sorting, enrichment >1000-fold was obtained. Spot ELISA indicated that after round 2 sorting 22/24 clones were positive for DR5 binding and 7/24 were capable of binding to DR4. After round 3, 24/24 clones bound DR5 and only 1/24 bound to DR4. The nucleotide sequence of the single-stranded DNA isolated from these 48 phage clones was determined as described above. The amino acid identities deduced from the DNA sequence at the library positions for the clones positive for DR5-, but not DR4-, binding are shown in Table VII. These results indicate that the residue identity at positions 264 and 267 may not be important for DR5-selectivity since there is no preference for the DR5-8 amino acid at these positions. In contrast, the DR5-8 amino acid is predominant at positions 189, 191, 193 and 266 indicating that these sites may be important for DR5-selectivity. At position 189 there was a strong preference for Gln which can be expressed through a Gln codon or through suppression of an amber stop codon.

On the basis of the preferences observed in the phage selection, two variants were subcloned into pAPOK5.0 0 for further testing. DR5-8B differs from wild-type Apo2L/TRAIL by Y189Q, R191K and Q193R, and DR5-8C has the additional substitution of I266L. These variants were expressed and purified, the purified proteins were assayed for receptor-binding by BIAcore^{®} and bioactivity on SK-MES lung carcinoma cells, as previously described (see, e.g. Hymowitz et al. (2000) Biochemistry 39, 633-640). As shown in Table VIII, both DR5-8B and DR5-8C have reduced affinity for DR4 while having slightly increased affinity for DRS. Variant DR5-8C has activity for apoptosis-induction, as reflected in the ED50 value, equivalent to- or slightly better than- that observed for the wild-type protein. DR5-8B showed about a 3-fold decrease in activity for apoptosis-induction. By comparison, DR5-8 had a larger decrease in affinity for DR4 while maintaining apoptosis-induction activity equivalent to wild-type Apo2L/TRAIL. In this experiment the affinity of DR5-8 for DR4 was too weak to accurately measure the Kd value. These results suggest that the amino acid substitutions H264R and D267Q provide further selection against DR4 binding.

### EXAMPLE 15

### Effect of PEG chain length on anti-tumor activity of PEG-K179CApo2L in a mouse xenograft model

The effect of PEG chain length on anti-COL0205 tumor activity was examined in the mouse xenograft model described above. For these experiments, the K179C variant of Apo2L/TRAIL(114-281) was tested. K179C-Apo2L was reacted with a 2:1 molar ratio of methoxy-PEG-maleimide of MW 1,000, 2,000, or 5,000 under conditions (described above, Example 9) that resulted in attachment of 3 PEG chains per trimer (1 per monomer). Trace levels of non-PEGylated protein, and also excess unreacted PEG-maleimide, were removed from the PEG-K179C-Apo2L preparation by cation-exchange chromatography on a column of CM-Sepharose. Analytical gel filtration (Figure 20) of the 2000-PEG-K179C indicated a homogeneous, PEGylated trimeric protein with a molar mass calculated from light scattering data consistent with attachment of 3-2000 MW PEG chains to the trimer. Each of the PEGylated-K179C-Apo2L preparations was assayed for apoptosis-induction on SK-MES cells. PEGylation of K179C-Apo2L resulted in an increase in ED50 in this assay with the magnitude of the increase dependent on PEG chain length. The 1000 MW PEG-K179CApo2L had a 4.3-fold increased ED50, the ED50 for 2000 MW PEG-K179C-Apo2L increased 8.7-fold, and the 5000 MW PEG-K179C-Apo2L had the weakest activity with a 23.2-fold increased ED50, all relative to Apo2L.0.

The dependence of clearance on PEG chain length was tested in the mouse. Pharmacokinetic experiments were conducted as described above except that injections of PEG-K179C-Apo2L or Apo2L.0 were given i.v. (intravenous). Injected doses were 5 or 30 mg/kg for Apo2L.0, 30 mg/kg for 1000 MW PEG-K179C-Apo2L, and 5 mg/kg for the 2000 and 5000 MW PEG-K179C-Apo2L. For the 5 mg/kg Apo2L.0 control, and the 2000 and 5000 MW PEG-K179C-Apo2L, plasma samples were collected at 1 minute, 2, 6, and 24 hours post injection. For the 1000 MW PEG-K179C-Apo2L and 30 mg/kg Apo2L.0 only the 1 minute and 24 hour samples were obtained. Apo2L concentrations were determined by ELISA to yield the pharmacokinetic curves shown in Figure 21. The data show that PEGylation results in reduced clearance of Apo2L with the half-life increasing with the chain length of the PEG. At 24 hours, for the 5 mg/kg doses, the plasma concentrations of the two PEGylated species are above the ED50s measured in the in vitro bioassay whereas Apo2L.0 declined to undetectable levels.

The 1000, 2000, and 5000 MW PEG-K179C-Apo2L were tested for effects on growth of COLO205 tumors in the mouse xenograft model. For these experiments a single i.v. dose (30 mg/kg) was used. The effects for the PEGylated proteins were compared to that observed for a single 30 mg/kg i.v. dose of Apo2L.0 and also to a "standard" treatment of 60 mg/kg Apo2L.0 i.p., 5x/wk for 1 week. Tumor volumes were measured for 2 weeks. As shown in Figure 22, the 2000 MW PEG-K179C-Apo2L gave an inhibition of tumor growth similar to the same dose of Apo2L.0 while the 5000 MW PEG-K179C-Apo2L showed less inhibition of tumor growth. The 1000 MW PEG-K179C-Apo2L gave a greater inhibition of tumor growth than the equivalent dose of Apo2L.0. Significantly, the activity approached that of the standard treatment which involves a 10-fold higher dose of Apo2L.0. The increased half-life of 1000 MW PEG-K179C-Apo2L, coupled with a high retention of bioactivity, resulted in a more efficacious molecule.

### TABLES

**Table I. Phage display of functional Apo2L/TRAIL on phage as determined by enrichment for specific DR5-IgG-binding.**

| Phagemid | Growth Temperature (°C) | Enrichment |
|---|---|---|
| PAPOK4 | 37 | 1.5 |
| PAPOK4 | 30 | 20 |
| PAPOK4.2 | 37 | 3.3 |
| PAPOK4.2 | 30 | 100 |

Enrichment was calculated from ratio of phage bound and eluted from DR5-IgG well to that observed for a blank well. Two wild-type Apo2L/TRAIL phagemid constructs were tested at two different growth temperatures.

**Table II. Amino acid sequence at library positions deduced from DNA sequence for DR4-selective phage clones. Residue**

| Clone# | 189 | 191 | 193 | 199 | 201 | 209 |
|---|---|---|---|---|---|---|
| WT | Y | R | Q | N | K | Y |
| 1 | X | R | R | K | H | Y |
| 2 | X | R | X | G | H | Y |
| 3 | X | R | S | G | A | Y |
| 4 | X | R | X | G | H | Y |
| 5 | A | R | T | G | X | Y |
| 6 | X | R | V | G | H | Y |
| 7 | X | R | R | G | H | Y |
| 8 | A | R | S | V | R | Y |
| 9 | A | R | S | R | R | Y |
| 10 | A | R | X | G | T | Y |
| 11 | A | R | S | G | S | Y |
| 12 | X | R | T | G | H | Y |
| 13 | A | R | K | G | G | Y |
| 14 | X | R | S | G | H | Y |

"X" indicates a position where residue identity could not be determined because of poor quality sequencing data. In the present disclosure, "DR4-8", for example, refers to the DR4 selective variant clone no. 8 identified in the Table. "WT" = wild-type

**Table III. Amino acid sequence at library positions deduced from DNA sequence for DR5-selective phage clones.**

| Residue | | | | | | | |
|---|---|---|---|---|---|---|---|
| Clone# | 189 | 191 | 193 | 264 | 266 | 267 | 269 |
| WT | Y | R | Q | H | I | D | D |
| 1 | A | K | K | A | I | D | D |
| 2 | A | K | K | H | I | D | D |
| 3 | S | K | K | G | L | D | S |
| 4 | A | R | R | Q | L | D | N |
| 5 | S | R | T | G | L | D | N |
| 6 | Q | K | R | D | M | S | D |
| 7 | A | R | K | D | L | E | S |
| 8 | Q | K | R | R | L | Q | D |
| 9 | G | R | K | P | V | D | A |
| 10 | G | K | K | G | L | D | S |
| 11 | A | R | K | G | V | D | S |
| 12 | S | R | K | S | M | D | D |
| 13 | Q | K | K | D | L | D | D |
| 14 | G | K | R | E | -L | D | A |
| 15 | S | K | R | K | M | D | S |
| 16 | A | R | K | N | L | E | R |
| 17 | A | R | K | D | L | E | S |
| 18 | A | K | K | K | V | D | S |
| 19 | Q | R | R | G | L | N | D |
| 20 | A | K | K | D | L | N | D |
| 21 | A | K | K | D | L | N | E |
| 22 | Q | R | K | G | L | E | D |
| 23 | A | K | R | S | L | D | E |
| 24 | G | K | K | A | V | D | D |

**Table IV. Receptor-binding and apoptosis-inducing activity of DR5-selective Apo2L/TRAIL mutant proteins.**

| Ratio (mutant/wt) | | | |
|---|---|---|---|
| Clone # | **Apoptosis (ED₅₀)** | **DR4-binding** K*_{D}* | **DR5-binding** K*_{D}* |
| DR5-4 | 2.5 | 3.1 | 1.3 |
| DR5-5 | 7.9 | 12.4 | 2.5 |
| DR5-11 | 2.2 | 5.0 | 2.2 |
| DR5-21 | 1.6 | 11.2 | 1.2 |
| DR5-23 | 1.8 | 105 | 2.4 |

DR5-selective mutants were produced in the 114-281 construct of Apo2L/TRAIL, purified, and assayed for apoptosis-induction on SK-MES, and receptor binding by BIAcore. The ratio of mutant to wild-type values are given.

**Table V. Receptor-binding and apoptosis-induction for flag-tagged versions of DR4-selective and DR5-selective Apo2L/TRAIL variants. K_{D} values determined by BIAcore analysis are reported relative to the value calculated for the wild-type protein. Apoptosis-induction is evaluated from the ED₅₀ value (ng/mL) in the presence and absence of 2 µg/mL M2 antibody (Sigma).**

| **Mutant** | **DR4 K_{D} (mut/wt)** | **DR5 K_{D} (mut/wt)** | **Apoptosis-induction on SK-MES ED₅₀ (ng/mL)** | |
|---|---|---|---|---|
| | | | **+anti-Flag** | |
| Flag.Apo2L.WT | 1 | 1 | 21.0 | 0.7 |
| Flag.Apo2L.DR4-8 | 0.2 | 1100 | 4000 | 4100 |
| Flag.Apo2L.DR4-9 | 4.3 | 1200 | 1000 | 170 |
| Flag.Apo2L.DR5-1 | 11.0 | 2.2 | 4.5 | 0.7 |
| Flag.Apo2L.DR5-2 | 11.0 | 1.9 | 2.9 | 0.7 |
| Flag.Apo2L.DR5-8 | NB | 0.8 | 1.3 | 0.6 |
| Flag.Apo2L.DR5-21 | 11.2 | 1 | 19.1 | 0.5 |
| Flag.Apo2L.DR5-23 | 105 | 2.4 | 90.6 | 0.4 |

**Table VI. Receptor-binding for the flag-tagged Apo2L/TRAIL variants determined by CARB-AQ assay. Mutants are compared to wild-type Flag-Apo2L/TRAIL on the basis of the IC₅₀ value. The">" symbol indicates that the IC₅₀ value was greater than the highest concentration of mutant tested in the assay and thus only a lower limit can be estimated for the fold change in receptor binding. )**

| IC50 ratio (mutant/wt | | | | | |
|---|---|---|---|---|---|
| **Protein** | **DcR1** | **DcR2** | **OPG** | **DR4** | **DR5** |
| Flag-Apo2L.WT | 1 | 1 | 1 | 1 | 1 |
| Flag-Apo2L.DR4-8 | 5.5 | 15 | >28 | 1 | >25 |
| Flag-Apo2L.DR4-9 | 4.0 | 18 | >28 | 1.1 | >25 |
| Flag-Apo2L.DR5-1 | >300 | 32 | >700 | >30 | 2.4 |
| Flag-Apo2L.DR5-2 | >300 | 5.0 | >700 | >30 | 2.8 |
| Flag-Apo2L.DR5-8 | >300 | >1900 | >700 | >30 | 0.7 |
| Flag-Apo2L.DR5-21 | >24 | 9.4 | >28 | >5 | 1.9 |
| Flag-Apo2L.DR5-23 | >24 | 24 | >28 | >5 | 2.3 |

**Table VII. Sequences selected from revertant library**

| Sequence No. | 189 | 191 | 193 | 264 | 266 | 267 |
|---|---|---|---|---|---|---|
| 1 | X | K | R | D | L | N |
| 2 | X | K | R | H | L | D |
| 3 | X | K | R | H | L | D |
| 4 | Q | K | R | H | L | D |
| 5 | Q | K | R | D | L | D |
| 6 | X | K | R | H | I | E |
| 7 | X | K | R | H | L | E |
| 8 | X | K | R | A | L | D |
| 9 | X | K | R | H | L | E |
| 10 | Q | K | R | H | I | D |
| 11 | X | K | Q | H | L | Q |
| 12 | X | K | R | P | L | D |
| 13 | X | K | Q | H | L | D |
| 14 | X | R | R | H | L | E |
| 15 | X | K | R | R | L | E |
| 16 | A | K | R | H | L | D |
| 17 | X | K | R | T | L | D |
| 18 | X | K | R | R | L | E |
| 19 | X | K | R | P | V | N |
| 20 | Q | K | R | H | L | Q |
| 21 | X | R | Q | H | L | Q |
| 22 | X | K | R | A | L | D |
| 23 | X | K | R | D | L | E |
| 24 | A | K | R | N | L | N |
| 25 | S | K | R | D | L | D |
| 26 | X | K | R | R | L | D |
| 27 | X | K | R | H | L | D |
| **WT** | **Y** | **R** | **Q** | **H** | **I** | **D** |
| **DRS-8** | **Q** | **K** | **R** | **R** | **L** | **Q** |

| | | | | | | |
|---|---|---|---|---|---|---|
| "X" = amber codon | | | | | | |

**Table VIII. Relative receptor-binding affinity and bioactivity of DR5-8 variants**

| Variant | Amino acid changes^{a} | DR4-binding^{b} | DR5-binding | DcR2-binding | Bioactivity |
|---|---|---|---|---|---|
| Apo2L.DR5-8 | Y189Q:R191K:Q193R:H264 R:I266L:D267Q | >25 | 0.6 | 0.6 | 0.9 |
| Apo2L.DR5-8B | Y189Q:R191K:Q193R | 2.5 | 0.6 | 0.5 | 2.7 |
| Apo2L.DR5-8C | Y189Q:R191K:Q193R:1266L | 5.5 | 0.6 | 0.3 | 0.6 |

| | | | | | |
|---|---|---|---|---|---|
| ^{a}Amino acid changes are relative to wild-type Apo2L ^{b}Receptor-binding and bioactivity values are the ratio relative to wild-type Apo2L | | | | | |

## Claims

1. An Apo-2 ligand variant polypeptide having at least 80% amino acid sequence identity to an Apo-2 ligand polypeptide sequence of Figure 1 (SEQ ID NO:1), wherein the Apo-2 ligand variant polypeptide comprises one or more amino acid substitutions, the substitutions conferring selective binding of the polypeptide to DR4 receptor or DR5 receptor such that the polypeptide binds ten-fold more to one receptor than the other (as compared to the polypeptide of Figure 1(SEQ ID NO:1); and wherein said Apo-2 ligand receptor binding induces apoptosis in at least one type of mammalian cell.

2. The Apo-2 ligand variant polypeptide of claim 1 wherein the polypeptide binds with at least 100 fold more affinity to one receptor than the other (as compared to the polypeptide of Figure 1(SEQ ID NO: 1).

3. The Apo-2 ligand variant polypeptide of claim 1 or 2, wherein the one or more amino acid substitutions are at positions 189, 193, 199 and/or 201.

4. The Apo-2 ligand variant polypeptide of claim 3 wherein the substitutions are at the residue position(s) in Figure 1 (SEQ ID NO: 1) selected from the group consisting of:
Y189A: Q193S: N199V: K201R (D4-8), and
Y189A: Q193A: N199R: K201R (D4-9).

5. The Apo-2 ligand variant polypeptide of claim 1 or 2, wherein the one or more amino acid substitutions are at positions 189, 191, 193, 264, 266, 267 and 269.

6. The Apo-2 ligand variant polypeptide of claim 5 wherein the substitutions are at the residue position(s) in Figure 1 (SEQ ID NO: 1) selected from the group consisting of:
Y189Q: R191K: Q193R: H264R: 1266L: D267Q (DR5-8), and
Y189A: R191K: Q193R: H264S: 1266L: D269E (DR5-23).

7. The Apo-2 ligand variant polypeptide of any of the preceding claims, wherein the polypeptide sequence comprises residues 114-281 of Figure 1 (SEQ ID NO:1).

8. An isolated nucleic acid molecule comprising DNA encoding the Apo-2 ligand variant polypeptide of any one of claims 1 to 7.

9. A vector comprising the encoding DNA of claim 8.

10. A host cell comprising the vector of claim 9, wherein said host cell is optionally an E. coli cell, CHO cell or yeast cell.

11. A method of producing Apo-2 ligand variant polypeptide comprising culturing the host cell of claim 10 under conditions sufficient to express said Apo-2 ligand variant polypeptide and recovering said Apo-2 ligand variant polypeptide from said culture.

12. A composition comprising the Apo-2 ligand variant polypeptide of any of claims 1 to 7, wherein said composition optionally comprises a therapeutically acceptable formulation which contains one or more divalent metal ions.

13. An *in vitro* or *ex vivo* method of inducing apoptosis in mammalian cells comprising exposing mammalian cells expressing DR4 and/or DR5 receptor to an effective amount of Apo-2 ligand variant polypeptide of any one of claims 1 to 7.

14. An Apo-2 ligand variant polypeptide of any of claims 1 to 7 for use in therapy.

15. An Apo-2 ligand variant polypeptide of any of claims 1 to 7 for use in a method of treating cancer or an immune-related disease.

16. The Apo-2 ligand variant polypeptide of claim 15, wherein said mammalian cancer cells comprise lung cancer cells, breast cancer cells, glioma cancer cells, or colon or colorectal cancer cells.

17. The Apo-2 ligand variant polypeptide of claim 15 or 16 further comprising a prodrug, cytotoxic agent, chemotherapeutic agent, growth inhibitory agent, or cytokine.

18. The Apo-2 ligand variant polypeptide of claim 15, wherein said immune-related disease is arthritis or multiple sclerosis.


Sequence Listing

<110> GENENTECH, INC.

<120> APO-2 LIGAND/TRAIL VARIANTS AND USES THEREOF

<130> SJK/FP6790539

<140> EP 11183690.4
<141> 2003-06-23

<150> EP 03761262.9
<151> 2003-06-23

<150> PCT/US2003/019750
<151> 2003-06-23

<150> US 60/391,050
<151> 2002-06-24

<160> 13

<210> 1
<211> 281
<212> PRT
<213> Homo sapiens

<400> 1

<210> 2
<211> 1042
<212> DNA
<213> Homo sapiens

<220>
<221> Unsure
<222> 447
<223> Unknown base

<400> 2
tttcctcact gactataaaa gaatagagaa ggaagggctt cagtgaccgg 50

ctgcctggct gacttacagc agtcagactc tgacaggatc atggctatga 100

tggaggtcca ggggggaccc agcctgggac agacctgcgt gctgatcgtg 150

atcttcacag tgctcctgca gtctctctgt gtggctgtaa cttacgtgta 200

ctttaccaac gagctgaagc agatgcagga caagtactcc aaaagtggca 250

ttgcttgttt cttaaaagaa gatgacagtt attgggaccc caatgacgaa 300

gagagtatga acagcccctg ctggcaagtc aagtggcaac tccgtcagct 350

cgttagaaag atgattttga gaacctctga ggaaaccatt tctacagttc 400

aagaaaagca acaaaatatt tctcccctag tgagagaaag aggtccncag 450

agagtagcag ctcacataac tgggaccaga ggaagaagca acacattgtc 500

ttctccaaac tccaagaatg aaaaggctct gggccgcaaa ataaactcct 550

gggaatcatc aaggagtggg cattcattcc tgagcaactt gcacttgagg 600

aatggtgaac tggtcatcca tgaaaaaggg ttttactaca tctattccca 650

aacatacttt cgatttcagg aggaaataaa agaaaacaca aagaacgaca 700

aacaaatggt ccaatatatt tacaaataca caagttatcc tgaccctata 750

ttgttgatga aaagtgctag aaatagttgt tggtctaaag atgcagaata 800

tggactctat tccatctatc aagggggaat atttgagctt aaggaaaatg 850

acagaatttt tgtttctgta acaaatgagc acttgataga catggaccat 900

gaagccagtt ttttcggggc ctttttagtt ggctaactga cctggaaaga 950

aaaagcaata acctcaaagt gactattcag ttttcaggat gatacactat 1000

gaagatgttt caaaaaatct gaccaaaaca aacaaacaga aa 1042

<210> 3

<211> 468
<212> PRT
<213> Homo sapiens

<400> 3
Met Ala Pro Pro Pro Ala Arg Val His Leu Gly Ala Phe Leu Ala
1               5                   10                  15

Val Thr Pro Asn Pro Gly Ser Ala Ala Ser Gly Thr Glu Ala Ala
                20                  25                  30

Ala Ala Thr Pro Ser Lys Val Trp Gly Ser Ser Ala Gly Arg Ile
                35                  40                  45

Glu Pro Arg Gly Gly Gly Arg Gly Ala Leu Pro Thr Ser Met Gly
                50                  55                  60

Gln His Gly Pro Ser Ala Arg Ala Arg Ala Gly Arg Ala Pro Gly
                65                  70                  75

Pro Arg Pro Ala Arg Glu Ala Ser Pro Arg Leu Arg Val His Lys
                80                  85                  90

Thr Phe Lys Phe Val Val Val Gly Val Leu Leu Gln Val Val Pro
                95                  100                 105

Ser Ser Ala Ala Thr Ile Lys Leu His Asp Gln Ser Ile Gly Thr
                110                 115                 120

Gln Gln Trp Glu His Ser Pro Leu Gly Glu Leu Cys Pro Pro Gly
                125                 130                 135

Ser His Arg Ser Glu Arg Pro Gly Ala Cys Asn Arg Cys Thr Glu
                140                 145                 150

Gly Val Gly Tyr Thr Asn Ala Ser Asn Asn Leu Phe Ala Cys Leu
                155                 160                 165

Pro Cys Thr Ala Cys Lys Ser Asp Glu Glu Glu Arg Ser Pro Cys
                170                 175                 180

Thr Thr Thr Arg Asn Thr Ala Cys Gln Cys Lys Pro Gly Thr Phe
                185                 190                 195

Arg Asn Asp Asn Ser Ala Glu Met Cys Arg Lys Cys Ser Thr Gly
                200                 205                 210

Cys Pro Arg Gly Met Val Lys Val Lys Asp Cys Thr Pro Trp Ser
                215                 220                 225

Asp Ile Glu Cys Val His Lys Glu Ser Gly Asn Gly His Asn Ile
                230                 235                 240

Trp Val Ile Leu Val Val Thr Leu Val Val Pro Leu Leu Leu Val
                245                 250                 255

Ala Val Leu Ile Val Cys Cys Cys Ile Gly Ser Gly Cys Gly Gly
                260                 265                 270

Asp Pro Lys Cys Met Asp Arg Val Cys Phe Trp Arg Leu Gly Leu
                275                 280                 285

Leu Arg Gly Pro Gly Ala Glu Asp Asn Ala His Asn Glu Ile Leu
                290                 295                 300

Ser Asn Ala Asp Ser Leu Ser Thr Phe Val Ser Glu Gln Gln Met
                305                 310                 315

Glu Ser Gln Glu Pro Ala Asp Leu Thr Gly Val Thr Val Gln Ser
                320                 325                 330

Pro Gly Glu Ala Gln Cys Leu Leu Gly Pro Ala Glu Ala Glu Gly
                335                 340                 345

Ser Gln Arg Arg Arg Leu Leu Val Pro Ala Asn Gly Ala Asp Pro
                350                 355                 360

Thr Glu Thr Leu Met Leu Phe Phe Asp Lys Phe Ala Asn Ile Val
                365                 370                 375

Pro Phe Asp Ser Trp Asp Gln Leu Met Arg Gln Leu Asp Leu Thr
                380                 385                 390

Lys Asn Glu Ile Asp Val Val Arg Ala Gly Thr Ala Gly Pro Gly
                395                 400                 405

Asp Ala Leu Tyr Ala Met Leu Met Lys Trp Val Asn Lys Thr Gly
                410                 415                 420

Arg Asn Ala Ser Ile His Thr Leu Leu Asp Ala Leu Glu Arg Met
                425                 430                 435

Glu Glu Arg His Ala Lys Glu Lys Ile Gln Asp Leu Leu Val Asp
                440                 445                 450

Ser Gly Lys Phe Ile Tyr Leu Glu Asp Gly Thr Gly Ser Ala Val
                455                 460                 465

Ser Leu Glu

<210> 4
<211> 1407
<212> DNA
<213> Homo sapiens

<400> 4
atggcgccac caccagctag agtacatcta ggtgcgttcc tggcagtgac 50

tccgaatccc gggagcgcag cgagtgggac agaggcagcc gcggccacac 100

ccagcaaagt gtggggctct tccgcgggga ggattgaacc acgaggcggg 150

ggccgaggag cgctccctac ctccatggga cagcacggac ccagtgcccg 200

ggcccgggca gggcgcgccc caggacccag gccggcgcgg gaagccagcc 250

ctcggctccg ggtccacaag accttcaagt ttgtcgtcgt cggggtcctg 300

ctgcaggtcg tacctagctc agctgcaacc atgatcaatc aattggcaca 350

aattggcaca cagcaatggg aacatagccc tttgggagag ttgtgtccac 400

caggatctca tagatcagaa cgtcctggag cctgtaaccg gtgcacagag 450

ggtgtgggtt acaccaatgc ttccaacaat ttgtttgctt gcctcccatg 500

tacagcttgt aaatcagatg aagaagagag aagtccctgc accacgacca 550

ggaacacagc atgtcagtgc aaaccaggaa ctttccggaa tgacaattct 600

gctgagatgt gccggaagtg cagcacaggg tgccccagag ggatggtcaa 650

ggtcaaggat tgtacgccct ggagtgacat cgagtgtgtc cacaaagaat 700

caggcaatgg acataatata tgggtgattt tggttgtgac tttggttgtt 750

ccgttgctgt tggtggctgt gctgattgtc tgttgttgca tcggctcagg 800

ttgtggaggg gaccccaagt gcatggacag ggtgtgtttc tggcgcttgg 850

gtctcctacg agggcctggg gctgaggaca atgctcacaa cgagattctg 900

agcaacgcag actcgctgtc cactttcgtc tctgagcagc aaatggaaag 950

ccaggagccg gcagatttga caggtgtcac tgtacagtcc ccaggggagg 1000

cacagtgtct gctgggaccg gcagaagctg aagggtctca gaggaggagg 1050

ctgctggttc cagcaaatgg tgctgacccc actgagactc tgatgctgtt 1100

ctttgacaag tttgcaaaca tcgtgccctt tgactcctgg gaccagctca 1150

tgaggcagct ggacctcacg aaaaatgaga tcgatgtggt cagagctggt 1200

acagcaggcc caggggatgc cttgtatgca atgctgatga aatgggtcaa 1250

caaaactgga cggaacgcct cgatccacac cctgctggat gccttggaga 1300

ggatggaaga gagacatgca aaagagaaga ttcaggacct cttggtggac 1350

tctggaaagt tcatctactt agaagatggc acaggctctg ccgtgtcctt 1400

ggagtga 1407

<210> 5
<211> 411
<212> PRT
<213> Homo sapiens

<400> 5

<210> 6
<211> 440
<212> PRT
<213> Homo sapiens

<400> 6

<210> 7
<211> 161
<212> PRT
<213> Homo sapiens

<400> 7
Arg Val Ala Ala His Ile Thr Gly Thr Arg Gly Arg Ser Asn Thr
1               5                   10                  15

Leu Ser Ser Pro Asn Ser Lys Asn Glu Lys Ala Leu Gly Arg Lys
                20                  25                  30

Ile Asn Ser Trp Glu Ser Ser Arg Ser Gly His Ser Phe Leu Ser
                35                  40                  45

Asn Leu His Leu Arg Asn Gly Glu Leu Val Ile His Glu Lys Gly
                50                  55                  60

Phe Tyr Tyr Ile Tyr Ser Gln Thr Tyr Phe Arg Phe Gln Glu Glu
                65                  70                  75

Ile Lys Glu Asn Thr Lys Asn Asp Lys Gln Met Val Gln Tyr Ile
                80                  85                  90

Tyr Lys Tyr Thr Ser Tyr Pro Asp Pro Ile Leu Leu Met Lys Ser
                95                  100                 105

Ala Arg Asn Ser Cys Trp Ser Lys Asp Ala Glu Tyr Gly Leu Tyr
                110                 115                 120

Ser Ile Tyr Gln Gly Gly Ile Phe Glu Leu Lys Glu Asn Asp Arg
                125                 130                 135

Ile Phe Val Ser Val Thr Asn Glu His Leu Ile Asp Met Asp His
                140                 145                 150

Glu Ala Ser Phe Phe Gly Ala Phe Leu Val Gly
                155                 160

<210> 8
<211> 144
<212> PRT
<213> Homo sapiens

<400> 8
Lys Pro Ala Ala His Leu Ile Gly Asp Pro Ser Lys Gln Asn Ser
1               5                   10                  15

Leu Leu Trp Arg Ala Asn Thr Asp Arg Ala Phe Leu Gln Asp Gly
                20                  25                  30

Phe Ser Leu Ser Asn Asn Ser Leu Leu Val Pro Thr Ser Gly Ile
                35                  40                  45

Tyr Phe Val Tyr Ser Gln Val Val Phe Ser Gly Lys Ala Tyr Ser
                50                  55                  60

Pro Lys Ala Thr Ser Ser Pro Leu Tyr Leu Ala His Glu Val Gln
                65                  70                  75

Leu Phe Ser Ser Gln Tyr Pro Phe His Val Pro Leu Leu Ser Ser
                80                  85                  90

Gln Lys Met Val Tyr Pro Gly Leu Gln Glu Pro Trp Leu His Ser
                95                  100                 105

Met Tyr His Gly Ala Ala Phe Gln Leu Thr Gln Gly Asp Gln Leu
                110                 115                 120

Ser Thr His Thr Asp Gly Ile Pro His Leu Val Leu Ser Pro Ser
                125                 130                 135

Thr Val Phe Phe Gly Ala Phe Ala Leu
                140

<210> 9
<211> 147
<212> PRT
<213> Homo sapiens

<400> 9
Lys Pro Val Ala His Val Val Ala Asn Pro Gln Ala Glu Gly Gln
1               5                   10                  15

Leu Gln Trp Leu Asn Arg Arg Ala Asn Ala Leu Leu Ala Asn Gly
                20                  25                  30

Val Glu Leu Arg Asp Asn Gln Leu Val Val Pro Ser Glu Gly Leu
                35                  40                  45

Tyr Leu Ile Tyr Ser Gln Val Leu Phe Lys Gly Gln Gly Cys Pro
                50                  55                  60

Ser Thr His Val Leu Leu Thr His Thr Ile Ser Arg Ile Ala Val
                65                  70                  75

Ser Tyr Gln Thr Lys Val Asn Leu Leu Ser Ala Ile Lys Ser Pro
                80                  85                  90

Cys Gln Arg Glu Thr Pro Glu Gly Ala Glu Ala Lys Pro Trp Tyr
                95                  100                 105

Glu Pro Ile Tyr Leu Gly Gly Val Phe Gln Leu Glu Lys Gly Asp
                110                 115                 120

Arg Leu Ser Ala Glu Ile Asn Arg Pro Asp Tyr Leu Leu Phe Ala
                125                 130                 135

Glu Ser Gly Gln Val Tyr Phe Gly Ile Ile Ala Leu
                140                 145

<210> 10
<211> 141
<212> PRT
<213> Homo sapiens

<400> 10
Gln Ile Ala Ala His Val Ile Ser Glu Ala Ser Ser Lys Thr Thr
1               5                   10                  15

Ser Val Leu Gln Trp Ala Glu Lys Gly Tyr Tyr Thr Met Ser Asn
                20                  25                  30

Asn Leu Val Thr Leu Glu Asn Gly Lys Gln Leu Thr Val Lys Arg
                35                  40                  45

Gln Gly Leu Tyr Tyr Ile Tyr Ala Gln Val Thr Phe Cys Ser Asn
                50                  55                  60

Arg Glu Ala Ser Ser Gln Ala Pro Phe Ile Ala Ser Leu Cys Leu
                65                  70                  75

Lys Ser Pro Gly Arg Phe Glu Arg Ile Leu Leu Arg Ala Ala Asn
                80                  85                  90

Thr His Ser Ser Ala Lys Pro Cys Gly Gln Gln Ser Ile His Leu
                95                  100                 105

Gly Gly Val Phe Glu Leu Gln Pro Gly Ala Ser Val Phe Val Asn

                110                 115                 120

Val Thr Asp Pro Ser Gln Val Ser His Gly Thr Gly Phe Thr Ser
                125                 130                 135

Phe Gly Leu Leu Lys Leu
                140

<210> 11
<211> 137
<212> PRT
<213> Homo sapiens

<400> 11
Arg Lys Val Ala His Leu Thr Gly Lys Ser Asn Ser Arg Ser Met
1               5                   10                  15

Pro Leu Glu Trp Glu Asp Thr Tyr Gly Ile Val Leu Leu Ser Gly
                20                  25                  30

Val Lys Tyr Lys Lys Gly Gly Leu Val Ile Asn Glu Thr Gly Leu
                35                  40                  45

Tyr Phe Val Tyr Ser Lys Val Tyr Phe Arg Gly Gln Ser Cys Asn
                50                  55                  60

Asn Leu Pro Leu Ser His Lys Val Tyr Met Arg Asn Ser Lys Tyr
                65                  70                  75

Pro Gln Asp Leu Val Met Met Glu Gly Lys Met Met Ser Tyr Cys
                80                  85                  90

Thr Thr Gly Gln Met Trp Ala Arg Ser Ser Tyr Leu Gly Ala Val
                95                  100                 105

Phe Asn Leu Thr Ser Ala Asp His Leu Tyr Val Asn Val Ser Glu
                110                 115                 120

Leu Ser Leu Val Asn Phe Glu Glu Ser Gln Thr Phe Phe Gly Leu
                125                 130                 135

Tyr Lys

<210> 12
<211> 152
<212> PRT
<213> Homo sapiens

<400> 12
Gln Pro Phe Ala His Leu Thr Ile Asn Ala Thr Asp Ile Pro Ser
1               5                   10                  15

Gly Ser His Lys Val Ser Leu Ser Ser Trp Tyr His Asp Arg Gly
                20                  25                  30

Trp Ala Lys Ile Ser Asn Met Thr Phe Ser Asn Gly Lys Leu Ile
                35                  40                  45

Val Asn Gln Asp Gly Phe Tyr Tyr Leu Tyr Ala Asn Ile Cys Phe
                50                  55                  60

Arg His His Glu Thr Ser Gly Asp Leu Ala Thr Glu Tyr Leu Gln
                65                  70                  75

Leu Met Val Tyr Val Thr Lys Thr Ser Ile Lys Ile Pro Ser Ser
                80                  85                  90

His Thr Leu Met Lys Gly Gly Ser Thr Lys Tyr Trp Ser Gly Asn
                95                  100                 105

Ser Glu Phe His Phe Tyr Ser Ile Asn Val Gly Gly Phe Phe Lys
                110                 115                 120

Leu Arg Ser Gly Glu Glu Ile Ser Ile Glu Val Ser Asn Pro Ser
                125                 130                 135

Leu Leu Asp Pro Asp Gln Asp Ala Thr Tyr Phe Gly Ala Phe Lys
                140                 145                 150

Val Arg

<210> 13
<211> 36
<212> DNA
<213> Artificial sequence

<220>
<223> sequence is synthesized

<400> 13
tgtaaaacga cggccagtca cacaggaaac agccag 36